(19) Europäisches Patentamt European Patent Office Office européen des brevets

(11) **EP 2 421 530 B1**

(12) **EUROPEAN PATENT SPECIFICATION**

(45) Date of publication and mention
of the grant of the patent:
**21.08.2019 Bulletin 2019/34**

(21) Application number: **10766517.6**

(22) Date of filing: **23.04.2010**

(51) Int Cl.:
*A61K 9/14* *(2006.01)*     *A61K 9/16* *(2006.01)*
*A61K 31/421* *(2006.01)*     *C07D 263/20* *(2006.01)*

(86) International application number:
**PCT/AU2010/000468**

(87) International publication number:
**WO 2010/121324 (28.10.2010 Gazette 2010/43)**

(54) **FORMULATION OF METAXALONE**

METAXALONFORMULIERUNG

FORMULATION DE MÉTAXALONE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **24.04.2009 AU 2009901743
24.04.2009 US 172281 P**

(43) Date of publication of application:
**29.02.2012 Bulletin 2012/09**

(73) Proprietor: **Iceutica Pty Ltd.
Mouth Hawthorn, WA 6016 (AU)**

(72) Inventors:
• **DODD, Aaron
Centennial Park
New South Wales 2021 (AU)**
• **MEISER, Felix
Mount Claremont
Western Australia 6010 (AU)**
• **NORRET, Marck
Darlington
Western Australia 6070 (AU)**

• **RUSSELL, Adrian
Rivervale
Western Australia 6103 (AU)**
• **BOSCH, H William
Bryn Mawr
Pennsylvania 19010 (US)**

(74) Representative: **Conroy, John et al
Fish & Richardson P.C.
Highlight Business Towers
Mies-van-der-Rohe-Straße 8
80807 München (DE)**

(56) References cited:
WO-A1-2004/019937     WO-A1-2005/016310
WO-A1-2005/016310     WO-A1-2006/009419
WO-A1-2008/000042     WO-A1-2010/121320
WO-A1-2010/121321     WO-A1-2010/121322
WO-A1-2010/121323     WO-A2-2004/058216
WO-A2-2007/001451     WO-A2-2007/070851
WO-A2-2008/118331     US-A1- 2005 163 839

**EP 2 421 530 B1**

**Description**

**Field of the Invention**

The present invention relates to methods for producing particles of metaxalone using dry milling processes.

**Background**

Poor bioavailability is a significant problem encountered in the development of compositions in the therapeutic, cosmetic, agricultural and food industries, particularly those materials containing a biologically active material that is poorly soluble in water at physiological pH. An active material's bioavailability is the degree to which the active material becomes available to the target tissue in the body or other medium after systemic administration through, for example, oral or intravenous means. Many factors affect bioavailability, including the form of dosage and the solubility and dissolution rate of the active material.

In therapeutic applications, poorly and slowly water-soluble materials tend to be eliminated from the gastrointestinal tract before being absorbed into the circulation. In addition, poorly soluble active agents tend to be disfavored or even unsafe for intravenous administration due to the risk of particles of agent blocking blood flow through capillaries.

It is known that the rate of dissolution of a particulate drug will increase with increasing surface area. One way of increasing surface area is decreasing particle size. Consequently, methods of making finely divided or sized drugs have been studied with a view to controlling the size and size range of drug particles for pharmaceutical compositions.

For example, dry milling techniques have been used to reduce particle size and hence influence drug absorption. However, in conventional dry milling the limit of fineness is reached generally in the region of about 100 microns (100,000 nm), at which point material cakes on the milling chamber and prevents any further diminution of particle size. Alternatively, wet grinding may be employed to reduce particle size, but flocculation restricts the lower particle size limit to approximately 10 microns (10,000 nm). The wet milling process, however, is prone to contamination, thereby leading to a bias in the pharmaceutical art against wet milling. Another alternative milling technique, commercial airjet milling, has provided particles ranging in average size from as low as about 1 to about 50 microns (1,000-50,000 nm).

There are several approaches currently used to formulate poorly soluble active agents. One approach is to prepare the active agent as a soluble salt. Where this approach cannot be employed, alternate (usually physical) approaches are employed to improve the solubility of the active agent. Alternate approaches generally subject the active agent to physical conditions that change the agent's physical and or chemical properties to improve its solubility. These include process technologies such as micronization, modification of crystal or polymorphic structure, development of oil based solutions, use of co-solvents, surface stabilizers or complexing agents, micro-emulsions, super critical fluid and production of solid dispersions or solutions. More than one of these processes may be used in combination to improve formulation of a particular therapeutic material. Many of these approaches commonly convert a drug into an amorphous state, which generally leads to a higher dissolution rate. However, formulation approaches that result in the production of amorphous material are not common in commercial formulations due to concerns relating to stability and the potential for material to recrystallize.

These techniques for preparing such pharmaceutical compositions tend to be complex. By way of example, a principal technical difficulty encountered with emulsion polymerization is the removal of contaminants, such as unreacted monomers or initiators (which may have undesirable levels of toxicity), at the end of the manufacturing process.

Another method of providing reduced particle size is the formation of pharmaceutical drug microcapsules, which techniques include micronizing, polymerisation and co-dispersion. However, these techniques suffer from a number of disadvantages including at least the inability to produce sufficiently small particles such as those obtained by milling, and the presence of co-solvents and/or contaminants such as toxic monomers which are difficult to remove, leading to expensive manufacturing processes.

Over the last decade, intense scientific investigation has been carried out to improve the solubility of active agents by converting the agents to ultra fine powders by methods such as milling and grinding. These techniques may be used to increase the dissolution rate of a particulate solid by increasing the overall surface area and decreasing the mean particle size. US Patent 6,634,576 discloses examples of wet-milling a solid substrate, such as a pharmaceutically active compound, to produce a "synergetic co-mixture".

International Patent Application PCT/AU2005/001977 (Nanoparticle Composition(s) and Method for Synthesis Thereof) describes, *inter alia,* a method comprising the step of contacting a precursor compound with a co-reactant under mechanochemical synthesis conditions wherein a solid-state chemical reaction between the precursor compound and the co-reactant produces therapeutically active nanoparticles dispersed in a carrier matrix. Mechanochemical synthesis, as discussed in International Patent Application PCT/AU2005/001977, refers to the use of mechanical energy to activate, initiate or promote a chemical reaction, a crystal structure transformation or a phase change in a material or a mixture of materials, for example by agitating a reaction mixture in the presence of a milling media to transfer mechanical energy to the reaction mixture, and includes without limitation "mechanochemical activation", "mechanochemical processing", "reactive milling", and related processes.

International Patent Application PCT/AU2007/000910 (Methods for the preparation of biologically active compounds in nanoparticulate form) describes, *inter alia,* a method for dry milling raloxifene with lactose and NaCl which produced nanoparticulate raloxifene without significant aggregation problems. The methods disclosed by the prior art produce nanoparticles at volume fractions of 15% or less and suggests that 25% is the upper limit for the volume fraction of the biologically active material that could be successfully converted to smaller particles.

WO 2005/016310 A1 describes nanoparticulate compositions comprising metaxalone, wherein the the metaxalone particles have an effective average particle size of less than 2 microns.

The present invention provides methods for an improved milling process which produces particles of active compound with increased surface area, yet allows for higher volume fractions of the biologically active material.

One example is the drug metaxalone which is commercially marketed under the name Skelaxin®. Skelaxin® is indicated as an adjunct to rest, physical therapy, and other measures for the relief of discomforts associated with acute, painful musculoskeletal conditions and is taken as an 800 mg tablet three to four times a day. Previous animal studies have shown that by reducing the size of metaxalone much higher rates of absorption and overall bioavaiability (as measured by AUC) can be achieved. The present invention being able to produce small particles (with increased surface area) at high volume volume fractions is thus well suited to a drug such as metaxalone. So a method such as the present invention which provides for improved dissolution and potentially higher bioavailability could result in a formulation of metaxalone where much less active is required to deliver the same therapeutic effect.

Although the background to the present invention is discussed in the context of improving the bioavailability of materials that are poorly or slowly water soluble, the applications of the methods of the present invention are not limited to such, as is evident from the following description of the invention.

Further, although the background to the present invention is largely discussed in the context of improving the bioavailability of therapeutic or pharmaceutical compounds, the applications of the methods of the present invention are clearly not limited to such. For example, as is evident from the following description, applications of the methods of the present invention include but are not limited to: nutraceutical and nutritional compounds, complementary medicinal compounds, veterinary therapeutic applications and agricultural chemical applications, such as pesticide, fungicide or herbicide.

Furthermore an application of the current invention would be to materials which contain a biologically active compound such as, but not limited to a therapeutic or pharmaceutical compound, a nutraceutical or nutrient, a complementary medicinal product such as active components in plant or other naturally occurring material, a veterinary therapeutic compound or an agricultural compound such as a pesticide, fungicide or herbicide. Specific examples would be the spice turmeric that contains the active compound curcumin, or flax seed that contains the nutrient ALA an omega 3 fatty acid. As these specific examples indicate this invention could be applied to, but not limited to, a range of natural products such as seeds, cocoa and cocoa solids, coffee, herbs, spices, other plant materials or food materials that contain a biologically active compound. The application of this invention to these types of materials would enable greater availability of the active compound in the materials when used in the relevant application. For example where material subject to this invention is orally ingested the active would be more bioavailable.

## Summary of the Invention

The present invention is defined by independent claim 1. The dependent claims depict other embodiments of the invention. The present invention is directed to the unexpected finding that particles of a biologically active material can be produced by dry milling processes wherein the composition produced by said method comprises particles of the biologically active material at or above a volume fraction of 25 v/v%. In one surprising aspect the crystallinity of the active material is unchanged or not substantially changed. In a preferred embodiment the present invention is directed to the unexpected finding that particles of metaxalone can be produced by dry milling processes at commercial scale.

Preferably the method comprises particles of the biologically active material at or above a volume fraction selected from the group consisting of 25 v/v%; 30 v/v%; 35 v/v%; 40 v/v%; 45 v/v%; 50 v/v%, 55 v/v% and 60 v/v%. Preferably the method comprises particles of the biologically active material at or below a volume fraction selected from the group consisting of 60 v/v%, 55 v/v%, 50 v/v%; 45 v/v%; 40 v/v%; and 35 v/v%.

Thus in a first aspect the invention comprises a method producing a composition, comprising the steps of dry milling a solid biologically active material and a millable grinding matrix in a mill comprising a plurality of milling bodies, for a time period sufficient to produce particles of the biologically active material dispersed in an at least partially milled grinding material, wherein the composition produced by said method comprises particles of the biologically active material at or above a volume fraction of 25 v/v%.

As an example, the average particle size, determined on a particle number basis, is equal to or less than a size selected from the group 2000 nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm and 100 nm. Preferably, the average particle size is equal to or greater than 25nm.

In a a preferred embodiment, the particles have a median particle size, determined on a particle volume basis, equal or less than a size selected from the group 500nm, 400 nm, 300nm, 200nm and 100 nm. Preferably, the median particle size is equal to or greater than 25nm. Preferably, the percentage of particles, on a particle volume basis, is selected from the group consisting of: 50%, 60%, 70%, 80%, 90%, 95% and 100 % less than 2000nm (% < 2000 nm). Preferably, the percentage of particles, on a particle volume basis, is selected from the group consisting of: 50%, 60%, 70%, 80%, 90%, 95% and 100 % less than 1000nm (% < 1000 nm). Preferably, the percentage of particles, on a particle volume basis, is selected from the group 0%, 10%, 20%, 30%, 40%, 50 %, 60%, 70%, 80%, 90%, 95% and 100 % less than 500nm (% < 500 nm). Preferably, the percentage of particles, on a particle volume basis, is selected from the group 0%, 10%, 20%, 30%, 40%, 50 %, 60%, 70%, 80%, 90%, 95% and 100 % less than 300nm (% < 300 nm). Preferably, the percentage of particles, on a particle volume basis, is selected from the group 0%, 10%, 20%, 30%, 40%, 50 %, 60%, 70%, 80%, 90%, 95% and 100 % less than 200nm (% < 200 nm). Preferably, the Dx of the particle size distribution, as measured on a particle volume basis, is selected from the group consisting of less than or equal to 10,000nm, 5000nm, 3000nm, 2000nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm, and 100 nm; wherein x is greater than or equal to 90.

In another preferred embodiment, the crystallinity profile of the biologically active material is selected from the group consisting of: at least 50% of the biologically active material is crystalline, at least 60% of the biologically active material is crystalline, at least 70% of the biologically active material is crystalline, at least 75% of the biologically active material is crystalline, at least 85% of the biologically active material is crystalline, at least 90% of the biologically active material is crystalline, at least 95% of the biologically active material is crystalline and at least 98% of the biologically active material is crystalline. More preferably, the crystallinity profile of the biologically active material is substantially equal to the crystallinity profile of the biologically active material before the material was subjected to the method as described herein.

In another preferred embodiment, the amorphous content of the biologically active material is selected from the group consisting of: less than 50% of the biologically active material is amorphous, less than 40% of the biologically active material is amorphous, less than 30% of the biologically active material is amorphous, less than 25% of the biologically active material is amorphous, less than 15% of the biologically active material is amorphous, less than 10% of the biologically active material is amorphous, less than 5% of the biologically active material is amorphous and less than 2% of the biologically active material is amorphous. Preferably, the biologically active material has no significant increase in amorphous content after subjecting the material to the method as described herein.

In another preferred embodiment, the milling time period is a range selected from the group consisting of: between 10 minutes and 2 hours, between 10 minutes and 90 minutes, between 10 minutes and 1 hour, between 10 minutes and 45 minutes, between 10 minutes and 30 minutes, between 5 minutes and 30 minutes, between 5 minutes and 20 minutes, between 2 minutes and 10 minutes, between 2 minutes and 5 minutes, between 1 minutes and 20 minutes, between 1 minute and 10 minutes, and between 1 minute and 5 minutes.

In another preferred embodiment, the milling medium is selected from the group consisting of: ceramics, glasses, polymers, ferromagnetics and metals. Preferably, the milling medium is steel balls having a diameter selected from the group consisting of: between 1 and 20 mm, between 2 and 15 mm and between 3 and 10 mm. In another preferred embodiment, the milling medium is zirconium oxide balls having a diameter selected from the group consisting of: between 1 and 20 mm, between 2 and 15 mm and between 3 and 10 mm. Preferably, the dry milling apparatus is a mill selected from the group consisting of: attritor mills (horizontal or vertical), nutating mills, tower mills, pearl mills, planetary mills, vibratory mills, eccentric vibratory mills, gravity-dependent-type ball mills, rod mills, roller mills and crusher mills. Preferably, the milling medium within the milling apparatus is mechanically agitated by 1, 2 or 3 rotating shafts. Preferably, the method is configured to produce the biologically active material in a continuous fashion.

Preferably, the total combined amount of biologically active material and grinding matrix in the mill at any given time is equal to or greater than a mass selected from the group consisting of: 200 grams, 500 grams, 1 kg, 2kg, 5kg, 10kg, 20kg, 30kg, 50kg, 75kg, 100kg, 150kg, 200kg. Preferably, the total combined amount of biologically active material and grinding matrix is less than 2000kg.

In another preferred embodiment, the grinding matrix is a single material or is a mixture of two or more materials in any proportion. Preferably, the single material or a mixture of two or more materials is selected from the group consisting of: mannitol, sorbitol, Isomalt, xylitol, maltitol, lactitol, erythritol, arabitol, ribitol, glucose, fructose, mannose, galactose, anhydrous lactose, lactose monohydrate, sucrose, maltose, trehalose, maltodextrins, dextrin, Inulin, dextrates, polydextrose, starch, wheat flour, corn flour, rice flour, rice starch, tapioca flour, tapioca starch, potato flour, potato starch, other flours and starches, milk powder, skim milk powders, other milk solids and dreviatives, soy flour, soy meal or other soy products, cellulose, microcystalline cellulose, microcrystalline cellulose based co blended materials, pregelatinized (or partially) starch, HPMC, CMC, HPC, citric acid, tartaric acid, malic acid, maleic acid fumaric acid , ascorbic acid, succinic acid, sodium citrate, sodium tartrate, sodium malate, sodium ascorbate, potassium citrate, potassium tartrate, potassium malate, potassium ascorbate, sodium carbonate, potassium carbonate, magnesium carbonate, sodium bicarbonate, potassium bicarbonate and calcium carbonate. dibasic calcium phosphate, tribasic calcium phosphate, sodium sulfate, sodium chloride, sodium metabisulphite, sodium thiosulfate, ammonium chloride, Glauber's salt, ammonium carbonate, sodium bisulfate, magnesium sulfate, potash alum, potassium chloride, sodium hydrogen sulfate, sodium hydroxide, crystalline hydroxides, hydrogen carbonates, ammonium chloride, methylamine hydrochloride, ammonium bromide, silica, thermal silica, alumina, titanium dioxide, talc, chalk, mica, kaolin, bentonite, hectorite, magnesium trisilicate, clay based materials or aluminium silicates, sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate , glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, , poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Soidum Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend,Calcium Dodecylbenzene Sulfonate, Sodium Dodecylbenzene Sulfonate,Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines. Preferably, the concentration of the single (or first) material is selected from the group consisting of: 5 - 99 % w/w, 10 - 95 % w/w, 15 - 85 % w/w, of 20 - 80% w/w, 25 - 75 % w/w, 30 - 60% w/w, 40 -50% w/w. Preferably, the concentration of the second or subsequent material is selected from the group consisting of: 5 - 50 % w/w, 5 - 40 % w/w, 5 - 30 % w/w, of 5 - 20% w/w, 10 - 40 % w/w, 10 -30% w/w, 10 -20% w/w, 20 - 40% w/w, or 20 - 30% w/w or if the second or subsequent material is a surfactant or water soluble polymer the concentration is selected from 0.1 -10 % w/w, 0.1 -5 % w/w, 0.1 -2.5 % w/w, of 0.1 - 2% w/w, 0.1 -1 %, 0.5 -5% w/w, 0.5 -3% w/w, 0.5 -2% w/w, 0.5 - 1.5%, 0.5 -1 % w/w, of 0.75 - 1.25 % w/w, 0.75 -1% and 1% w/w.

Preferably, the grinding matrix is selected from the group consisting of:

(a) lactose monohydrate or lactose monohydrate combined with at least one material selected from the group consisting of: xylitol; lactose anhydrous; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(b) lactose anhydrous or lactose anhydrous combined with at least one material selected from the group consisting of: lactose monohydrate; xylitol; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(c) mannitol or mannitol combined with at least one material selected from the group consisting of: lactose monohydrate; xylitol; lactose anhydrous; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(d) Sucrose or sucrose combined with at least one material selected from the group consisting of: lactose monohydrate; lactose anhydrous; mannitol; microcrystalline cellulose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(e) Glucose or glucose combined with at least one material selected from the group consisting of: lactose monohydrate; lactose anhydrous; mannitol; microcrystalline cellulose; sucrose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(f) Sodium chloride or sodium chloride combined with at least one material selected from the group consisting of: lactose monohydrate; lactose anhydrous; mannitol; microcrystalline cellulose; sucrose; glucose; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(g) xylitol or xylitol combined with at least one material selected from the group consisting of: lactose monohydrate; lactose anhydrous; mannitol; microcrystalline cellulose; sucrose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(h) Tartaric acid or tartaric acid combined with at least one material selected from the group consisting of: lactose monohydrate; lactose anhydrous; mannitol; microcrystalline cellulose; sucrose; glucose; sodium chloride; talc; kaolin; calcium carbonate; malic acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(i) microcrystalline cellulose or microcrystalline cellulose combined with at least one material selected from the group consisting of: lactose monohydrate; xylitol; lactose anhydrous; mannitol; sucrose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(j) Kaolin combined with at least one material selected from the group consisting of: lactose monohydrate; xylitol; lactose anhydrous; mannitol; microcrystalline cellulose; sucrose; glucose; sodium chloride; talc; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

(k) Talc combined with at least one material selected from the group consisting of: lactose monohydrate; xylitol; lactose anhydrous; mannitol; microcrystalline cellulose; sucrose; glucose; sodium chloride; kaolin; calcium carbonate; malic acid; tartaric acid; trisodium citrate dihydrate; D,L-Malic acid; sodium pentane sulfate; sodium octadecyl sulfate; Brij700; Brij76; sodium n-lauroyl sacrosine; lecithin; docusate sodium; polyoxyl-40-stearate; Aerosil R972 fumed silica; sodium lauryl sulfate or other alkyl sulfate surfactants with a chain length between C5 to C18; polyvinyl pyrrolidone; sodium lauryl sulfate and polyethylene glycol 40 stearate, sodium lauryl sulfate and polyethylene glycol 100 stearate, sodium lauryl sulfate and PEG 3000, sodium lauryl sulphate and PEG 6000, sodium lauryl sulphate and PEG 8000, sodium lauryl sulphate and PEG 10000, sodium lauryl sulfate and Brij700, sodium lauryl sulfate and Poloxamer 407, sodium lauryl sulfate and Poloxamer 338, sodium lauryl sulfate and Poloxamer 188; Poloxamer 407, Poloxamer 338, Poloxamer 188, alkyl naphthalene sulfonate condensate/Lignosulfonate blend; Calcium Dodecylbenzene Sulfonate (Branched); Diisopropyl naphthalenesulphonate; erythritol distearate; linear and branched dodecylbenzene sulfonic acids; Naphthalene Sulfonate Formaldehyde Condensate; nonylphenol ethoxylate, POE-30; Phosphate Esters, Tristyrylphenol Ethoxylate, Free Acid; Polyoxyethylene (15) tallowalkylamines; sodium alkyl naphthalene sulfonate; sodium alkyl naphthalene sulfonate condensate; sodium alkylbenzene sulfonate; sodium isopropyl naphthalene sulfonate; Sodium Methyl Naphthalene; Formaldehyde Sulfonate; sodium salt of n-butyl naphthalene sulfonate; tridecyl alcohol ethoxylate, POE-18; Triethanolamine isodecanol phosphate ester; Triethanolamine tristyrylphosphate ester; Tristyrylphenol Ethoxylate Sulfate; Bis(2-hydroxyethyl)tallowalkylamines.

Preferably, the grinding matrix is selected from the group consisting of: a material considered to be Generally Regarded as Safe (GRAS) for pharmaceutical products; a material considered acceptable for use in an agricultural formulation; and a material considered acceptable for use in a veterinary formulation.

In another preferred embodiment, a milling aid or combination of milling aids is used. Preferably, the milling aid is selected from the group consisting of: colloidal silica, a surfactant, a polymer, a stearic acid and derivatives thereof.

Preferably, the surfactant is in a solid form or can be manufactured into a solid form. Preferably, the surfactant is selected from the group consisting of: polyoxyethylene alkyl ethers, polyoxyethylene stearates, polyethylene glycols (PEG), poloxamers, poloxamines, sarcosine based surfactants, polysorbates, aliphatic alcohols, alkyl and aryl sulfates, alkyl and aryl polyether sulfonates and other sulfate surfactants, trimethyl ammonium based surfactants, lecithin and other phospholipids, bile salts, polyoxyethylene castor oil derivatives, polyoxyethylene sorbitan fatty acid esters, Sorbitan fatty acid esters, Sucrose fatty acid esters, alkyl glucopyranosides, alkyl maltopyranosides, glycerol fatty acid esters, Alkyl Benzene Sulphonic Acids, Alkyl Ether Carboxylic Acids, Alkyl and aryl Phosphate esters, Alkyl and aryl Sulphate esters, Alkyl and aryl Sulphonic acids, Alkyl Phenol Phosphates esters, Alkyl Phenol Sulphates esters, Alkyl and Aryl Phosphates, Alkyl Polysaccharides, Alkylamine Ethoxylates, Alkyl-Naphthalene Sulphonates formaldehyde condensates, Sulfosuccinates, lignosulfonates, Ceto-Oleyl Alcohol Ethoxylates, Condensed Naphthalene Sulphonates, Dialkyl and Alkyl Naphthalene Sulphonates,Di-alkyl Sulphosuccinates, Ethoxylated nonylphenols, Ethylene Glycol Esters, Fatty Alcohol Alkoxylates, Hydrogenated tallowalkylamines, Mono-alkyl Sulphosuccinamates, Nonyl Phenol Ethoxylates, Sodium Oleyl N-methyl Taurate, Tallowalkylamines, linear and branched dodecylbenzene sulfonic acids.

Preferably, the surfactant is selected from the group consisting of: sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate , glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, poloxamer 338, poloxamer 407, polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Soidum Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend,Calcium Dodecylbenzene Sulfonate, Sodium Dodecylbenzene Sulfonate,Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines. Preferably the polymer is selected from the list of: polyvinylpyrrolidones (PVP), polyvinylalcohol, Acrylic acid based polymers and copolymers of acrylic acid

Preferably, the milling aid has a concentration selected from the group consisting of: 0.1 -10 % w/w, 0.1 -5 % w/w, 0.1 -2.5 % w/w, of 0.1 - 2% w/w, 0.1 -1 %, 0.5 -5% w/w, 0.5 -3% w/w, 0.5 - 2% w/w, 0.5 - 1.5%, 0.5 -1 % w/w, of 0.75 - 1.25 % w/w, 0.75 -1% and 1% w/w.

In another preferred embodiment of the invention, a facilitating agent is used or combination of facilitating agents is used. Preferably, the facilitating agent is selected from the group consisting of: surfactants, polymers, binding agents, filling agents, lubricating agents, sweeteners, flavouring agents, preservatives, buffers, wetting agents, disintegrants, effervescent agents, agents that may form part of a medicament, including a solid dosage form or a dry powder inhalation formulation and other material required for specific drug delivery. Preferably, the facilitating agent is added during dry milling. Preferably, the facilitating agent is added to the dry milling at a time selected from the group consisting of: with 1-5 % of the total milling time remaining, with 1-10 % of the total milling time remaining, with 1-20 % of the total milling time remaining, with 1-30 % of the total milling time remaining, with 2-5% of the total milling time remaining, with 2-10% of the total milling time remaining, with 5-20% of the total milling time remaining and with 5-20% of the total milling time remaining. Preferably, the disintegrant is selected from the group consisting of: crosslinked PVP, cross linked carmellose and sodium starch glycolate. Preferably, the facilitating agent is added to the milled biologically active material and grinding matrix and further processed in a mechanofusion process. Mechanofusion milling causes mechanical energy to be applied to powders or mixtures of particles in the micrometre and nanometre range.

The reasons for including facilitating agents include, but are not limited to providing better dispersibility, control of agglomeration, the release or retention of the active particles from the delivery matrix. Examples of facilitating agents include, but are not limited to crosslinked PVP (crospovidone), cross linked carmellose (croscarmellose), sodium starch glycolate, Povidone (PVP), Povidone K12, Povidone K17, Povidone K25, Povidone K29/32 and Povidone K30, stearic acid, magnesium stearate, calcium stearate, sodium stearyl fumarate, sodium stearyl lactylate, zinc stearate, sodium stearate or lithium stearate, other solid state fatty acids such as oleic acid, lauric acid, palmitic acid, erucic acid, behenic acid, or derivatives (such as esters and salts), Amino acids such as leucine, isoleucine, lysine, valine, methionine, phenylalanine, aspartame or acesulfame K. In a preferred aspect of manufacturing this formulation the facilitating agent is added to the milled mixture of biologically active material and co-grinding matrix and further processed in another milling device such as Mechnofusion, Cyclomixing, or impact milling such as ball milling, jet milling, or milling using a high pressure homogeniser, or combinations thereof. In a highly preferred aspect the facilitating agent is added to the milling of the mixture of biologically active material and co-grinding matrix as some time before the end of the milling process.

In another preferred embodiment, metaxalone is milled with lactose monohydrate and alkyl sulfates. Preferably metaxalone is milled with lactose monohydrate and sodium lauryl sulfate. Preferably metaxalone is milled with lactose monohydrate and sodium octadecyl sulfate. In another preferred embodiment, Metaxalone is milled with lactose monohydrate, alkyl sulfates and another surfactant or polymers. Preferably metaxalone is milled with lactose monohydrate, sodium lauryl sulfate and polyether sulfates. Preferably metaxalone is milled with lactose monohydrate, sodium lauryl sulfate and polyethylene glycol 40 stearate. Preferably metaxalone is milled with lactose monohydrate, sodium lauryl sulfate and polyethylene glycol 100 stearate. Preferably metaxalone is milled with lactose monohydrate, sodium lauryl sulfate and a poloxamer. Preferably metaxalone is milled with lactose monohydrate, sodium lauryl sulfate and poloxamer 407. Preferably metaxalone is milled with lactose monohydrate, sodium lauryl sulfate and poloxamer 338. Preferably metaxalone is milled with lactose monohydrate, sodium lauryl sulfate and poloxamer 188. Preferably metaxalone is milled with lactose monohydrate, sodium lauryl sulfate and a solid polyethylene glycol. Preferably metaxalone is milled with lactose monohydrate, sodium lauryl sulfate and polyethylene glycol 6000. Preferably metaxalone is milled with lactose monohydrate, sodium lauryl sulfate and polyethylene glycol 3000. In another preferred embodiment, Metaxalone is milled with lactose monohydrate and polyether sulfates. Preferably metaxalone is milled with lactose monohydrate and polyethylene glycol 40 stearate. Preferably metaxalone is milled with lactose monohydrate and polyethylene glycol 100 stearate In another preferred embodiment metaxalone is milled with lactose monohydrate and polyvinyl-pyrrolidine. Preferably metaxalone is milled with lactose monohydrate and polyvinyl-pyrrolidone with an approximate molecular weight of 30,000-40,000. In another preferred embodiment, metaxalone is milled with lactose monohydrate and alkyl sulfonates. Preferably metaxalone is milled with lactose monohydrate and docusate sodium. In another preferred embodiment, metaxalone is milled with lactose monohydrate and a surfactant. Preferably metaxalone is milled with lactose monohydrate and lecithin. Preferably metaxalone is milled with lactose monohydrate and sodium n-lauroyl sarcosine. Preferably metaxalone is milled with lactose monohydrate and polyoxyethylene alkyl ether surfactants. Preferably metaxalone is milled with lactose monohydrate and PEG 6000. In another preferred formulation metaxalone is milled with lactose monohydrate and silica. Preferably metaxalone is milled with lactose monohydrate and Aerosil R972 fumed silica. In another preferred embodiment, metaxalone is milled with with lactose monohydrate, tartaric acid and sodium lauryl sulfate. In another preferred embodiment, metaxalone is milled with with lactose monohydrate, sodium bicarbonate and sodium lauryl sulfate. In another preferred embodiment, metaxalone is milled with with lactose monohydrate, sodium bicarbonate, poloxamer 407 and sodium lauryl sulfate. In another preferred embodiment, metaxalone is milled with lactose monohydrate, potassium bicarbonate and sodium lauryl sulfate. In another preferred embodiment, metaxalone is milled with with lactose monohydrate, potassium bicarbonate, poloxamer 407 and sodium lauryl sulfate. In another preferred embodiment, metaxalone is milled with mannitol and alkyl sulfates. Preferably metaxalone is milled with mannitol and sodium lauryl sulfate. Preferably metaxalone is milled with mannitol and sodium octadecyl sulfate. In another preferred embodiment, Metaxalone is milled with mannitol, alkyl sulfates and another surfactant or polymers. Preferably metaxalone is milled with mannitol, sodium lauryl sulfate and polyether sulfates. Preferably metaxalone is milled with mannitol, sodium lauryl sulfate and polyethylene glycol 40 stearate. Preferably metaxalone is milled with mannitol, sodium lauryl sulfate and polyethylene glycol 100 stearate. Preferably metaxalone is milled with mannitol, sodium lauryl sulfate and a poloxamer. Preferably metaxalone is milled with mannitol, sodium lauryl sulfate and poloxamer 407. Preferably metaxalone is milled with mannitol, sodium lauryl sulfate and poloxamer 338. Preferably metaxalone is milled with mannitol, sodium lauryl sulfate and poloxamer 188. Preferably metaxalone is milled with mannitol, sodium lauryl sulfate and a solid polyethylene glycol. Preferably metaxalone is milled with mannitol, sodium lauryl sulfate and polyethylene glycol 6000. Preferably metaxalone is milled with mannitol, sodium lauryl sulfate and polyethylene glycol 3000. In another preferred embodiment, Metaxalone is milled with mannitol and polyether sulfates. Preferably metaxalone is milled with mannitol and polyethylene glycol 40 stearate Preferably metaxalone is milled with mannitol and polyethylene glycol 100 stearate In another preferred embodiment metaxalone is milled with mannitol and polyvinyl-pyrrolidine. Preferably metaxalone is milled with mannitol and polyvinyl-pyrrolidone with an approximate molecular weight of 30,000-40,000. In another preferred embodiment, metaxalone is milled with mannitol and alkyl sulfonates. Preferably metaxalone is milled with mannitol and docusate sodium. In another preferred embodiment, metaxalone is milled with mannitol and a surfactant. Preferably metaxalone is milled with mannitol and lecithin. Preferably metaxalone is milled with mannitol and sodium n-lauroyl sarcosine. Preferably metaxalone is milled with mannitol and polyoxyethylene alkyl ether surfactants. Preferably metaxalone is milled with mannitol and PEG 6000.In another preferred formulation metaxalone is milled with mannitol and silica. Preferably metaxalone is milled with mannitol and Aerosil R972 fumed silica. In another preferred embodiment, metaxalone is milled with with mannitol, tartaric acid and sodium lauryl sulfate. In another preferred embodiment, metaxalone is milled with with mannitol, sodium bicarbonate and sodium lauryl sulfate. In another preferred embodiment, metaxalone is milled with mannitol, potassium bicarbonate and sodium lauryl sulfate. In another preferred embodiment, metaxalone is milled with mannitol, sodium bicarbonate and sodium lauryl sulphate and Poloxamer 407. In another preferred embodiment, metaxalone is milled with mannitol, potassium bicarbonate and sodium lauryl sulphate and Poloxamer 407.

The method of the present invention enables production of highly water-soluble biologically active materials. Such materials may exhibit advantages over conventional materials by way of, for example, more rapid therapeutic action or lower dose. In contrast, wet grinding techniques utilizing water (or other comparably polar solvents) are incapable of being applied to such materials, as the particles dissolve appreciably in the solvent.

Other aspects and advantages of the invention will become apparent to those skilled in the art from a review of the ensuing description.

**Brief Description of the Drawings**

Figure 1A. Powder charge composition and particle size distribution of material milled in SPEX mill, examples A to S.

Figure 1B. Powder charge composition and particle size distribution of material milled in SPEX mill, examples T to AL.

Figure 1C. Powder charge composition and particle size distribution of material milled in SPEX mill, examples AM to BE.

Figure 1D. Powder charge composition and particle size distribution of material milled in SPEX mill, examples BF to BX.

Figure 1E. Powder charge composition and particle size distribution of material milled in SPEX mill, examples BY to CQ.

Figure 1F. Powder charge composition and particle size distribution of material milled in SPEX mill, examples CR to DJ.

Figure 1G. Powder charge composition and particle size distribution of material milled in SPEX mill, examples DK to EC.

Figure 1H. The figure shows the X-Ray diffraction patterns: (A) after milling of Naproxen sodium in tartaric acid; (B) unmilled Naproxen sodium and (C) unmilled Naproxen acid.

Figure 2A. Powder charge composition and particle size distribution of material milled in 110 mL HD01 Attritor mill, examples A to F.

Figure 3A. Powder charge composition and particle size distribution of material containing a mixture of 2 matrices, milled in SPEX mill, examples A to E.

Figure 4A. Powder charge composition and particle size distribution of material milled in 1L HD01 Attritor mill, examples A to G.

Figure 5A. Powder charge composition and particle size distribution of material milled in 750mL 1S Attritor mill, examples A to F.

Figure 6A. Powder charge composition and particle size distribution of material milled in ½ Gallon 1S Attritor mill, examples A to R.

Figure 6B. Powder charge composition and particle size distribution of material milled in ½ Gallon 1S Attritor mill, examples S to AK.

Figure 6C. Powder charge composition and particle size distribution of material milled in ½ Gallon 1S Attritor mill, examples AL to AU.

Figure 7A. Powder charge composition and particle size distribution of Metaxalone milled in a variety of mills, examples A to O.

Figure 8A. Powder charge composition and particle size distribution of material milled in HICOM mill, examples A to P.

Figure 9A. Powder charge composition and particle size distribution of material milled in 1½ Gallon 1S Attritor mill, examples A to S.

Figure 9B. Powder charge composition and particle size distribution of material milled in 1½ Gallon 1S Attritor mill, examples T to AL.

Figure 10A. Powder charge composition and particle size distribution of material milled in a variety of large scale mills, examples A to F.

Figure 11A. Powder charge composition and particle size distribution of Naproxen Acid milled in Mannitol in a ½ Gallon 1S Attritor mill, examples A to M.

Figure 12A. Powder charge composition and particle size distribution of Naproxen Acid milled in SPEX mill and particle size distribution after filtration, examples A to L.

**Detailed Description of the Invention**

*General*

The present invention is not to be limited in scope by the specific embodiments described herein, which are intended for the purpose of exemplification only.

The invention described herein may include one or more ranges of values (e.g. size, concentration etc). A range of values will be understood to include all values within the range, including the values defining the range.

Throughout this specification, unless the context requires otherwise, the word "comprise" or variations, such as "comprises" or "comprising" will be understood to imply the inclusion of a stated integer, or group of integers, but not the exclusion of any other integers or group of integers. It is also noted that in this disclosure, and particularly in the claims and/or paragraphs, terms such as "comprises", "comprised", "comprising" and the like can mean "includes", "included", "including", and the like.

"Therapeutically effective amount" as used herein with respect to methods of treatment and in particular drug dosage, shall mean that dosage that provides the specific pharmacological response for which the drug is administered in a significant number of subjects in need of such treatment. It is emphasized that "therapeutically effective amount," administered to a particular subject in a particular instance will not always be effective in treating the diseases described herein, even though such dosage is deemed a "therapeutically effective amount" by those skilled in the art. It is to be further understood that drug dosages are, in particular instances, measured as oral dosages, or with reference to drug levels as measured in blood.

The term "inhibit" is defined to include its generally accepted meaning which includes prohibiting, preventing, restraining, and lowering, stopping, or reversing progression or severity, and such action on a resultant symptom. As such the present invention includes both medical therapeutic and prophylactic administration, as appropriate.

The term "biologically active material" is defined to mean a biologically active compound or a substance which comprises a biologically active compound. In this definition, a compound is generally taken to mean a distinct chemical entity where a chemical formula or formulas can be used to describe the substance. Such compounds would generally, but not necessarily be identified in the literature by a unique classification system such as a CAS number. Some compounds may be more complex and have a mixed chemical structure. For such compounds they may only have an empirical formula or be qualitatively identified. A compound would generally be a pure material, although it would be expected that up to 10%, 20%, 30%, 40%, 50%, 60%, 70%, 80%, 90% of the substance could be other impurities and the like. Examples of biologically active compounds are, but not limited to, pharmaceutical actives, homologs and first order derivatives thereof. A substance that contains a biologically active compound is any substance which has as one of its components a biologically active compound. Examples of substances containing biologically active compounds are, but not limited to, pharmaceutical formulations and products.

Any of the terms, "biological(ly) active", "active", "active material" shall have the same meaning as biologically active material.

The term "grinding matrix" is defined as any inert substance that a biologically active material can or is combined with and milled. The terms "co-grinding matrix" and "matrix" are interchangeable with "grinding matrix".

***Particle Size***

There are a wide range of techniques that can be utilized to characterize the particle size of a material. Those skilled in the art also understand that almost all these techniques do not physically measure the actually particle size, as one might measure something with a ruler, but measure a physical phenomena which is interpreted to indicate a particle size. As part of the interpretation process some assumptions need to be made to enable mathematical calculations to be made. These assumptions deliver results such as an equivalent spherical particle size, or a hydrodynamic radius.

Amongst these various methods, two types of measurements are most commonly used. Photon correlation spectroscopy (PCS), also known as 'dynamic light scattering' (DLS) is commonly used to measure particles with a size less than 10 micrometer. Typically this measurement yields an equivalent hydrodynamic radius often expressed as the average size of a number distribution. The other common particle size measurement is laser diffraction which is commonly used to measure particle size from 100 nm to 2000 micrometer. This technique calculates a volume distribution of equivalent spherical particles that can be expressed using descriptors such as the median particle size or the % of particles under a given size.

Those skilled in the art recognize that different characterization techniques such as photon correlation spectroscopy and laser diffraction measure different properties of a particle ensemble. As a result multiple techniques will give multiple answers to the question, "what is the particle size." In theory one could convert and compare the various parameters each technique measures, however, for real world particle systems this is not practical. As a result the particle size used to describe this invention will be given as two different sets of values that each relate to these two common measurement techniques, such that measurements could be made with either technique and then evaluated against the description of this invention.

For measurements made using a photo correlation spectroscopy instrument, or an equivalent method known in the art, the term "number average particle size" is defined as the average particle diameter as determined on a number basis.

For measurements made using a laser diffraction instrument, or an equivalent method known in the art, the term "median particle size" is defined as the median particle diameter as determined on an equivalent spherical particle volume basis. Where the term median is used, it is understood to describe the particle size that divides the population in half such that 50 % of the population is greater than or less than this size. The median particle size is often written as D50, D(0.50) or D[0.5] or similar. As used herein D50, D(0.50) or D[0.5] or similar shall be taken to mean 'median particle size'.

The term "Dx of the particle size distribution" refers to the xth percentile of the distribution; thus, D90 refers to the 90th percentile, D95 refers to the 95th percentile, and so forth. Taking D90 as an example this can often be written as, D(0.90) or D[0.9] or simialr. With respect to the median particle size and Dx an upper case D or lowercase d are interchangeable and have the same meaning.

Another commonly used way of describing a particle size distribution measured by laser diffraction, or an equivalent method known in the art, is to describe what % of a distribution is under or over a nominated size. The term "percentage less than" also written as "%<" is defined as the percentage, by volume, of a particle size distribution under a nominated size -for example the % < 1000 nm. The term "percentage greater than" also written as "%>" is defined as the percentage, by volume, of a particle size distribution over a nominated size -for example the % > 1000 nm.

The particle size used to describe this invention should be taken to mean the particle size as measured at or shortly before the time of use. For example, the particle size is measured 2 months after the material is subject to the milling method of this invention. In a preferred form, the particle size is measured at a time selected from the group consisting of: 1 day after milling, 2 days after milling, 5 days after milling, 1 month after milling, 2 months after milling, 3 months after milling, 4 months after milling, 5 months after milling, 6 months after milling, 1 year after milling, 2 years after milling, 5 years after milling.

For many of the materials subject to the methods of this invention the particle size can be easily measured. Where the active material has poor water solubility and the matrix it is milled in has good water solubility the powder can simply be dispersed in an aqueous solvent. In this scenario the matrix dissolves leaving the active material dispersed in the solvent. This suspension can then be measured by techniques such as PCS or laser diffraction.

Suitable methods to measure an accurate particle size where the active material has substantive aqueous solubility or the matrix has low solubility in a water based dispersant are outlined below.

1. In the circumstance where insoluble matrix such as microcrystalline cellulose prevents the measurement of the active material separation techniques such as filtration or centrifugation could be used to separate the insoluble matrix from the active material particles. Other ancillary techniques would also be required to determine if any active material was removed by the separation technique so that this could be taken into account.

2. In the case where the active material is too soluble in water other solvents could be evaluated for the measurement of particle size. Where a solvent could be found that active material is poorly soluble in but is a good solvent for the matrix a measurement would be relatively straight forward. If such a solvent is difficult to find another approach would be to measure the ensemble of matrix and active material in a solvent (such as iso-octane) which both are insoluble in. Then the powder would be measured in another solvent where the active material is soluble but the matrix is not. Thus with a measurement of the matrix particle size and a measurement of the size of the matrix and active material together an understanding of the active material particle size can be obtained.

3. In some circumstances image analysis could be used to obtain information about the particle size distribution of the active material. Suitable image measurement techniques might include transmission electron microscopy (TEM), scanning electron microscopy (SEM), optical microscopy and confocal microscopy. In addition to these standard techniques some additional technique would be required to be used in parallel to differentiate the active material and matrix particles. Depending on the chemical makeup of the materials involved possible techniques could be elemental analysis, raman spectroscopy, FTIR spectroscopy or fluorescence spectroscopy.

### Other Definitions

Throughout this specification, unless the context requires otherwise, the phrase "dry mill" or variations, such as "dry milling", should be understood to refer to milling in at least the substantial absence of liquids. If liquids are present, they are present in such amounts that the contents of the mill retain the characteristics of a dry powder.

"Flowable" means a powder having physical characteristics rendering it suitable for further processing using typical equipment used for the manufacture of pharmaceutical compositions and formulations.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

The term "millable" means that the grinding matrix is capable of being physically degraded under the dry milling conditions of the method of the invention. In one embodiment of the invention, the milled grinding matrix is of a comparable particle size to the biologically active material. In another embodiment of the invention the particle size of the matrix is substantially reduced but not as small as the biologically active material

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

*Specific*

In one embodiment, the present invention is directed to a method for producing a composition, comprising the steps of: dry milling a solid biologically active material and a millable grinding matrix in a mill comprising a plurality of milling bodies, for a time period sufficient to produce particles of the biologically active material dispersed in an at least partially milled grinding material, wherein the composition produced by said method comprises particles of the biologically active compound at or above a volume fraction of 25 v/v%.

The mixture of active material and matrix may then be separated from the milling bodies and removed from the mill.

In one aspect the mixture of active material and matrix is then further processed. In another aspect, the grinding matrix is separated from the particles of biologically active material. In a further aspect, at least a portion of the milled grinding matrix is separated from the particulate biologically active material.

The milling bodies are essentially resistant to fracture and erosion in the dry milling process. The quantity of the grinding matrix relative to the quantity of biologically active material in particulate form, and the extent of milling of the grinding matrix, is sufficient to inhibit re-agglomeration of the particles of the active material.

The present invention also relates to biologically active materials produced by said methods, to medicaments produced using said biologically active materials and to methods of treatment of an animal, including man, using a therapeutically effective amount of said biologically active materials administered by way of said medicaments.

### Increasing the volume fraction load

The present invention is directed to the unexpected finding that particles of a biologically active material can be produced by dry milling processes wherein the composition produced by said method comprises particles of the biologically active compound at or above a volume fraction of 25 v/v%. In one surprising aspect the particle size produced by the process is equal to or less than 2000nm. In another surprising aspect the particle size produced by the process is equal to or less than 1000nm. This can result in a more efficient and cost effective process.

### Improving the dissolution profile

The process results in the biologically active material having an improved dissolution profile. An improved dissolution profile has significant advantages including the improvement of bioavailability of the biologically active material *in vivo.* Preferably, the improved dissolution profile is observed *in vitro.* Alternatively, the improved dissolution profile is observed *in vivo* by the observation of an improved bioavailability profile. Standard methods for determining the dissolution profile of a material *in vitro* are available in the art. A suitable method to determine an improved dissolution profile *in vitro* may include determining the concentration of the sample material in a solution over a period of time and comparing the results from the sample material to a control sample. An observation that peak solution concentration for the sample material was achieved in less time than the control sample would indicate (assuming it is statistically significant), that the sample material has an improved dissolution profile. The measurement sample is herein defined as the mixture of biologically active material with grinding matrix and/or other additives that has been subject to the processes of the invention described here. Herein a control sample is defined as a physical mixture (not subject to the processes described in this invention) of the components in the measurement sample with the same relative proportions of active, matrix and/or additive as the measurement sample. For the purposes of the dissolution testing a prototype formulation of the measurement sample could also be used. In this case the control sample would be formulated in the same way. Standard methods for determining the improved dissolution profile of a material *in vivo* are available in the art. A suitable method to determine an improved dissolution profile in a human may be after delivering the dose to measure the rate of active material absorption by measuring the plasma concentration of the sample compound over a period of time and comparing the results from the sample compound to a control. An observation that peak plasma concentration for the sample compound was achieved in less time than the control would indicate (assuming it is statistically significant) that the sample compound has improved bioavailability and an improved dissolution profile. Preferably, the improved dissolution profile is observed at a relevant gastrointestinal pH, when it is observed *in vitro.* Preferably, the improved dissolution profile is observed at a pH which is favourable at indicating improvements in dissolution when comparing the measurement sample to the control compound. Suitable methods for quantifying the concentration of a compound in an *in vitro* sample or an *in vivo* sample are widely available in the art. Suitable methods could include the use of spectroscopy or radioisotope labeling. In one preferred embodiment the method of quantification of dissolution is determined in a solution with a pH selected from the group consisting of: pH 1, pH 2, pH 3, pH 4, pH 5, pH 6, pH 7, pH 7.3, pH 7.4, pH 8, pH 9, pH 10, pH 11, pH 12, pH 13, pH 14 or a pH with 0.5 of a pH unit of any of this group.

***Crystallization Profile***

Methods for determining the crystallinity profile of the biologically active material are widely available in the art. Suitable methods may include X-ray diffraction, differential scanning calorimetry, raman or IR spectrocopy.

***Amorphicity Profile***

Methods for determining the amorphous content of the biologically active material are widely available in the art. Suitable methods may include X-ray diffraction, differential scanning calorimetry, raman or IR spectroscopy.

***Grinding Matrix***

As will be described subsequently, selection of an appropriate grinding matrix affords particular advantageous applications of the method of the present invention.

A highly advantageous application of the method of the invention is the use of a water-soluble grinding matrix in conjunction with a poorly water-soluble biologically active material. This affords at least two advantages. The first being when the powder containing the biologically active material is placed into water - such as the ingestion of the powder as part of an oral medication - the matrix dissolves, releasing the particulate active material such that there is maximum surface area exposed to solution, thereby allowing a rapid dissolution of the active compound. The second key advantage is the ability, if required, to remove or partially remove the matrix prior to further processing or formulation.

Another advantageous application of the method of the invention is the use of a water-insoluble grinding matrix, particularly in the area of agricultural use, when a biologically active material such as a fungicide is commonly delivered as part of a dry powder or a suspension. The presence of a water insoluble matrix will afford benefits such as increased rain fastness. Without wishing to be bound by theory, it is believed that the physical degradation (including but not limited to particle size reduction) of the millable grinding matrix affords the advantage of the invention, by acting as a more effective diluent than grinding matrix of a larger particle size. Again, as will be described subsequently, a highly advantageous aspect of the present invention is that certain grinding matrixes appropriate for use in the method of the invention are also appropriate for use in a medicament. The present invention encompasses methods for the production of a medicament incorporating both the biologically active material and the grinding matrix or in some cases the biologically active material and a portion of the grinding matrix, medicaments so produced, and methods of treatment of an animal, including man, using a therapeutically effective amount of said biologically active materials by way of said medicaments.

The medicament may include only the biologically active material together with the milled grinding matrix or, more preferably, the biologically active material and milled grinding matrix may be combined with one or more pharmaceutically acceptable carriers, as well as any desired excipients or other like agents commonly used in the preparation of medicaments. Analogously, the agricultural chemical composition may include only the biologically active material together with the milled grinding matrix or, more preferably, the biologically active materials and milled grinding matrix may be combined with one or more carriers, as well as any desired excipients or other like agents commonly used in the preparation of agricultural chemical compositions.

In one particular form of the invention, the grinding matrix is both appropriate for use in a medicament and readily separable from the biologically active material by methods not dependent on particle size. Such grinding matrixes are described in the following detailed description of the invention. Such grinding matrixes are highly advantageous in that they afford significant flexibility in the extent to which the grinding matrix may be incorporated with the biologically active material into a medicament.

In a highly preferred form, the grinding matrix is harder than the biologically active material, and is thus capable of reducing the particle size of the active material under the dry milling conditions of the invention. Again without wishing to be bound by theory, under these circumstances it is believed that the millable grinding matrix affords the advantage of the present invention through a second route, with the smaller particles of grinding matrix produced under the dry milling conditions enabling greater interaction with the biologically active material. The quantity of the grinding matrix relative to the quantity of biologically active material, and the extent of physical degradation of the grinding matrix, is sufficient to improve to inhibit re-agglomeration of the particles of the active material. Preferably, the quantity of the grinding matrix relative to the quantity of biologically active material, and the extent of physical degradation of the grinding matrix, is sufficient to inhibit re-agglomeration of the particles of the active material in nanoparticulate form.

The grinding matrix is not generally selected to be chemically reactive with the biologically active material under the milling conditions of the invention, excepting for example, where the matrix is deliberately chosen to undergo a mechanico-chemical reaction. Such a reaction might be the conversion of a free base or acid to a salt or vice versa.

As stated above, the method of the present invention requires the grinding matrix to be milled with the biologically active material; that is, the grinding matrix will physically degrade under the dry milling conditions of the invention to facilitate the formation and retention of particulates of the biologically active material with reduced particle size. The precise extent of degradation required will depend on certain properties of the grinding matrix and the biologically active material, the ratio of biologically active material to grinding matrix, and the particle size distribution of the particles comprising the biologically active material.

The physical properties of the grinding matrix necessary to achieve the requisite degradation are dependent on the precise milling conditions. For example, a harder grinding matrix may degrade to a sufficient extent provided it is subjected to more vigorous dry milling conditions. Physical properties of the grinding matrix relevant to the extent that the agent will degrade under dry milling conditions include hardness, friability, as measured by indicia such as hardness, fracture toughness and brittleness index.

A low hardness (typically a Mohs Hardness less than 7) of the biologically active material is desirable to ensure fracture of the particles during processing, so that composite microstructures develop during milling. Preferably, the hardness is less than 3 as determined using the Mohs Hardness scale.

Preferably, the grinding matrix is of low abrasivity. Low abrasivity is desirable to minimise contamination of the mixture of the biologically active material in the grinding matrix by the milling bodies and/or the milling chamber of the media mill. An indirect indication of the abrasivity can be obtained by measuring the level of milling-based contaminants.

Preferably, the grinding matrix has a low tendency to agglomerate during dry milling. While it is difficult to objectively quantify the tendency to agglomerate during milling, it is possible to obtain a subjective measure by observing the level of "caking" of the grinding matrix on the milling bodies and the milling chamber of the media mill as dry milling progresses.

The grinding matrix may be an inorganic or organic substance.

In one embodiment, the grinding matrix is selected from the following, either as a single substance or a combination of two or more substances: Polyols (sugar alcohols) for example (but not limited to) mannitol, sorbitol, isomalt, xylitol, maltitol, lactitol, erythritol, arabitol, ribitol, monosaccharides for example (but not limited to) glucose, fructose, mannose, galactose, disaccharides and trisaccharides for example (but not limited to) anhydrous lactose, lactose monohydrate, sucrose, maltose, trehalose, polysaccharides for example (but not limited to) maltodextrins, dextrin, Inulin, dextrates, polydextrose, other carbohyrates for example (but not limited to) starch, wheat flour, corn flour, rice flour, rice starch, tapioca flour, tapioca starch, potato flour, potato starch, other flours and starches, , soy flour, soy meal or other soy products, cellulose, microcrystalline cellulose, microcrystalline cellulose based co blended excipients, chemically modified excipients such as pregelatinized (or partially) starch, modified celluloses such as HPMC, CMC, HPC, enteric polymer coatings such as hypromellose phthalate, cellulose acetate phthalate (Aquacoat®), polyvinyl acetate phthalate (Sureteric®), hypromellose acetate succinate (AQOAT®), and polmethacrylates (Eudragit® and Acryl-EZE®), Milk products for example (but not limited to) milk powder, skim milk powders, other milk solids and derivatives, other functional Excipients, organic acids for example (but not limited to) citric acid, tartaric acid, malic acid, maleic acid fumaric acid , ascorbic acid, succinic acid, the conjugate salt of organic acids for example (but not limited to) sodium citrate, sodium tartrate, sodium malate, sodium ascorbate, potassium citrate, potassium tartrate, potassium malate, potassium ascorbate, inorganics such as sodium carbonate, potassium carbonate, magnesium carbonate, sodium bicarbonate, potassium bicarbonate and calcium carbonate. dibasic calcium phosphate, tribasic calcium phosphate, sodium sulfate, sodium chloride, sodium metabisulphite, sodium thiosulfate, ammonium chloride, Glauber's salt, ammonium carbonate, sodium bisulfate, magnesium sulfate, potash alum, potassium chloride, sodium hydrogen sulfate, sodium hydroxide, crystalline hydroxides, hydrogen carbonates, hydrogen carbonates of pharmaceutical acceptable alkali metals, such as but not limited by, sodium, potassium, lithium, calcium, and barium, ammonium salts (or salts of volatile amines), for example (but not limited to) ammonium chloride, methylamine hydrochloride, ammonium bromide, other inorganics for example (but not limited to), thermal silica, chalk, mica, silica, alumina, titanium dioxide, talc, kaolin, bentonite, hectorite, magnesium trisilicate, other clay or clay derivatives or aluminium silicates, a surfactant for example (but not limited to) sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate , glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, , poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Soidum Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend,Calcium Dodecylbenzene Sulfonate, Sodium Dodecylbenzene Sulfonate,Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines.

In a preferred embodiment, the grinding matrix is a matrix that is considered GRAS (generally regarded as safe) by persons skilled in the pharmaceutical arts.

In another preferred aspect a combination of two or more suitable matrices, such as those listed above, can be used as the grinding matrix to provide improved properties such as the reduction of caking, and greater improvement of particle size reduction. Combination matrices may also be advantageous when the matrices have different solubility's allowing the removal or partial removal of one matrix, while leaving the other or part of the other to provide encapsulation or partial encapsulation of the biologically active material.

Another highly preferred aspect of the method is the inclusion of a suitable milling aid in the matrix to improve milling performance. Improvements to milling performance would be things such as, but not limited to, a reduction in caking or higher recovery of powder from the mill. Examples of suitable milling aids include surfactants, polymers and inorganics such as silica (including colloidal silica), aluminium silicates and clays.

There are a wide range of surfactants that will make suitable milling aids. The highly preferred form is where the surfactant is a solid, or can be manufactured into a solid. Preferably, the surfactant is selected from the group consisting of: polyoxyethylene alkyl ethers, polyoxyethylene stearates, polyethylene glycols (PEG), poloxamers, poloxamines, sarcosine based surfactants, polysorbates, aliphatic alcohols, alkyl and aryl sulfates, alkyl and aryl polyether sulfonates and other sulfate surfactants, trimethyl ammonium based surfactants, lecithin and other phospholipids, bile salts, polyoxyethylene castor oil derivatives, polyoxyethylene sorbitan fatty acid esters, Sorbitan fatty acid esters, Sucrose fatty acid esters, alkyl glucopyranosides, alkyl maltopyranosides, glycerol fatty acid esters, Alkyl Benzene Sulphonic Acids, Alkyl Ether Carboxylic Acids, Alkyl and aryl Phosphate esters, Alkyl and aryl Sulphate esters, Alkyl and aryl Sulphonic acids, Alkyl Phenol Phosphates esters, Alkyl Phenol Sulphates esters, Alkyl and Aryl Phosphates, Alkyl Polysaccharides, Alkylamine Ethoxylates, Alkyl-Naphthalene Sulphonates formaldehyde condensates, Sulfosuccinates, lignosulfonates, Ceto-Oleyl Alcohol Ethoxylates, Condensed Naphthalene Sulphonates, Dialkyl and Alkyl Naphthalene Sulphonates,Di-alkyl Sulphosuccinates, Ethoxylated nonylphenols, Ethylene Glycol Esters,Fatty Alcohol Alkoxylates, Hydrogenated tallowalkylamines, Mono-alkyl Sulphosuccinamates, Nonyl Phenol Ethoxylates, Sodium Oleyl N-methyl Taurate, Tallowalkylamines, linear and branched dodecylbenzene sulfonic acidsPreferably, the surfactant is selected from the group consisting of: sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate , glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, , poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, Sucrose Palmitate, Sucrose Stearate, Sucrose Distearate, Sucrose laurate, Glycocholic acid, sodium Glycholate, Cholic Acid, Soidum Cholate, Sodium Deoxycholate, Deoxycholic acid, Sodium taurocholate, taurocholic acid, Sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG10000, PEG20000, alkyl naphthalene sulfonate condensate/Lignosulfonate blend,Calcium Dodecylbenzene Sulfonate, Sodium Dodecylbenzene Sulfonate,Diisopropyl naphthaenesulphonate, erythritol distearate, Naphthalene Sulfonate Formaldehyde Condensate, nonylphenol ethoxylate (poe-30), Tristyrylphenol Ethoxylate, Polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, Sodium Methyl Naphthalene Formaldehyde Sulfonate, sodium n-butyl naphthalene sulfonate, tridecyl alcohol ethoxylate (poe-18), Triethanolamine isodecanol phosphate ester, Triethanolamine tristyrylphosphate ester, Tristyrylphenol Ethoxylate Sulfate, Bis(2-hydroxyethyl)tallowalkylamines.

Preferably the polymer is selected from the list of: polyvinylpyrrolidones (PVP), polyvinylalcohol, Acrylic acid based polymers and copolymers of acrylic acid

Preferably, the milling aid has a concentration selected from the group consisting of: 0.1 -10 % w/w, 0.1 -5 % w/w, 0.1 -2.5 % w/w, of 0.1 - 2% w/w, 0.1 -1 %, 0.5 -5% w/w, 0.5 -3% w/w, 0.5 - 2% w/w, 0.5 - 1.5%, 0.5 -1 % w/w, of 0.75 - 1.25 % w/w, 0.75 -1% and 1% w/w.

## Milling bodies

In the method of the present invention, the milling bodies are preferably chemically inert and rigid. The term "chemically-inert", as used herein, means that the milling bodies do not react chemically with the biologically active material or the grinding matrix.

As described above, the milling bodies are essentially resistant to fracture and erosion in the milling process.

The milling bodies are desirably provided in the form of bodies which may have any of a variety of smooth, regular shapes, flat or curved surfaces, and lacking sharp or raised edges. For example, suitable milling bodies can be in the form of bodies having ellipsoidal, ovoid, spherical or right cylindrical shapes. Preferably, the milling bodies are provided in the form of one or more of beads, balls, spheres, rods, right cylinders, drums or radius-end right cylinders (i.e., right cylinders having hemispherical bases with the same radius as the cylinder).

Depending on the nature of the biologically active material and the grinding matrix, the milling media bodies desirably have an effective mean particle diameter (i.e. "particle size") between about 0.1 and 30 mm, more preferably between about 1 and about 15 mm, still more preferably between about 3 and 10 mm.

The milling bodies may comprise various substances such as ceramic, glass, metal or polymeric compositions, in a particulate form. Suitable metal milling bodies are typically spherical and generally have good hardness (i.e. RHC 60-70), roundness, high wear resistance, and narrow size distribution and can include, for example, balls fabricated from type 52100 chrome steel, type 316 or 440C stainless steel or type 1065 high carbon steel.

Preferred ceramics, for example, can be selected from a wide array of ceramics desirably having sufficient hardness and resistance to fracture to enable them to avoid being chipped or crushed during milling and also having sufficiently high density. Suitable densities for milling media can range from about 1 to 15 g/cm$^3$, preferably from about 1 to 8 g/cm$^3$. Preferred ceramics can be selected from steatite, aluminum oxide, zirconium oxide, zirconia-silica, yttria-stabilized zirconium oxide, magnesia-stabilized zirconium oxide, silicon nitride, silicon carbide, cobalt-stabilized tungsten carbide, and the like, as well as mixtures thereof.

Preferred glass milling media are spherical (e.g. beads), have a narrow size distribution, are durable, and include, for example, lead-free soda lime glass and borosilicate glass. Polymeric milling media are preferably substantially spherical and can be selected from a wide array of polymeric resins having sufficient hardness and friability to enable them to avoid being chipped or crushed during milling, abrasion-resistance to minimize attrition resulting in contamination of the product, and freedom from impurities such as metals, solvents, and residual monomers. Preferred polymeric resins, for example, can be selected from crosslinked polystyrenes, such as polystyrene crosslinked with divinylbenzene, styrene copolymers, polyacrylates such as polymethylmethacrylate, polycarbonates, polyacetals, vinyl chloride polymers and copolymers, polyurethanes, polyamides, high density polyethylenes, polypropylenes, and the like. The use of polymeric milling media to grind materials down to a very small particle size (as opposed to mechanochemical synthesis) is disclosed, for example, in U.S. patents 5,478,705 and 5,500,331. Polymeric resins typically can have densities ranging from about 0.8 to 3.0 g/cm$^3$. Higher density polymeric resins are preferred. Alternatively, the milling media can be composite particles comprising dense core particles having a polymeric resin adhered thereon. Core particles can be selected from substances known to be useful as milling media, for example, glass, alumina, zirconia silica, zirconium oxide, stainless steel, and the like. Preferred core substances have densities greater than about 2.5 g/cm$^3$.

In one embodiment of the invention, the milling media are formed from a ferromagnetic substance, thereby facilitating removal of contaminants arising from wear of the milling media by the use of magnetic separation techniques.

Each type of milling body has its own advantages. For example, metals have the highest specific gravities, which increase grinding efficiency due to increased impact energy. Metal costs range from low to high, but metal contamination of final product can be an issue. Glasses are advantageous from the standpoint of low cost and the availability of small bead sizes as low as 0.004 mm. However, the specific gravity of glasses is lower than other media and significantly more milling time is required. Finally, ceramics are advantageous from the standpoint of low wear and contamination, ease of cleaning, and high hardness.

*Dry Milling*

In the dry milling process of the present invention, the metaxalone and grinding matrix, in the form of crystals, powders, or the like, are combined in suitable proportions with the plurality of milling bodies in a milling chamber that is mechanically agitated (i.e. with or without stirring) for a predetermined period of time at a predetermined intensity of agitation. Typically, a milling apparatus is used to impart motion to the milling bodies by the external application of agitation, whereby various translational, rotational or inversion motions or combinations thereof are applied to the milling chamber and its contents, or by the internal application of agitation through a rotating shaft terminating in a blade, propeller, impeller or paddle or by a combination of both actions.

During milling, motion imparted to the milling bodies can result in application of shearing forces as well as multiple impacts or collisions having significant intensity between milling bodies and particles of the biologically active material and grinding matrix. The nature and intensity of the forces applied by the milling bodies to the biologically active material and the grinding matrix is influenced by a wide variety of processing parameters including: the type of milling apparatus; the intensity of the forces generated, the kinematic aspects of the process; the size, density, shape, and composition of the milling bodies; the weight ratio of the biologically active material and grinding matrix mixture to the milling bodies; the duration of milling; the physical properties of both the biologically active material and the grinding matrix; the atmosphere present during activation; and others.

Advantageously, the media mill is capable of repeatedly or continuously applying mechanical compressive forces and shear stress to the biologically active material and the grinding matrix. Suitable media mills include but are not limited to the following: high-energy ball, sand, bead or pearl mills, basket mill, planetary mill, vibratory action ball mill, multi-axial shaker/mixer, stirred ball mill, horizontal small media mill, multi-ring pulverizing mill, and the like, including small milling media. The milling apparatus also can contain one or more rotating shafts.

In a preferred form of the invention, the dry milling is performed in a ball mill. Throughout the remainder of the specification reference will be made to dry milling being carried out by way of a ball mill. Examples of this type of mill are attritor mills, nutating mills, tower mills, planetary mills, vibratory mills and gravity-dependent-type ball mills. It will be appreciated that dry milling in accordance with the method of the invention may also be achieved by any suitable means other than ball milling. For example, dry milling may also be achieved using jet mills, rod mills, roller mills or crusher mills.

*Biologically active material*

The biologically active material for use in the invention is metaxalone. As discussed in the context of the background to the invention, biologically active materials that are poorly water soluble at gastrointestinal pH will particularly benefit from being prepared, and the method of the present invention is particularly advantageously applied to materials that are poorly water soluble at gastrointestinal pH.

Conveniently, the biologically active material is capable of withstanding temperatures that are typical in uncooled dry milling, which may exceed 80 °C. Therefore, materials with a melting point about 80 °C or greater are highly suitable. For biologically active materials with lower melting points, the media mill may be cooled, thereby allowing materials with significantly lower melting temperatures to be processed according to the method of the invention. For instance, a simple water-cooled mill will keep temperatures below 50 °C, or chilled water could be used to further lower the milling temperature. Those skilled in the art will understand that a high energy ball mill could be designed to run at any temperature between say -30 to 200 °C. For some biologically active materials it may be advantageous to control the milling temperature to temperatures significantly below the melting points of the biologically active materials.

The biologically active material is obtained in a conventional form commercially and/or prepared by techniques known in the art.

It is preferred, but not essential, that the particle size of the biologically active material be less than about 1000 $\mu$m, as determined by sieve analysis. If the coarse particle size of the biologically active material is greater than about 1000 $\mu$m, then it is preferred that the particles of the biologically active material substrate be reduced in size to less than 1000 $\mu$m using another standard milling method.

*Processed biologically active material*

Preferably, the biologically active materials, which have been subject to the methods of the invention, comprises particles of biologically active material of an average particle size, determined on a particle number basis, is equal to or less than a size selected from the group 2000 nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm and 100 nm. The biologically active materials, which have been subject to the methods of the invention, comprises particles of biologically active material of a median particle size, determined on a particle volume basis, equal or less than 500nm, 400 nm, 300nm, 200nm and 100 nm. Preferably, the biologically active materials, which have been subject to the methods of the invention, comprises particles of biologically active material and wherein the Dx of the particle size distribution, as measured on a particle volume basis, is selected from the group consisting of less than or equal to 10,000nm, 5000nm, 3000nm, 2000nm, 1900 nm, 1800nm, 1700nm, 1600nm, 1500nm, 1400nm, 1300nm, 1200 nm, 1100nm, 1000nm, 900nm, 800nm, 700nm, 600nm, 500nm, 400 nm, 300nm, 200nm, and 100 nm; wherein x is greater than or equal to 90, These sizes refer to particles either fully dispersed or partially agglomerated.

### Processing Time

Preferably, the biologically active material and the grinding matrix are dry milled for the shortest time necessary to form the mixture of the biologically active material in the grinding matrix such that the active material has improved dissolution to minimise any possible contamination from the media mill and/or the plurality of milling bodies. This time varies greatly, depending on the biologically active material and the grinding matrix, and may range from as short as 1 minute to several hours. Dry milling times in excess of 2 hours may lead to degradation of the biologically active material and an increased level of undesirable contaminants.

Suitable rates of agitation and total milling times are adjusted for the type and size of milling apparatus as well as the milling media, the weight ratio of the biologically active material and grinding matrix mixture to the plurality of milling bodies, the chemical and physical properties of the biologically active material and grinding matrix, and other parameters that may be optimized empirically.

### Inclusion of the grinding matrix with the biologically active material and separation of the grinding matrix from the biologically active material

In a preferred aspect, the grinding matrix is not separated from the biologically active material but is maintained with the biologically active material in the final product. Preferably the grinding matrix is considered to be Generally Regarded as Safe (GRAS) for pharmaceutical products.

In an alternative aspect, the grinding matrix is separated from the biologically active material. In one aspect, where the grinding matrix is not fully milled, the unmilled grinding matrix is separated from the biologically active material. In a further aspect, at least a portion of the milled grinding matrix is separated from the biologically active material.

Any portion of the grinding matrix may be removed, including but not limited to 10%, 25%, 50%, 75%, or substantially all of the grinding matrix.

In some embodiments of the invention, a significant portion of the milled grinding matrix may comprise particles of a size similar to and/or smaller than the particles comprising the biologically active material. Where the portion of the milled grinding matrix to be separated from the particles comprising the biologically active material comprises particles of a size similar to and/or smaller than the particles comprising the biologically active material, separation techniques based on size distribution are inapplicable.

In these circumstances, the method of the present invention may involve separation of at least a portion of the milled grinding matrix from the biologically active material by techniques including but not limited to electrostatic separation, magnetic separation, centrifugation (density separation), hydrodynamic separation, froth flotation.

Advantageously, the step of removing at least a portion of the milled grinding matrix from the biologically active material may be performed through means such as selective dissolution, washing, or sublimation.

An advantageous aspect of the invention would be the use of grinding matrix that has two or more components where at least one component is water soluble and at least one component has low solubility in water. In this case washing can be used to remove the matrix component soluble in water leaving the biologically active material encapsulated in the remaining matrix components. In a highly advantageous aspect of the invention the matrix with low solubility is a functional excipient.

A highly advantageous aspect of the present invention is that certain grinding matrixes appropriate for use in the method of the invention (in that they physically degrade to the desired extent under dry milling conditions) are also pharmaceutically acceptable and thus appropriate for use in a medicament. Where the method of the present invention does not involve complete separation of the grinding matrix from the biologically active material, the present invention encompasses methods for the production of a medicament incorporating both the biologically active material and at least a portion of the milled grinding matrix, medicaments so produced and methods of treatment of an animal, including man, using a therapeutically effective amount of said biologically active materials by way of said medicaments.

The medicament may include only the biologically active material and the grinding matrix or, more preferably, the biologically active materials and grinding matrix may be combined with one or more pharmaceutically acceptable carriers, as well as any desired excipients or other like agents commonly used in the preparation of medicaments.

Analogously, a highly advantageous aspect of the present invention is that certain grinding matrixes appropriate for use in the method of the invention (in that they physically degrade to a desirable extent under dry milling conditions) are also appropriate for use in an agricultural chemical composition. Where the method of the present invention does not involve complete separation of the grinding matrix from the biologically active material, the present invention encompasses methods for the production of a agricultural chemical composition incorporating both the biologically active material and at least a portion of the milled grinding matrix, agricultural chemical composition so produced and methods of use of such compositions.

The agricultural chemical composition may include only the biologically active material and the grinding matrix or, more preferably, the biologically active materials and grinding matrix may be combined with one or more acceptable carriers, as well as any desired excipients or other like agents commonly used in the preparation of agricultural chemical compositions.

In one particular form of the invention, the grinding matrix is both appropriate for use in a medicament and readily separable from the biologically active material by methods not dependent on particle size. Such grinding matrixes are described in the following detailed description of the invention. Such grinding matrixes are highly advantageous in that they afford significant flexibility in the extent to which the grinding matrix may be incorporated with the biologically active material into a medicament.

The mixture of biologically active material and grinding matrix may then be separated from the milling bodies and removed from the mill.

In one embodiment, the grinding matrix is separated from the mixture of biologically active material and grinding matrix. Where the grinding matrix is not fully milled, the unmilled grinding matrix is separated from the biologically active material. In a further aspect, at least a portion of the milled grinding matrix is separated from the biologically active material.

The milling bodies are essentially resistant to fracture and erosion in the dry milling process. The quantity of the grinding matrix relative to the quantity of biologically active material, and the extent of milling of the grinding matrix, is sufficient to provide reduced particle size of the biologically active material.

The grinding matrix is neither chemically nor mechanically reactive with the pharmaceutical material under the dry milling conditions of the method of the invention except, for example, where the matrix is deliberately chosen to undergo a mechanico-chemical reaction. Such a reaction might be the conversion of a free base or acid to a salt or vice versa.

Preferably, the medicament is a solid dosage form, however, other dosage forms may be prepared by those of ordinary skill in the art.

In one form, after the step of separating said mixture of biologically active material and grinding matrix from the plurality of milling bodies, and before the step of using said mixture of biologically active material and grinding matrix in the manufacture of a medicament, the method may comprise the step of:

> removing a portion of the grinding matrix from said mixture of biologically active material and grinding matrix to provide a mixture enriched in the biologically active material;
> and the step of using said mixture of biologically active material and grinding matrix in the manufacture of a medicament, more particularly comprises the step of using the mixture of biologically active material and grinding matrix enriched in the biologically active material form in the manufacture of a medicament.

In another embodiment of the invention, a facilitating agent or a combination of facilitating agents is also comprised in the mixture to be milled. Such facilitating agents appropriate for use in the invention include diluents, surfactants, polymers, binding agents, filling agents, lubricating agents, sweeteners, flavouring agents, preservatives, buffers, wetting agents, disintegrants, effervescent agents and agents that may form part of a medicament, including a solid dosage form, or other excipients required for other specific drug delivery, such as the agents and media listed below under the heading *Medicinal and Pharmaceutical Compositions,* or any combination thereof.

### *Medicaments*

The medicaments may include the pharmaceutically acceptable material, optionally together with the grinding matrix or at least a portion of the grinding matrix, with or without milling aids, facilitating agents, combined with one or more pharmaceutically acceptable carriers, as well as other agents commonly used in the preparation of pharmaceutically acceptable compositions.

As used herein "pharmaceutically acceptable carrier" includes any and all solvents, dispersion media, coatings, antibacterial and antifungal agents, isotonic and absorption delaying agents, and the like that are physiologically compatible. Preferably, the carrier is suitable for parenteral administration, intravenous, intraperitoneal, intramuscular, sublingual, pulmonary, transdermal or oral administration. Pharmaceutically acceptable carriers include sterile aqueous solutions or dispersions and sterile powders for the extemporaneous preparation of sterile injectable solutions or dispersion. The use of such media and agents for the manufacture of medicaments is well known in the art. Except insofar as any conventional media or agent is incompatible with the pharmaceutically acceptable material, use thereof in the manufacture of a pharmaceutical composition is contemplated.

Pharmaceutical acceptable carriers may include one or more of the following examples:

(1) surfactants and polymers,, including, but not limited to polyethylene glycol (PEG), polyvinylpyrrolidone (PVP), polyvinylalcohol, crospovidone, polyvinylpyrrolidone-polyvinylacrylate copolymer, cellulose derivatives, hydroxypropylmethyl cellulose, hydroxypropyl cellulose, carboxymethylethyl cellulose, hydroxypropyllmethyl cellulose phthalate, polyacrylates and polymethacrylates, urea, sugars, polyols, and their polymers, emulsifiers, sugar gum, starch, organic acids and their salts, vinyl pyrrolidone and vinyl acetate; and or

(2) binding agents such as various celluloses and cross-linked polyvinylpyrrolidone, microcrystalline cellulose; and or

(3) filling agents such as lactose monohydrate, lactose anhydrous, microcrystalline cellulose and various starches; and or

(4) lubricating agents such as agents that act on the flowability of the powder to be compressed, including colloidal silicon dioxide, talc, stearic acid, magnesium stearate, calcium stearate, silica gel; and or

(5) sweeteners such as any natural or artificial sweetener including sucrose, xylitol, sodium saccharin, cyclamate, aspartame, and accsulfame K; and or

(6) flavouring agents; and or

(7) preservatives such as potassium sorbate, methylparaben, propylparaben, benzoic acid and its salts, other esters of parahydroxybenzoic acid such as butylparaben, alcohols such as ethyl or benzyl alcohol, phenolic chemicals such as phenol, or quarternary compounds such as benzalkonium chloride; and or

(8) buffers; and or

(9) Diluents such as pharmaceutically acceptable inert fillers, such as microcrystalline cellulose, lactose, dibasic calcium phosphate, saccharides, and/or mixtures of any of the foregoing; and or

(10) wetting agents such as corn starch, potato starch, maize starch, and modified starches, croscarmellose sodium, crosspovidone, sodium starch glycolate, and mixtures thereof; and or

(11) disintegrants; and or

(12) effervescent agents such as effervescent couples such as an organic acid (e.g., citric, tartaric, malic, fumaric, adipic, succinic, and alginic acids and anhydrides and acid salts), or a carbonate (e.g. sodium carbonate, potassium carbonate, magnesium carbonate, sodium glycine carbonate, L-lysine carbonate, and arginine carbonate) or bicarbonate (e.g. sodium bicarbonate or potassium bicarbonate); and or

(13) other pharmaceutically acceptable excipients.

Medicaments suitable for use in animals and in particular in man typically must be stable under the conditions of manufacture and storage. The medicaments comprising the biologically active material can be formulated as a solid, a solution, a microemulsion, a liposome, or other ordered structures suitable to high drug concentration. Actual dosage levels of the biologically active material in the medicament may be varied in accordance with the nature of the biologically active material, as well as the potential increased efficacy due to the advantages of providing and administering the biologically active material (e.g., increased solubility, more rapid dissolution, increased surface area of the biologically active material, etc.). Thus as used herein "therapeutically effective amount" will refer to an amount of biologically active material required to effect a therapeutic response in an animal. Amounts effective for such a use will depend on: the desired therapeutic effect; the route of administration; the potency of the biologically active material; the desired duration of treatment; the stage and severity of the disease being treated; the weight and general state of health of the patient; and the judgment of the prescribing physician.

In another embodiment, the biologically active material, optionally together with the grinding matrix or at least a portion of the grinding matrix, may be combined into a medicament with another biologically active material, or even the same biologically active material. In the latter embodiment, a medicament may be achieved which provides for different release characteristics - early release from the biologically active material, and later release from a larger average size biologically active material.

***Pharmacokinetic Properties of Metaxalone Compositions***

Suitable animal models to determine pharmacokinetic parameters are described in the prior art, such as the beagle dog model described in United States Patent No. 7,101,576.

*Fast Onset of Activity*

The metaxalone compositions exhibit faster therapeutic effects. In one example, following administration the metaxalone compositions have a $T_{max}$ of less than about 5 hours, less than about 4.5 hours, less than about 4 hours, less than about 3.5 hours, less than about 3 hours, less than about 2.75 hours, less than about 2.5 hours, less than about 2.25 hours, less than about 2 hours, less than about 1.75 hours, less than about 1.5 hours, less than about 1.25 hours, less than about 1.0 hours, less than about 50 minutes, less than about 40 minutes, less than about 30 minutes, less than about 25 minutes, less than about 20 minutes, less than about 15 minutes, less than about 10 minutes, less than about 5 minutes, or less than about 1 minute.

*Increased Bioavailability*

The metaxalone compositions preferably exhibit increased bioavailability (AUC) and require smaller doses as compared to prior conventional compositions administered at the same dose. Any drug composition can have adverse side effects. Thus, lower doses of drugs which can achieve the same or better therapeutic effects as those observed with larger doses of conventional compositions are desired. Such lower doses can be realized with the compositions of the invention because the greater bioavailability observed with the compositions as compared to conventional drug formulations means that smaller doses of drug are required to obtain the desired therapeutic effect.

*The Pharmacokinetic Profiles of the Compositions are not Substantially Affected by the Fed or Fasted State of the Subject Ingesting the Compositions*

The pharmacokinetic profile of the metaxalone compositions is not substantially affected by the fed or fasted state of a subject ingesting the composition. This means that there is no substantial difference in the quantity of composition or the rate of composition absorption when the compositions are administered in the fed versus the fasted state. Thus, the compositions substantially eliminate the effect of food on the pharmacokinetics of the composition.

The difference in absorption of the metaxalone composition when administered in the fed versus the fasted state, is less than about 35%, less than about 30%, less than about 25%, less than about 20%, less than about 15%, less than about 10%, less than about 5%, or less than about 3%. This is an especially important feature in treating patients with difficulty in maintaining a fed state.

In addition, preferably the difference in the rate of absorption (i.e., $T_{max}$) of the metaxalone compositions when administered in the fed versus the fasted state, is less than about 100%, less than about 90%, less than about 80%, less than about 70%, less than about 60%, less than about 50%, less than about 40%, less than about 30%, less than about 20%, less than about 15%, less than about 10%, less than about 5%, less than about 3%, or essentially no difference. Benefits of a dosage form which substantially eliminates the effect of food include an increase in subject convenience, thereby increasing subject compliance, as the subject does not need to ensure that they are taking a dose either with or without food. Preferably, the $T_{max}$ of an administered dose of a metaxalone composition is less than that of a conventional drug active composition, administered at the same dosage.

A preferred metaxalone composition exhibits in comparative pharmacokinetic testing with a standard conventional drug active composition, in oral suspension, capsule or tablet form, a $T_{max}$ which is less than about 100%, less than about 90%, less than about 80%, less than about 70%, less than about 60%, less than about 50%, less than about 40%, less than about 30%, less than about 25%, less than about 20%, less than about 15%, or less than about 10% of the $T_{max}$ exhibited by the standard conventional drug active composition.

In addition, preferably the $C_{max}$ of a metaxalone composition is greater than the $C_{max}$ of a conventional drug active composition, administered at the same dosage. A preferred composition exhibits in comparative pharmacokinetic testing with a standard conventional drug active composition, in oral suspension, capsule or tablet form, a $C_{max}$ which is greater than about 5%, greater than about 10%, greater than about 15%, greater than about 20%, greater than about 30%, greater than about 40%, greater than about 50%, greater than about 60%, greater than about 70%, greater than about 80%, greater than about 90%, greater than about 100%, greater than about 110%, greater than about 120%, greater than about 130%, greater than about 140%, or greater than about 150% than the $C_{max}$ exhibited by the standard conventional drug active composition.

In addition, preferably the metaxalone composition has an AUC greater than that of the equivalent conventional composition administered at the same dosage. A preferred composition exhibits in comparative pharmacokinetic testing with a standard conventional drug active composition, in oral suspension, capsule or tablet form, a AUC which is greater than about 5%, greater than about 10%, greater than about 15%, greater than about 20%, greater than about 30%, greater than about 40%, greater than about 50%, greater than about 60%, greater than about 70%, greater than about 80%, greater than about 90%, greater than about 100%, greater than about 110%, greater than about 120%, greater than about 130%, greater than about 140%, or greater than about 150% than the AUC exhibited by the standard conventional drug active composition.

Any standard pharmacokinetic protocol can be used to determine blood plasma concentration profile in humans following administration of a composition, and thereby establish whether that composition meets the pharmacokinetic criteria set out herein. For example, a randomized single-dose crossover study can be performed using a group of healthy adult human subjects. The number of subjects should be sufficient to provide adequate control of variation in a statistical analysis, and is typically about 10 or greater, although for certain purposes a smaller group can suffice. Each subject receives by oral administration at time zero a single dose (e.g., 300 mg) of a test formulation of composition, normally at around 8 am following an overnight fast. The subjects continue to fast and remain in an upright position for about 4 hours after administration of the composition. Blood samples are collected from each subject prior to administration (e.g., 15 minutes) and at several intervals after administration. For the present purpose it is preferred to take several samples within the first hour, and to sample less frequently thereafter. Illustratively, blood samples could be collected at 15, 30, 45, 60, and 90 minutes after administration, then every hour from 2 to 10 hours after administration. Additional blood samples may also be taken later, for example at 12 and 24 hours after administration. If the same subjects are to be used for study of a second test formulation, a period of at least 7 days should elapse before administration of the second formulation. Plasma is separated from the blood samples by centrifugation and the separated plasma is analyzed for composition by a validated high performance liquid chromatography (HPLC) or liquid chromatography mass spectrometry (LCMS) procedure.. Plasma concentrations of composition referenced herein are intended to mean total concentrations including both free and bound composition.

Any formulation giving the desired pharmacokinetic profile is suitable for administration according to the present methods. Exemplary types of formulations giving such profiles are liquid dispersions and solid dose forms of composition. If the liquid dispersion medium is one in which the composition has very low solubility, the particles are present as suspended particles. The smaller the particles the higher the probability that the formulation will exhibit the desired pharmacokinetic profile.

Thus, a metaxalone composition upon administration to a subject, provides improved pharmacokinetic and/or pharmacodynamic properties compared with a standard reference indomethacin composition as measured by at least one of speed of absorption, dosage potency, efficacy, and safety.

### Modes of administration of medicaments comprising biologically active materials

Medicaments can be administered to animals, including man, in any pharmaceutically acceptable manner, such as orally, rectally, pulmonary, intravaginally, locally (powders, ointments or drops), transdermal, parenteral administration, intravenous, intraperitoneal, intramuscular, sublingual or as a buccal or nasal spray.

Solid dosage forms for oral administration include capsules, tablets, pills, powders, pellets, and granules. Further, incorporating any of the normally employed excipients, such as those previously listed, and generally 5-95% of the biologically active agent, and more preferably at a concentration of 10%-75% will form a pharmaceutically acceptable non-toxic oral composition. Medicaments may be parenterally administered as a solution of the biologically active agent suspended in an acceptable carrier, preferably an aqueous carrier. A variety of aqueous carriers may be used, e.g. water, buffered water, 0.4% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration.

For aerosol administration, medicaments are preferably supplied along with a surfactant or polymer and propellant. The surfactant or polymer must, of course, be non-toxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant or polymer may constitute 0.1%-20% by weight of the composition, preferably 0.25-5%. The balance of the composition is ordinarily propellant. A carrier can also be included, as desired, as with, e.g., lecithin for intranasal delivery.

Medicaments may also be administered via liposomes, which serve to target the active agent to a particular tissue, such as lymphoid tissue, or targeted selectively to cells. Liposomes include emulsions, foams, micelles, insoluble monolayers, liquid crystals, phospholipid dispersions, lamellar layers and the like. In these preparations the composite microstructure composition is incorporated as part of a liposome, alone or in conjunction with a molecule that binds to or with other therapeutic or immunogenic compositions.

As described above, the biologically active material can be formulated into a solid dosage form (e.g., for oral or suppository administration), together with the grinding matrix or at least a portion of it. In this case there may be little or no need to add stabilizing agents since the grinding matrix may effectively act as a solid-state stabilizer.

However, if the biologically active material is to be utilized in a liquid suspension, the particles comprising the biologically active material may require further stabilization once the solid carrier has been substantially removed to ensure the elimination, or at least minimisation of particle agglomeration.

### Therapeutic uses

Therapeutic uses of the medicaments include pain relief, anti-inflammatory, migraine, asthma, and other disorders that require the active agent to be administered with a high bioavailability.

One of the main areas when rapid bioavailability of a biologically active material is required is in the relief of pain. The minor analgesics, such as cyclooxgenase inhibitors (aspirin related drugs) may be prepared as medicaments. The medicaments may also be used for treatment of eye disorders. That is, the biologically active material may be formulated for administration on the eye as an aqueous suspension in physiological saline, or a gel. In addition, the biologically active material may be prepared in a powder form for administration via the nose for rapid central nervous system penetration.

Treatment of cardiovascular disease may also benefit from biologically active materials, such as treatment of angina pectoris and, in particular, molsidomine may benefit from better bioavailability.

Other therapeutic uses of the medicaments include treatment of hair loss, sexual dysfunction, or dermal treatment of psoriasis.

The present invention will now be described with reference to the following non-limiting Examples. The description of the Examples is in no way limiting on the preceding paragraphs of this specification, but is provided for exemplification of the methods of the invention.

**Examples**

The invention is to be determined from the foregoing description and the appended claims. Furthermore, the following Examples are provided for illustrative purposes only, and are not intended to limit the scope of the processes or compositions of the invention.

*The following materials were used in the examples*

Active pharmaceutical ingredients were sourced from commercial suppliers, excipients from either commercial suppliers such as Sigma-Aldrich or retailers, while food ingredients were sourced from retailers.

*The following mills were used for the grinding experiments*

Spex-type Mill:

Small scale milling experiments were conducted using a vibratory Spex 8000D mixer/mill. Twelve 3/8" stainless steel balls were used as the grinding media. The powder charge and grinding media were loaded into a hardened steel vial with an internal volume of approximately 75 mL. Following milling, the milled material was discharged from the vial and sieved to remove grinding media.

Attritor-type Mill:

Small scale attritor milling experiments were performed using a 1HD Union Process attritor mill with a 110 mL grinding chamber. The grinding media consisted of 330g 5/16" stainless steel balls. The mill was loaded through the loading port, with dry materials added initially, followed by the grinding media. The milling process was conducted with the jacket cooled at 10-20°C and the shaft rotating at 500 rpm. Upon completion of milling, the milled material was discharged from the mill and sieved to remove the grinding media.

Medium scale attritor milling experiments were performed using a 1HD Union Process attritor mill with a 1 L grinding chamber or a 1S Union Process attritor mill with a 750 mL grinding chamber. The grinding media consisted of 3 kg of 5/16" stainless steel balls or 1.5 kg of 3/8" stainless steel balls for the 1S attritor. The 1HD mill was loaded through the loading port, with dry materials added initially, followed by the grinding media, while the grinding media was added initially, followed by the dry materials in the 1S attritor mill. The milling process was conducted with the jacket cooled at 10-20°C with the shaft rotating at 350 rpm in the 1HD attritor or 550 rpm in the 1S attritor. Upon completion of milling, the milled material was discharged from the mill and sieved to remove the grinding media.

Medium to large scale attritor milling experiments were performed using a 1S Union Process attritor mill with a ½ gallon grinding chamber. The grinding media consisted of 7 kg of 3/8" stainless steel balls. The mill was loaded through the loading port, with the grinding media added initially, followed by the dry powders. The milling process was conducted with the jacket cooled at 18°C and the shaft rotating at 550-555 rpm. Upon completion of milling, the milled powder was discharged from the mill through the bottom discharge port at 77rpm for 5min.

Large scale attritor milling experiments were performed using a 1S Union Process attritor mill with a 1½ gallon grinding chamber. The grinding media consisted of 20 kg of 3/8" stainless steel balls. The mill was loaded through the loading port, with the grinding media added initially, then followed by the dry powders. The milling process was conducted with the jacket cooled to ambient temperature and the shaft rotating at 300 rpm. Upon completion of milling, the milled powder was discharged from the mill through the bottom discharge port at 77rpm for 5 min.

The largest scale attritor millings were done in a 30S Union Process mill with a 25 gallon grinding chamber (Union Process, Akron OH, USA). The grinding media consisted of 454kg of 3/8" stainless steel balls. The mill was loaded through its split top lid, with the grinding media added initially, then followed by the dry powders (25kg). The milling process was conducted with the jacket cooled to 10°C and the shaft rotating at 130 rpm. Upon completion of milling, the milled powder was discharged from the mill through the bottom discharge port at 77rpm for 5 min.

Siebtechnik Mill

Medium scale milling experiments were also performed in a Siebtechnik GSM06 (Siebtechnik ,GmbH, Germany) with two 1 L milling chambers. Each chamber was filled with 2.7 kg stainless steel media with a diameter of 3/8". The media and powder were loaded with the lid off. The mill was operated at ambient temperature. The vibration speed was the standard mill settings. Upon completion of the milling the media was separated from the powder by sieving.

Simolover Mill

Medium scale milling experiments were performed in a Simoloyer CM01 (ZOZ GmbH, Germany) with a 2 L milling chamber. The grinding media consisted of 2.5 kg stainless steel media with a diameter of 5 mm. the media was loaded though the loading port followed by the dry materials. The milling vessel was cooled using water at a temperature of about 18°C. The mill speed was operated in cycle mode: at 1300 rpm for two minutes and at 500 rpm for 0.5 min and so forth. Upon completion of the milling the media was discharged from the mill using a grated valve to retain the grinding media.

Large scale milling experiments were performed in a Simoloyer CM100 (ZOZ GmbH, Germany) with a 100 L milling chamber. The grinding media consisted of 100 kg stainless steel media with a diameter of 3/16". The powder charge (11kg) was added to the milling chamber, which already contained the grinding media, through a loading port. The milling chamber was cooled to 18°C and the powder was milled for a total of 20 minutes using a cycling mode equivalent to a tip speed at 1300/500 rpm for 2/0.5 min in the CM-01 type mill. Upon completion of the milling the mill was discharged by sucking the powder into a cyclone.

Hicom Mill

Millings performed in a nutating Hicom mill utilized 14kg of stainless steel 0.25" grinding media together with a powder charge of 480g. The mill was loaded by pre-mixing media and powder, then adding the mixture to the grinding chamber through the loading port at the top of the mill. The milling was done at 1000rpm and the mill discharged by inverting the mill and emptying through the loading port. The recovered material was sieved to separate the grinding media from the powder.

Variations to the milling conditions set out above are indicated in the variations column in the data tables. The key to these variations is shown in Table A.

Particle size measurement:

The particle size distribution (PSD) was determined using a Malvern Mastersizer 2000 fitted with a Malvern Hydro 2000S pump unit. Measurement settings used: Measurement Time: 12 seconds, Measurement cycles: 3. Final result generated by averaging the 3 measurements. Samples were prepared by adding 200mg of milled material to 5.0mL of 1% PVP in 10mM hydrochloric acid (HCl), vortexing for 1 min and then sonicating. From this suspension enough was added into the dispersant (10mM HCl) to attain a desired obscuration level. If necessary an extra 1-2 minutes of sonication was applied using the internal sonication probe in the measurement cell. The refractive index of the active ingredient to be measured was in the range of 1.49-1.73. Any variations to this general method are summarized in Table B.

XRD Analysis:

Powder X-Ray diffraction (XRD) patterns were measured with a Diffractometer D 5000, Kristalloflex (Siemens). The measurement range was from 5-18 degrees 2-Theta. The slit width was set to 2 mm and the cathode ray tube was operated at 40 kV and 35 mA. Measurements were recorded at room temperature. The recorded traces were subsequently processed using Bruker EVA software to obtain the diffraction pattern.

Table A. Variations to milling conditions. Only conditions reported in the table have changed as compared to conditions reported above.

| Variation # | Mill type | Milling Speed (rpm) | Media size in mm (inch) | Media Mass (kg) | Offload spped (rpm) |
|---|---|---|---|---|---|
| A | 1HD 1L | | 6.3 (0.25) | | |

(continued)

| Variation # | Mill type | Milling Speed (rpm) | Media size in mm (inch) | Media Mass (kg) | Offload spped (rpm) |
|---|---|---|---|---|---|
| B | 1S 0.5gal | | | 5 | |
| C | 1S 0.5gal | | | 4 | |
| D | 1S 0.5gal | 500 | | | |
| E | 1S 0.5gal | 550-555 | | | |
| F | 1S 1.5gal | 316-318 | | 21 | |
| G | 1S 1.5gal | 500 | | 21 | |
| H | 1S 1.5gal | 355 | | 21 | |
| I | 1S 1.5gal | 355 | | 18 | |
| J | 1S 1.5gal | | | 21 | |
| K | 1S 1.5gal | | | 18.4 | |
| L | 1S 1.5gal | 400 | | | |
| M | 1S 1.5gal | | | 21 | 57 |
| N | 1S 1.5gal | | | | 57 |
| O | 1S 0.5gal | 400 | | | 400 |
| P | 1S 0.5gal | 500 | | | 350 |
| Q | HICOM | | 3.2(1/8) | | |
| R | HICOM | | | 11.7 | |

| Variation # | Sample Dispersant | Measurement Dispersant | Addition Method |
|---|---|---|---|
| 1 | | 0.1%PVP in DI water | Powder addition |
| 2 | 0.2% Pluronic L81 in DI water | DI water | |
| 3 | | Saturated glyphosate in DI water | Powder addition |
| 4 | | Saturated glyphosate in DI water | Powder addition |
| 5 | 1%PVP in DI water | DI water | |
| 6 | | DI water | Powder addition |
| 7 | 1%PVP in DI water | Saturated creatine in DI water | |
| 8 | 1%PVP in DI water | 10mM HCl | |
| 9 | 0.2% Pluronic L81 in DI water | Acidified with 1M HCl | |
| 10 | 1%PVP in DI water | 0.1%PVP in DI water | |
| 11 | 1%PVP in DI water | 1%PVP in DI water | |
| 12 | | | Filtered before PSD measurement |
| Table B. Variations to particle size measurement conditions. | | | |

Abbreviations:

HCl: Hydrochloric acid

Nap: Naproxen acid
PSD: Particles size distribution
PVP: Polyvinyl pyrrolidone
RI: Refractive index
Rpm: Revolutions per minute
SLS: Sodium lauryl sulphate
SSB: Stainless Steel Balls
XRD: X-Ray Diffraction

Other abbreviations used in the data tables are listed below in Table C (for actives), Table D (for matrices) and Table E (for surfactants). In the data tables single letter with example number abbreviations have been used to identify specific sample numbers within the table. The data tables shown in the figures the use of surfactant , matrix are interchangeable and do not necessarily define the nature of that material.

Table C. Abbreviations used for active pharmaceutical ingredients.

| API Name | Abbreviation |
|---|---|
| 2,4-Dichlorophenoxyacetic acid | 2,4D |
| Anthraquinone | ANT |
| Celecoxib | CEL |
| Cilostazol | CIL |
| Ciprofloxacin | CIP |
| Creatine Monohydrate | CRM |
| Cyclosporin A | CYA |
| Diclofenac Acid | DIC |
| Glyphosate | GLY |
| Halusulfuron | HAL |
| Indomethacin | IND |
| Mancozeb | MAN |
| Meloxicam | MEL |
| Metaxalone | MTX |
| Metsulfuron | MET |
| Naproxen Acid | NAA |
| Naproxen Sodium | NAS |
| Progesterone | PRO |
| Salbutamol | SAL |
| Sulfur | SUL |
| Tribenuran | TRI |

Table D. Abbreviations used for excipients.

| Matrix Name | Abbreviation |
|---|---|
| Calcium Carbonate | CAC |
| Glucose | GLU |
| Lactose Anhydrous | LAA |
| Lactose Monohydrate | LAC |

(continued)

| Matrix Name | Abbreviation |
|---|---|
| Lactose Monohydrate Food Grade | LFG |
| Malic Acid | MAA |
| Maltitol | MAL |
| Mannitol | MAN |
| Sodium Bicarbonate | SB |
| Sodium Chloride | SC |
| Sorbitol | SOR |
| Sucrose | SUC |
| Tartaric Acid | TA |
| TriSodium Citrate Dihydrate | TCD |
| Whey Powder | WP |
| Xylitol | XYL |

Table E. Abbreviations used for surfactants

| Surfactant Name | Abbreviation |
|---|---|
| Aerosil R972 Silica | AS |
| Benzalkonium Chloride | BC |
| Brij700 | B700 |
| Brij76 | B76 |
| Cremophor EL | CEL |
| Cremophor RH-40 | C40 |
| Dehscofix 920 | D920 |
| Docusate Sodium | DS |
| Kollidon 25 | K25 |
| Kraftsperse 1251 | K1251 |
| Lecithin | LEC |
| Poloxamer 188 | P188 |
| Microcrystalline Cellulose | MCC |
| Poloxamer 407 | P407 |
| Polyethylene Glycol 3000 | P3000 |
| Polyethylene Glycol 8000 | P8000 |
| Polyoxyethylene 40 Stearate | P40S |
| Polyvinyl Pyrrolidone (Kollidon 30) | PVP |
| Primellose | PML |
| Primojel | PRI |
| Sodium Deoxycholate | SDC |
| Sodium Dodecyl Sulphate | SDS |

(continued)

| Surfactant Name | Abbreviation |
|---|---|
| Sodium Dodecylbenzenesulphonic Acid | SDA |
| Sodium N-Lauroyl Sarcosine | SNS |
| Sodium Octadecyl Sulphate | SOS |
| Sodium Pentane Sulphonate | SPS |
| Soluplus HS15 | SOL |
| Teric 305 | T305 |
| Tersperse 2700 | T2700 |
| Terwet 1221 | T1221 |
| Terwet 3785 | T3785 |
| Tween 80 | T80 |

**Example 1: Spex Milling**

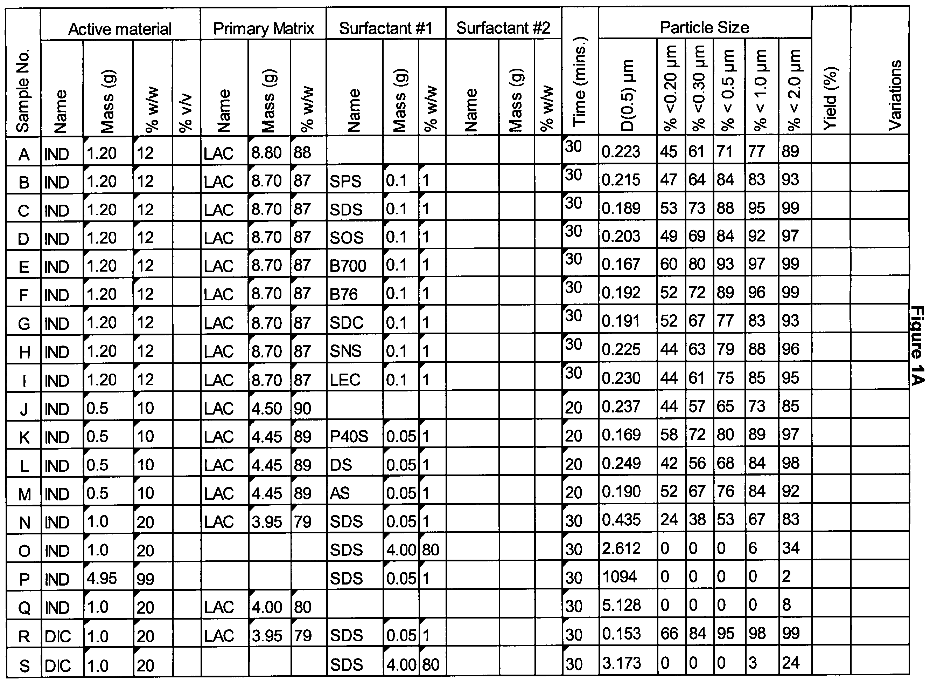

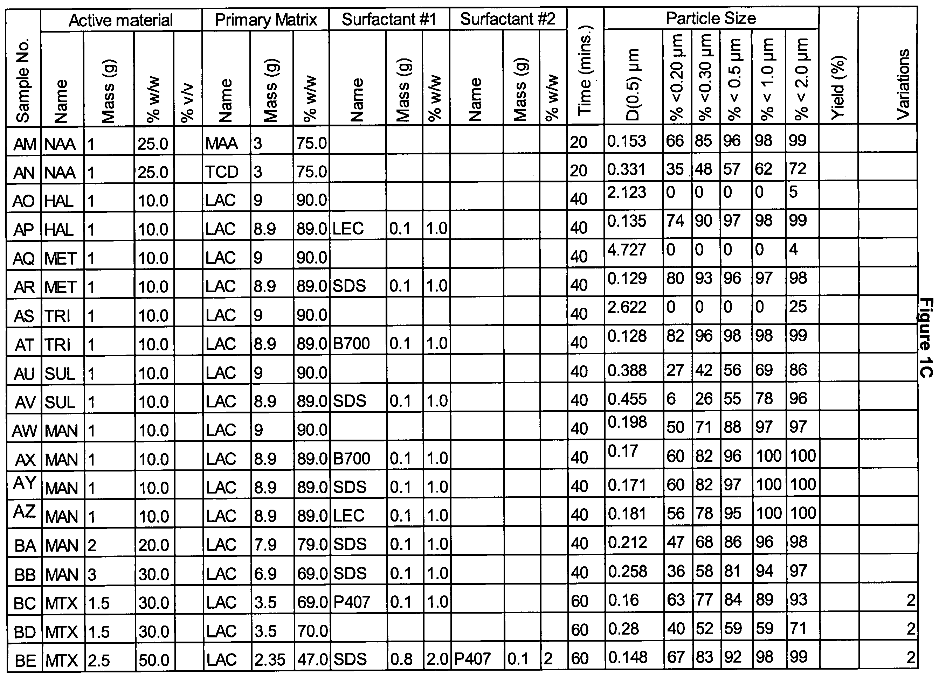

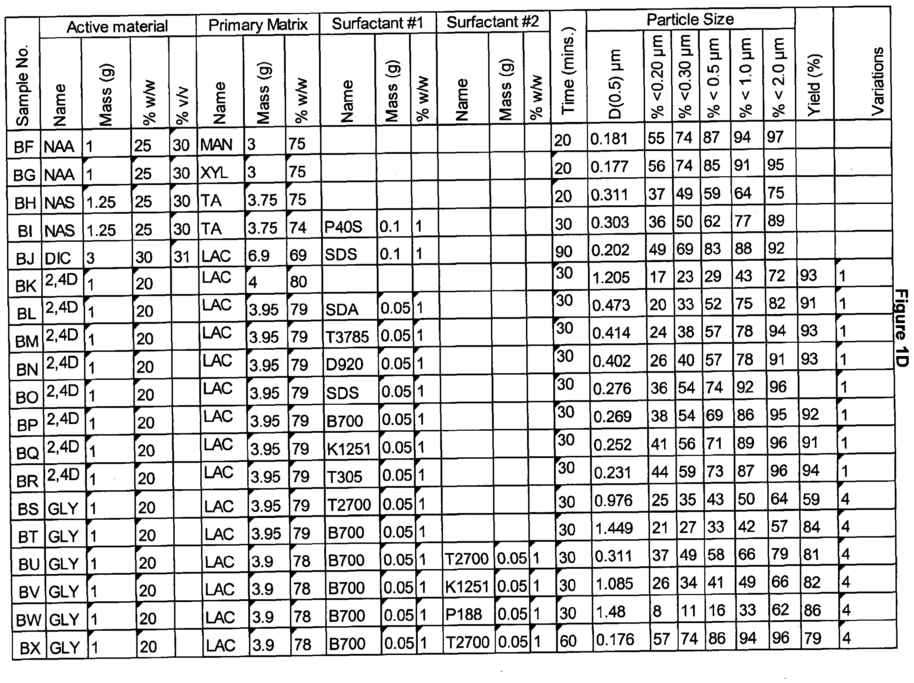

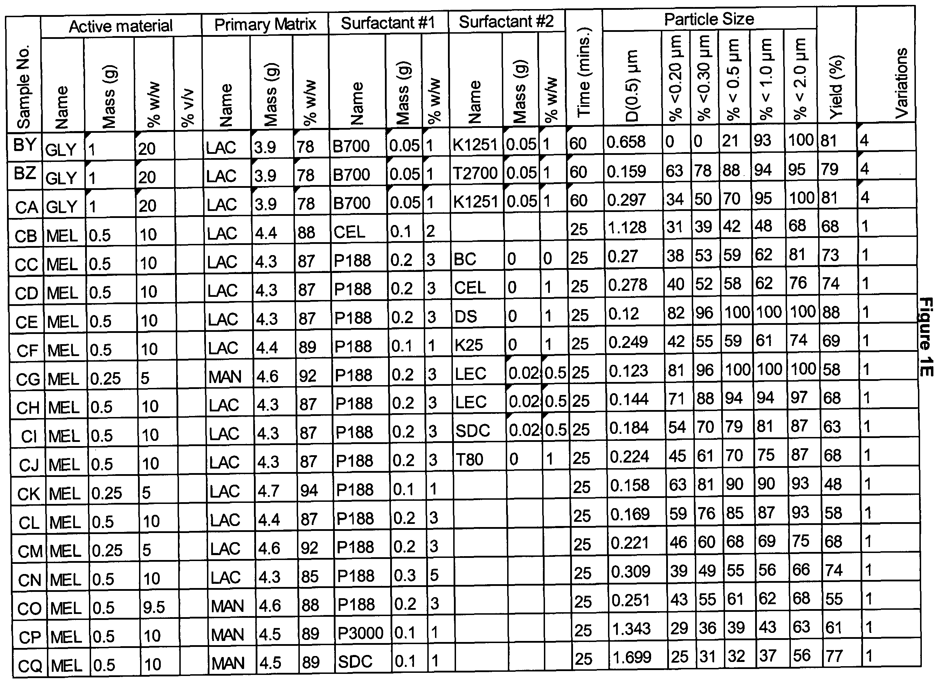

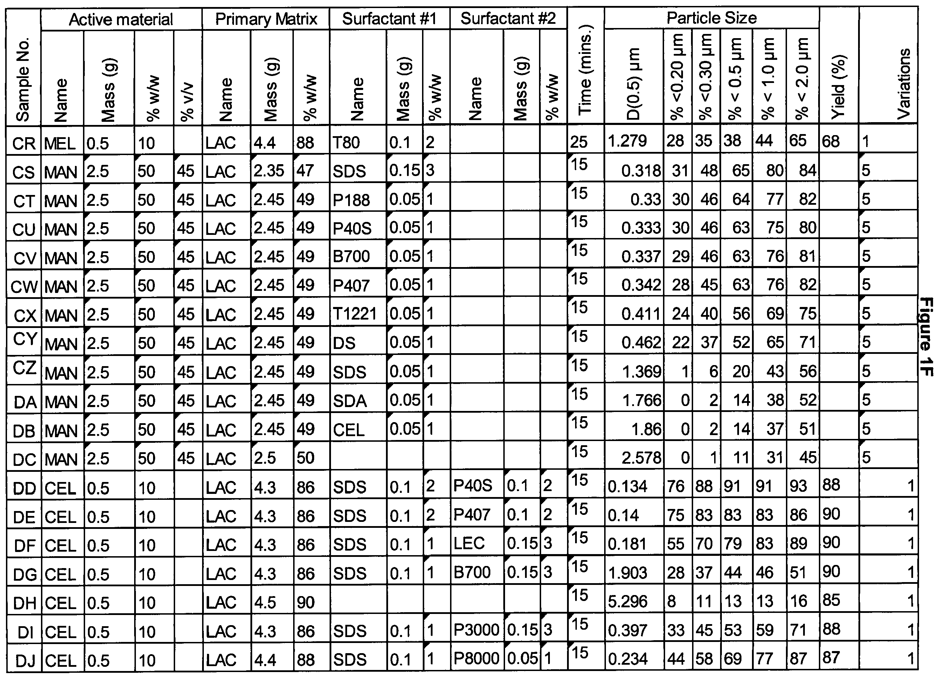

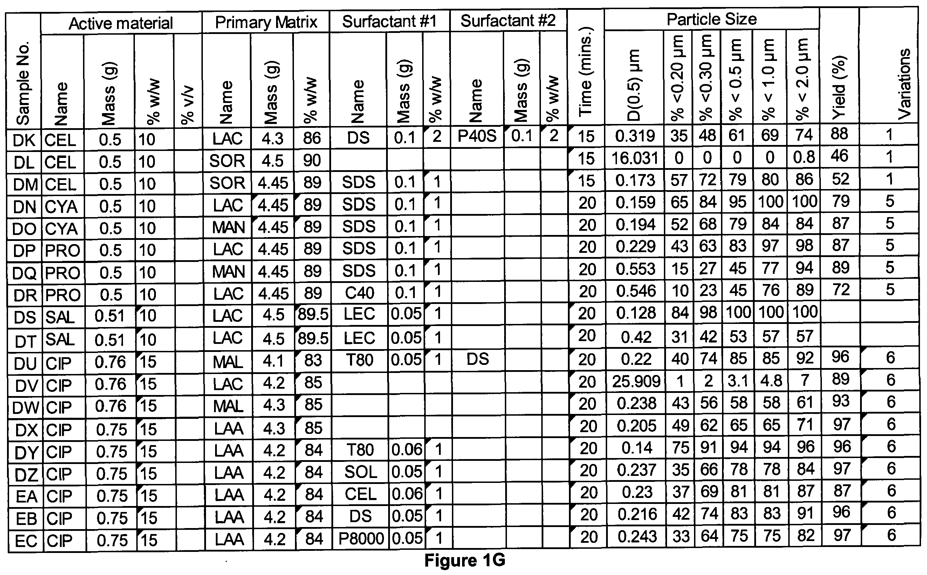

A range of actives, matrices and surfactants in a variety of combinations were milled using the Spex mill. The details of these millings are shown in Figures 1A-1G together with the particle size distributions of actives that were milled.

These millings demonstrate that the addition of a small amount of surfactant to the milling matrix delivers a smaller particle size compared to millings of just an active and a single matrix. Some examples of this are samples Z and AA compared to sample Y; Sample AB compared to sample AC; sample AE compared to sample AD; sample AG compared to sample AF; sample AP compared to sample AO; sample AR compared to sample AQ, sample AT compared to sample AS; Samples AX, AY and AZ compared to sample AW; sample BC compared to sample BD; sample BI compared to BH; samples BL-BR compared to sample BK; samples CS-DB compared to sample DC. This last example is particularly noteworthy as these millings were undertaken at 45 % v/v. This demonstrates the broad applicability of this invention. Some other examples of surfactant addition being beneficial for size reduction are samples DD-DG and DI-DK compared to sample DH; sample DM compared to sample DL. Other samples such as samples DY-EC compared to sample DX; sample AV compared to sample AU; samples B-H compared to sample A and samples K-M compared to sample J show this to be also true when particle size statistics such the % < 1 micrometer as used.

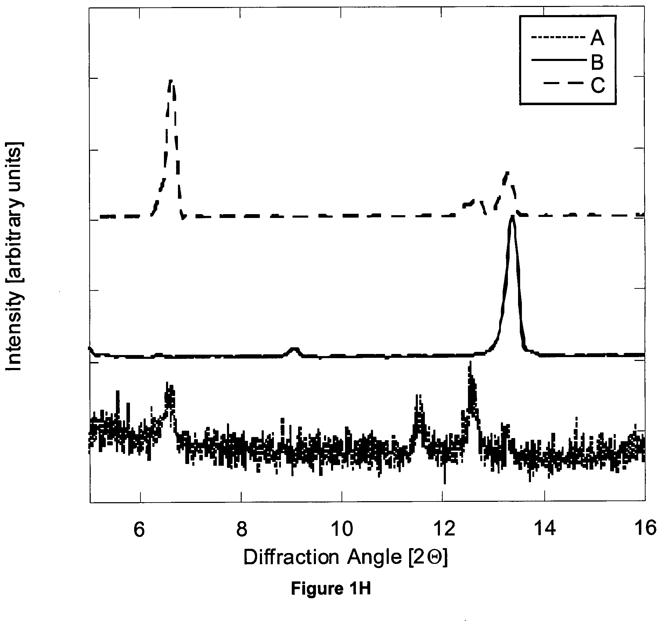

Note that this applies to mechanochemcial matrix milling as well. This is demonstrated by sample BI where naproxen sodium is milled with tartaric acid and converted to naproxen acid. Figure 1H shows XRD data that demonstrates the transformation.

Other samples such as CB-CR show examples were surfactants suitable for use with IV formulations can be used to manufacture very small particles.

It is also noteworthy that samples DS and DT could be sized using a saturated solution of the active (salbutamol) demonstrating that actives with high water solubility can be measured as long as care is taken when measuring the size.

Two sets of data, samples N-Q and samples R-U, also demonstrate that the invention described herein is unique. In these samples the active milled with a matrix and surfactant produces small particles. When milled with matrix alone the particles sizes are larger, in the case of sample Q they are not even nanoparticles. When the active is milled with just 1% surfactant the resultant particle size is very large. Even when 80 % surfactant is used the size is large.

**Example 2: 110mL Attritor**

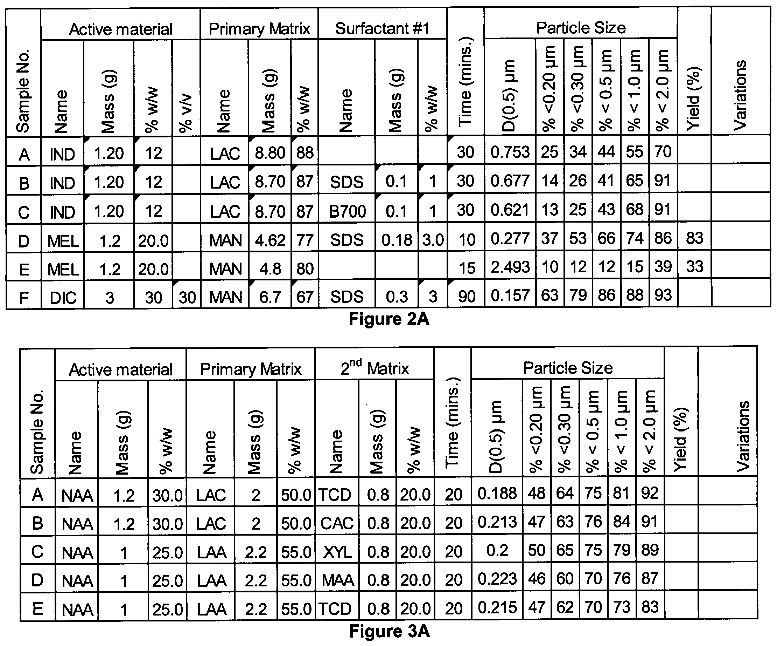

A range of actives, matrices and surfactants in a variety of combinations were milled using the 110 ml stirred attritor mill. The details of these millings are shown in Figure 2A together with the particle size distributions of actives that were milled.

These millings also demonstrate that the addition of a small amount of surfactant to the milling matrix delivers a smaller particle size compared to millings of just an active and a single matrix in a small scale stirred mill as well as the vibratory Spex mill. Sample F also demonstrates that small particles can be achieved at high % actives when a surfactant is present. Sample D and E also show that the addition of the surfactant also increased the yield of powder from the mill.

**Example 3: Second Matrix**

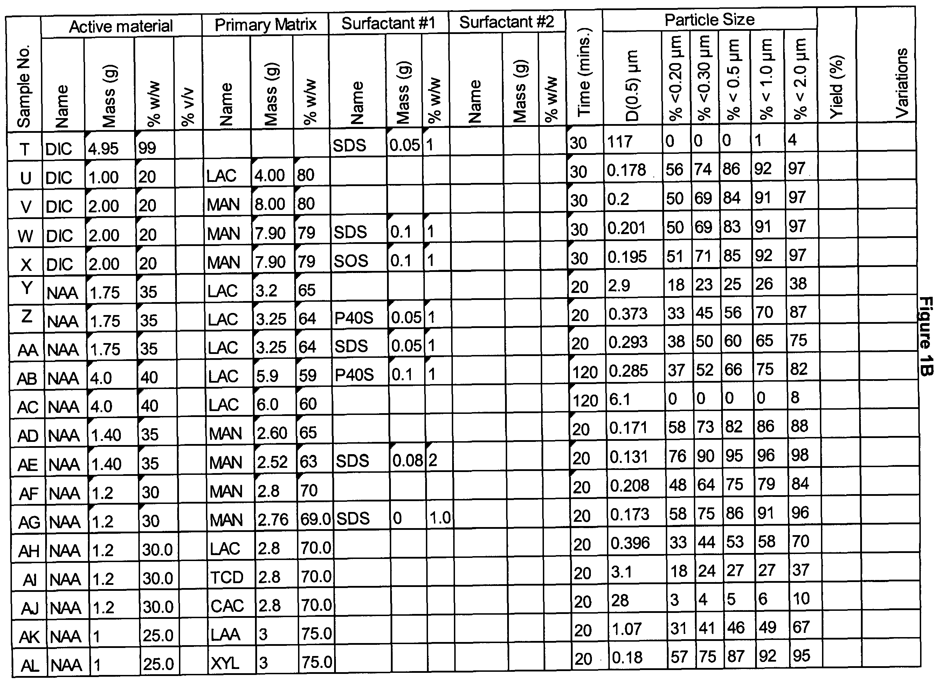

In this example naproxen was milled with a mixture of two matrices using the Spex mill. The details of these millings are shown in Figure 3A together with the particle size distributions of actives that were milled. Samples A and B were milled in a primary matrix of lactose monohydrate and 20 % of second matrix. The particle size of these millings is smaller than the same milling with just lactose monohydrate (See example 1 sample No AH, Figure 1B). The particle size is also smaller than naproxen milled in the secondary matrices (See example 1 sample No AI and AJ, Figure 1B). This shows the mixed matrices have synergy together. Samples C-E were milled in anhydrous lactose with 20 % of a second matrix. All these samples had a particle size much smaller than naproxen milled in anhydrous lactose alone (See example 1 sample No AK, Figure 1B).

These millings demonstrate that the addition of a second matrix to the primary milling matrix delivers a smaller particle size compared to millings with just a single matrix.

**Example 4: 1L Attritor**

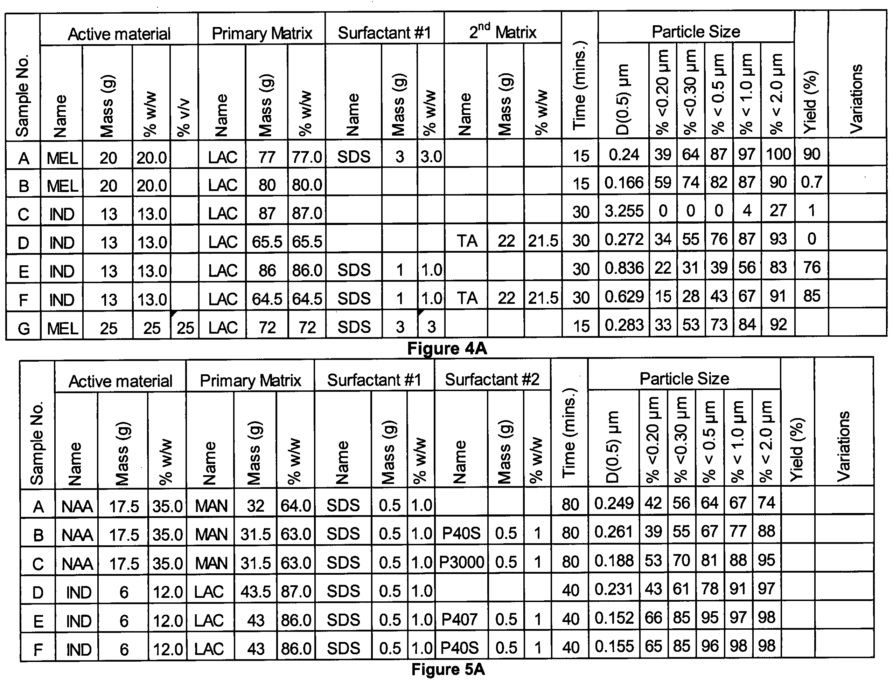

Two actives with various combinations of lactose monohydrate and SDS were milled using the 1 L stirred attritor mill. The details of these millings are shown in Figure 4A together with the particle size distributions of actives that were milled.

Sample A and B are millings of meloxicam at 20 %. While sample B has a slightly smaller particle size than sample A there is a dramatic difference in the amount of material recovered from the milling. Sample A, milled with 3 % SDS has a high yield of 90% whereas sample B with no surfactant has practically no yield with all the powder caked in the mill.

In samples C-F the milling of 13 % indomethacin shows that the use of a second matrix (tartaric acid) in combination with 1% SDS delivers the best outcome of a good particle size and high yield. Sample D which has just the mixed matrix has very good particle size but poor yield.

These results show that the addition of a small amount of surfactant improves milling performance.

**Example 5: 750mL Attritor**

Two actives with various combinations surfactants were milled using the 750 ml stirred attritor mill. The details of these millings are shown in Figure 5A together with the particle size distributions of actives that were milled.

In samples A-C three millings of naproxen are shown. Sample A has just 1% SDS as a surfactant. Samples B and C have a second surfactant present and these samples have a smaller particle size as measured by the %< 500 nm, % < 1000nm and % < 2000 nm.

In samples D-F three millings of indomethacin are shown. Sample D has just 1% SDS as a surfactant. Samples E and F have a second surfactant present and these samples have a smaller particle size compared to sample D.

These examples demonstrate that the use of combination of surfactants can be useful to achieve better reduction in particle size.

**Example 6: 1/2Gallon 1S**

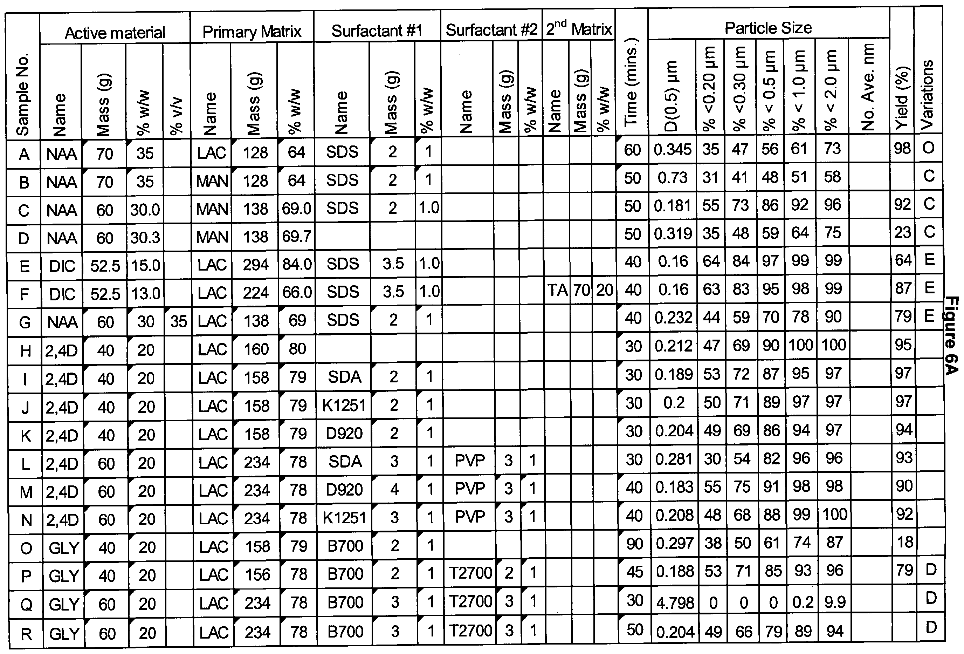

A range of actives, matrices and surfactants in a variety of combinations were milled using the ½ gallon 1S mill. The details of these millings are shown in Figures 6A-C together with the particle size distributions of actives that were milled.

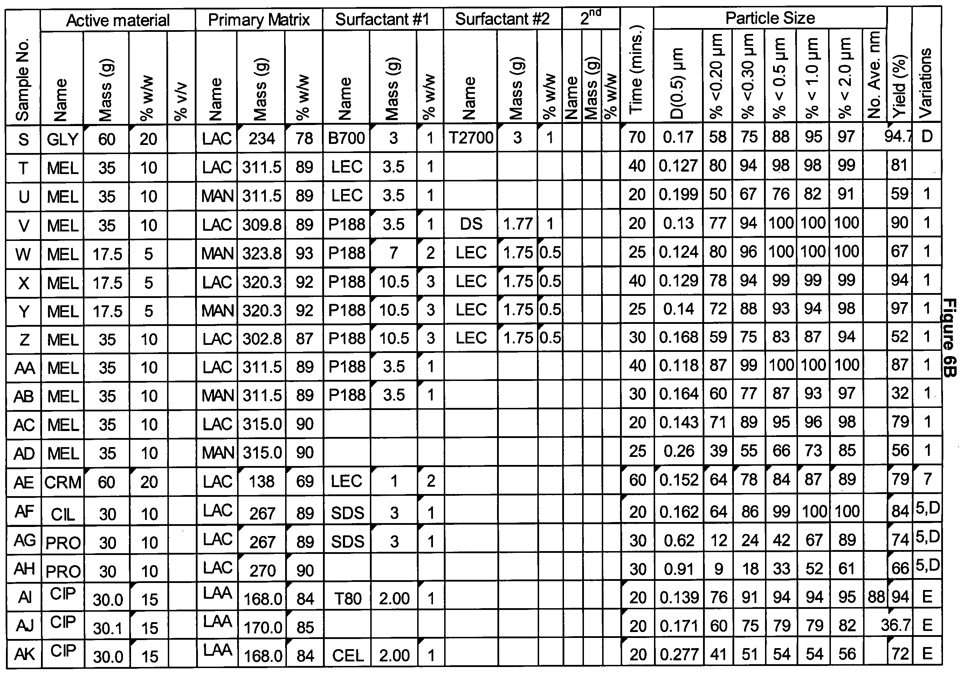

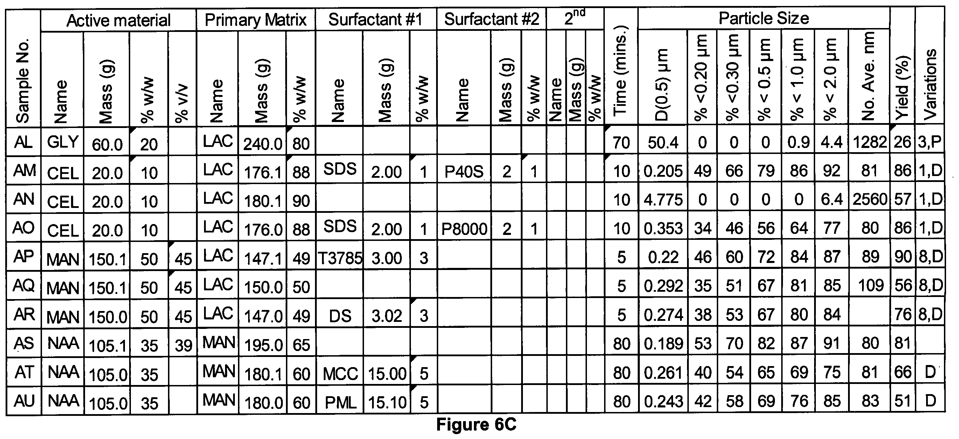

The following examples demonstrate the increased yield obtained when milling an active in a 1/2gallon 1S attritor mill with a surfactant as compared to no surfactant, with all other factors being identical. Sample C and D (Figure 6A) shows Naproxen acid milled in Mannitol with yields of 92% and 23%, with and without surfactant. Sample S and AL (Figure 6B and C) show the same for glyphosate with yields of 95% and 26%, respectively. Sample AI and AJ (Figure 6B) show Ciprofloxacin yields of 94% and 37% with and without surfactant while sample AM an AN (Figure 6C) show Celecoxib yields of 86% and 57% with and without surfactants. Finally, samples AP and AQ (Figure 6C) shows milling Mancozeb with or without surfactants results in yields of 90% and 56%, respectively.

The following examples illustrates that milling an active in a 1/2gallon 1S attritor mill with a surfactant as compared to without surfactant and all other factors identical, leads to smaller particle size after milling. Sample C and D (Figure 6A) shows a D(0.5) of 0.181 and 0.319 with or without surfactant, while sample AM and AN (Figure 6C) shows D(0.5) of 0.205 and 4.775 with and without surfactants.

The series of samples Q-S are timepoints taken from a single glyphosate milling. The data demonstrates that the size of the actives decreases with milling time.

Other samples such as V-AA show examples were surfactants suitable for use with IV formulations can be used to manufacture very small particles.

Some of the particle size data in Figures 6A-C was converted to a number average particle size and is shown in the tables. This number was calculated in the following way. The Volume distribution was transformed to the number distribution using the Malvern Mastersizer software. For each size bin the size of the bin was multiplied by the % of particles in the bin. This numbers were added together and divided by 100 to give the number average particle size.

### Example 7: Metaxalone

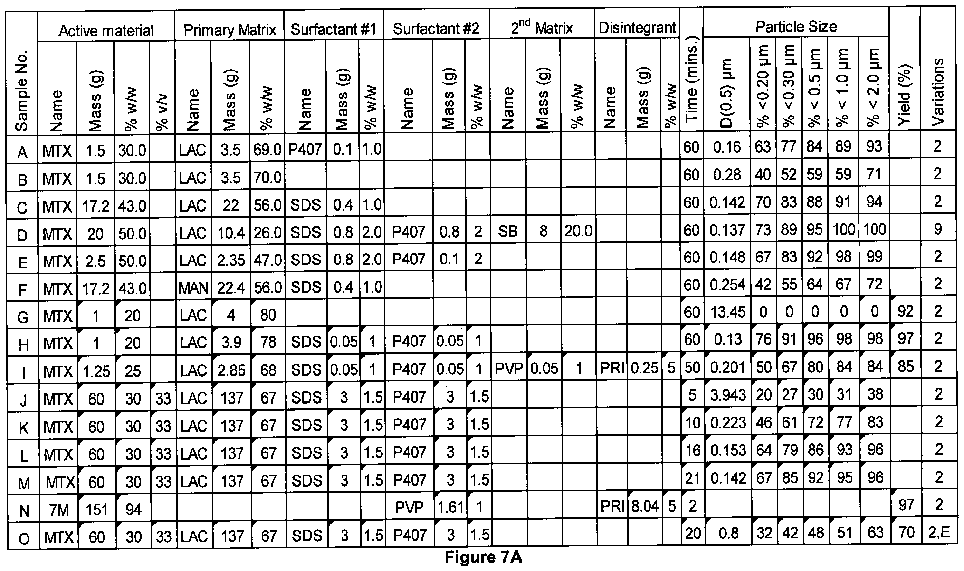

Metaxalone was milled with various combinations of matrices and surfactants using a variety of mills. The details of these millings are shown in Figure 7A together with the particle size distributions of actives that were milled. Samples A, B, E, G, H and I were milled in a Spex mill. Samples C, D and F were milled in the 750 ml atrittor. The remaining samples were milled in the ½ gallon 1S mill.

Samples A compared to sample B and sample H compared to sample G demonstrate that the addition of one or more surfactants enables the production of smaller active particles. Other millings such as samples C-F show that metaxalone can be milled small at very high active loadings. Sample I shows that disintegrant can be added during milling and not effect the production of small active particles. Note that the particle size in sample I is after filtration through a 10 micrometer filter. Sample N shows an alternative way to manufacture a formulation with small particles and disintegrants. In this example the powder from sample M was left in the mill and a wetting agent (PVP) and disintegrant were added. The powder was milled for a further 2 minutes and then unloaded with a very high yield of 97%.

The series of samples J-M are timepoints taken from a single milling. The data demonstrates that the size of the actives decreases with milling time.

### Example 8: Hicom

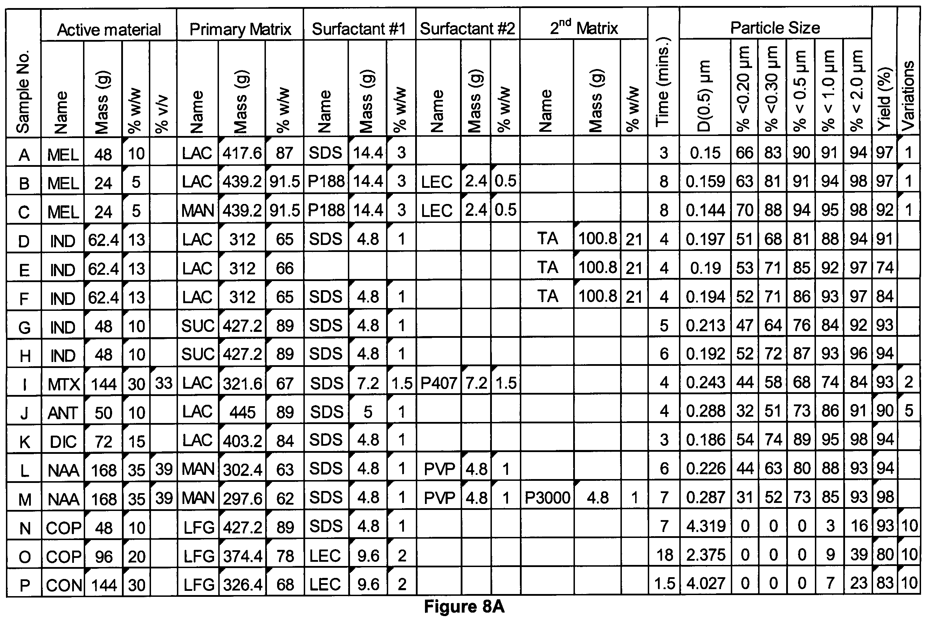

A range of actives, matrices and surfactants in a variety of combinations were milled using the Hicom mill. The details of these millings are shown in Figure 8A together with the particle size distributions of actives that were milled.

The data shows that the invention described herein can be used with the Hicom mill with its nutating action. The data in Figure 8A shows that a variety of actives can be milled small in very short times and give very good yields at 500 gram scale.

Sample N and O show that cocoa powder can be reduced to very fine sizes in short times using the invention describes here in in combination with the Hicom nutating mill. Likewise Sample P shows that this is also the case for cocoa nibs.

### Example 9: 1.5Gallon 1S

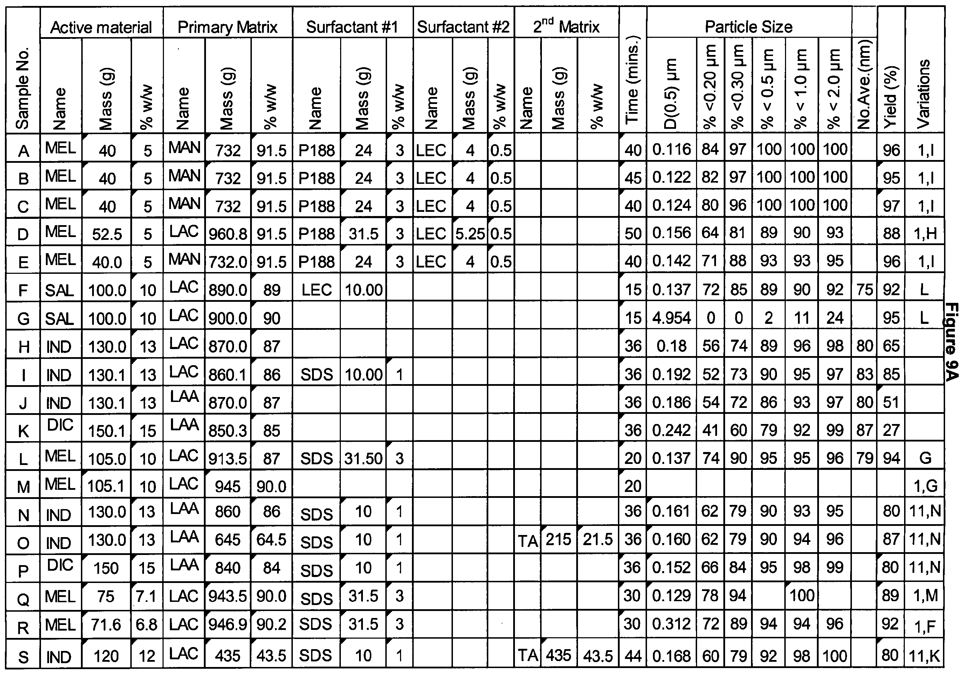

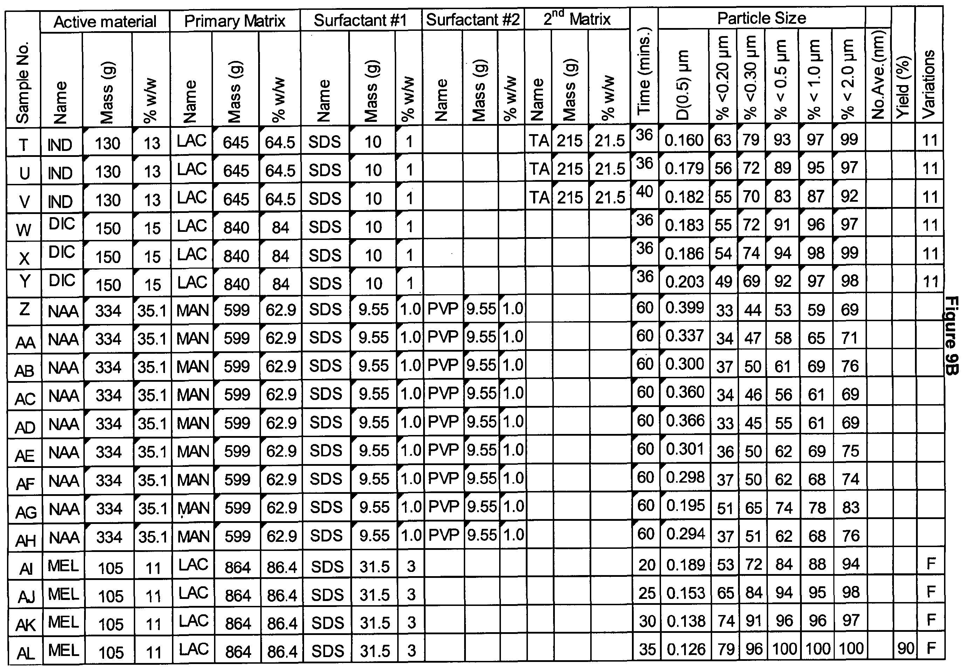

A range of actives, matrices and surfactants in a variety of combinations were milled using the 1.5 Gallon 1S mill. The details of these millings are shown in Figures 9A-B together with the particle size distributions of actives that were milled.

The following examples demonstrate the increased yield obtained when milling an active in a 1.5gallon 1S attritor mill with a surfactant as compared to no surfactant, with all other factors being identical. Sample J and N (Figure 9A) shows yields of 51% and 80%, without and with surfactant. Sample K and P (Figure 9A) show yields of 27% and 80%, without and with surfactant, while sample L (Figure 9A) show a yield of 94% with surfactant and the control without surfactant (sample M, Figure 9A) resulted in no yield due to caking within the mill.

The following examples illustrates that milling an active in a 1.5gallon 1S attritor mill with a surfactant as compared to without surfactant and all other factors identical, leads to smaller particle size after milling. Sample F and G (Figure 9A) shows a D(0.5) of 0.137 and 4.94 with or without surfactant, while sample K and P (Figure 9A) shows D(0.5) of 0.242 and 0.152 without and with surfactants.

The series of samples AI-AL are timepoints taken from a single meloxicam milling. The data demonstrates that the size of the actives decreases with milling time.

Other samples such as A-E show examples were surfactants suitable for use with IV formulations can be used to manufacture very small particles.

Sample M was a milling of meloxicam in lactose monohydrate without surfactant. 3 minutes into the milling the mill refused to turn. The milling was stopped and started again but only ran for another 3 minutes before stopping again. At this point the mill was taken apart and no evidence of caking was found. However the powder had a gritty feeling to it and was locking the medium and shaft such that it was not possible to turn. The media was weighed and it as found that 150 grams of powder was on the media indicating that it was sticking to the media and making it hard to move. At this point the mill was re-assembled and the powder and media put back in. 30.4 grams of SDS was included in the milling making it similar to milling L. After the addition of the surfactant the mill was run for another 14 minutes (giving a total of 20 mins) without incident. After offloading the powder the media was weighed and the weigh of powder on the media was only 40.5 grams. This indicates the addition of surfactant has improved the milling performance and ability to mill the powder.

Some of the particle size data in Figures 9A-B was converted to a number average particle size and is shown in the tables. This number was calculated in the following way. The Volume distribution was transformed to the number distribution using the Malvern Mastersizer software. For each size bin the size of the bin was multiplied by the % of particles in the bin. This numbers were added together and divided by 100 to give the number average particle size.

**Example 10: Large scale 25/11kg**

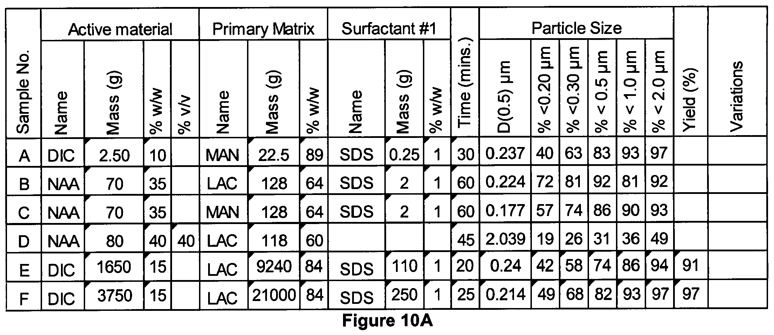

Sample A (Figure 10A) was milled in the Siebtechnik mill for 15 minutes. After this time the powder was completely caked onto the walls of the mill and the media. No powder could be removed to measure the particle size. At this point 0.25 g (1 w/w%) SLS was added to mill chamber and milling was then undertaken for a further 15 minutes. After the second period of milling in the presence of SLS powder was no longer caked onto the media and some free powder was also present. The observations made before and after the addition of the SLS demonstrate that the addition of the surfactant lessens the problem of caking. With the addition of surfactant the caked material could be recovered to become free powder again with small particle size.

Sample B-E was milled in horizontal Simoloyer mills. The details of these millings are shown in Figure 10A together with the particle size distributions of actives that were milled.

The data shows that the invention described herein can be used with Simoloyer mills with their horizontal attritor action. Of particular note is example E which was milled at 11kg scale. This demonstrates the invention described herein is suitable for commercial scale milling.

Sample F was milled in a vertical attritor mill (Union Process S-30). The details of this milling is shown in Figure 10A together with the particle size distribution of the active milled.

The data shows that the invention described herein can be used with a S-30 mills with its vertical attritor action. Of particular note is that this milling was at 25kg scale. This demonstrates the invention described herein is suitable for commercial scale milling.

**Example 11**: **Naproxen (not according to the invention)**

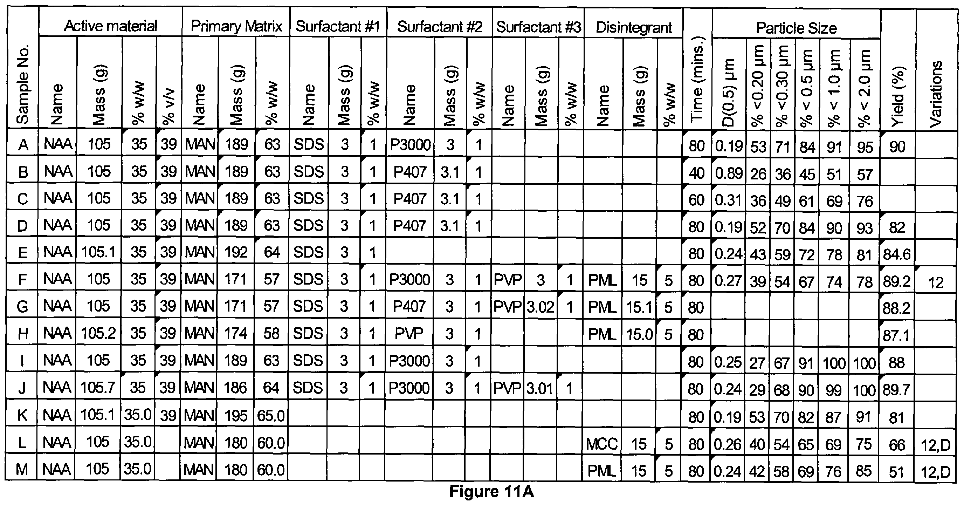

Naproxen was milled in mannitol with a range of surfactants using the 1/2 Gallon 1S mill. The details of these millings are shown in Figures 11A together with the particle size distributions of actives that were milled.

Naproxen acid milled in Mannitol with a surfactant (Sample A, D-J in Figure 11A) leads to higher yields, as compared to Naproxen acid milled in Mannitol without surfactant (Sample K,

Figure 11A). Naproxen acid milled in Mannitol and either microcrystalline cellulose or the disintegrant primellose (sample L or M, Figure 11A) leads to small particle size with D(0.5) around 0.25 in both cases.

**Example 12: Filtration**

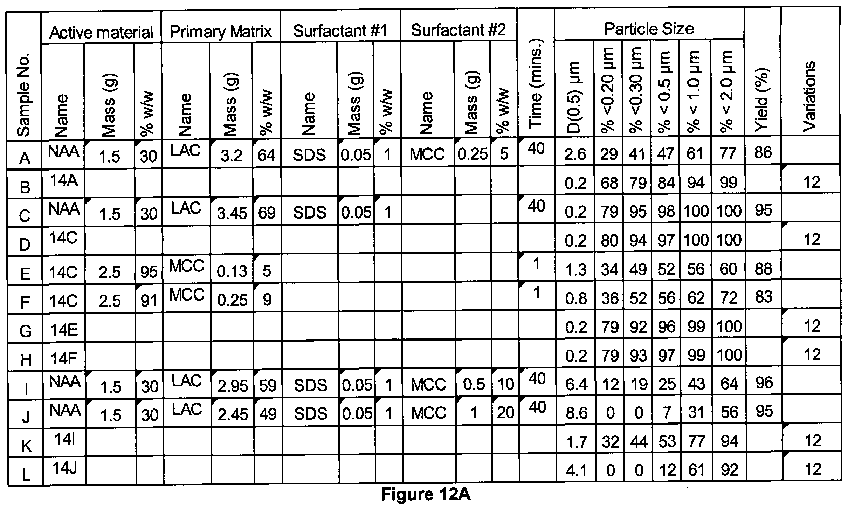

Some matrices, milling aids or facilitating agents that are used by this invention are not water soluble. Examples of these are microcrystalline cellulose and disintegrants such as croscarmellose and sodium starch glycolate. In order to more easily characterise the particle size of the active after milling with these materials filtration methods can be used to remove them allowing a characterisation of the active. In the following examples naproxen was milled with lactose monohydrate and microcrystalline cellulose (MCC). The particle size was characterised before and after filtration and the ability of the filters to let through the naproxen was demonstrated using HPLC assays. The milling details and the particle size are shown in Figure 12a. Note in this table the particle size with milling details is un-filtered. The particle size in the rows with no milling details is after filtration. The sample that was filtered is indicated in the Active material section. The HPLC assays were performed by taking samples before and after filtration through 10 micrometer poroplast filters. The samples taken were diluted to give a nominal concentration of 100 $\mu$g/ml. The HPLC assay data is shown in Table 12 Sample A was milled with 5% MCC. Before filtration the D50 was 2.5 $\mu$m, after filtration (sample B) the D50 was 183 nm. When sample B was assayed the concentration was 94 $\mu$g/ml indicating that filtration process retained little naproxen. A second milling (sample C) was undertaken without MCC. The D50 was 160nm as would be expected. After filtration (sample D) the particle size was unchanged indicating that if the filtration process did remove any naproxen then it was removed in an even way. Some of sample C was then milled with MCC for 1 minute. This is long enough to incorporate the MCC into the powder but not long enough to affect the particle size distribution. Two millings were undertaken. Sample E incorporated 5 % w/w MCC into the powder and Sample F 9 % w/w. After incorporation of the MCC the particle size increased dramatically. These samples where then filtered (Sample E and F) and the size remeasured. After filtration the particle size is the same as Sample C which was the starting material. The assay of samples E-H indicates that filtration did not remove any naproxen of any significance. The combination of particle size and assay data clearly shows that material such as MCC can easily and successfully be removed allowing the true particle size of the active to be measured.

Samples I and J were millings conducted with 10 and 20 % w/w MCC. The particle size post filtration is show as sample K and L. Again the filtration has delivered a reduction in particle size due to the removal of the MCC component. And again the HPLC assay of sample I-L shows little naproxen was lost during filtration.

This data also demonstrates that MCC can successfully be used as co matrix in the invention disclosed herein.

Table 12: The HPLC assay of naproxen before and after filtration of samples.

| Sample No. | HPLC Assay ($\mu$g/ml) |
|---|---|
| B | 94 |
| D | 93 |
| E | 99 |
| F | 96 |
| G | 98 |
| H | 97 |
| I | 94 |
| J | 89 |
| K | 91 |
| L | 84 |

### Example 13: Manufacture of Nanoformulation Capsules.

*Example 13(a) Manufacture of Metaxalone (100 mg) Nanoformulation Capsules.*

Milled powder (Example 7, Sample N) was manually encapsulated using a capsule filling device (Profil) into hard-gelatin capsules.

*Example 13(b): Manufacture of indomethacin (20 mg) Nanoformulation Capsules* (not according to the invention)

Indomethacin milled powder (750.0 g, Example 9, Sample T) was charged into the bowl of a KG-5 high shear granulator. Separately, a 30% solution of povidone K30 in purified water was prepared by dissolving 47.8 g of povidone in 111.6 g of purified water.

The high shear granulator was operated with an impeller speed of 250 rpm and a chopper speed of 2500 rpm. A portion of the povidone solution (80.3 g) was introduced into the granulator over a period of approximately 8 minutes using a peristaltic pump. An additional 30 g of purified water was then added to the granulation.

After the additions of povidone solution and water were completed, the wet granules were spread on to paper-lined trays to a thickness of approximately ½", and were dried in an oven at 70°C for approximately 1 hour. The granules were then manually screened through a 10 mesh hand screen, and spread on to paper-lined trays for additional drying. The granules were dried for a second hour, and then tested for loss on drying; the LOD value was 1.987%.

The dried granules were processed in a Quadro CoMill (20 mesh screen, 0.225 inch spacer) at 2500 rpm, yielding 689.9 g of milled granules having the final composition of 12.60% indomethacin, 62.50% lactose monohydrate, 20.86% tartaric acid, 0.95% sodium lauryl sulfate, 3.09% povidone K30.

The granules were manually filled into size 4 white opaque hard gelatin capsules using a MiniCap 100 Capsule Filling Machine set up with size 4 capsule change parts. The target fill weight of each capsule was 158.7 mg and the average empty capsule shell weight was 38 mg. Capsules were filled manually using a scraper and periodically tested for gross weight. Tamping and vibration were adjusted as necessary to achieve the target fill weight.

The filled capsules were polished in a Capsule Polishing Machine, yielding a net weight of 803 g of filled capsules (approximately 4,056 capsules).

*Example 13(c): Manufacture of indomethacin (40 mg) Nanoformulation Capsules* (not according to the invention)

Two separate granulation sublots were manufactured and combined to produce Indomethacin Nanoformulation capsules 40 mg.

Granulation sublot A was prepared by charging indomethacin milled powder (750.0 g, Example 9, Sample U) into the bowl of a KG-5 high shear granulator. Separately, a 30% solution of povidone K30 in purified water was prepared by dissolving 47.8 g of povidone in 111.5 g of purified water. The granulator was operated with an impeller speed of 250 rpm and a chopper speed of 2500 rpm. A portion of the povidone solution (80.3 g) was introduced into the granulator over a period of approximately 9 minutes, using a peristaltic pump. An additional 20 g of purified water was then added to the granulation.

After the additions of povidone solution and water were completed, the wet granules were spread on to paper-lined trays to a thickness of approximately ½".

Granulation sublot B was prepared by charging indomethacin milled powder (731.6 g, Example 9, Sample V and 18.4 g, Example 9, Sample U) into the bowl of a KG-5 high shear granulator. Separately, a 30% solution of povidone K30 in purified water was prepared by dissolving 47.8 g of povidone in 111.5 g of purified water. The granulator was operated with an impeller speed of 250 rpm and a chopper speed of 2500 rpm. A portion of the povidone solution (80.3 g) was introduced into the granulator over a period of approximately 10 minutes, using a peristaltic pump. An additional 20 g of purified water was then added to the granulation. After the additions of povidone solution and water were completed, the wet granules were spread on to paper-lined trays to a thickness of approximately ½". The wet granules from both sublots were dried in an oven at 70°C for approximately 2.5 hours. The granules were then manually screened through a 10 mesh hand screen, and spread on to paper-lined trays for additional drying. The granules were dried for another 1.5 hours, until the LOD value was 1.699%.

The dried granules were processed in a Quadro CoMill (20 mesh screen, 0.225 inch spacer) at 2500 rpm. The milled granules were then added to an 8 qt V-blender and mixed for 5 minutes, yielding 1390.7 g of granules with a final composition of 12.60% indomethacin, 62.50% lactose monohydrate, 20.86% tartaric acid, 0.95% sodium lauryl sulfate, 3.09% povidone K30.

An IN-CAP® automated capsule filling machine (Dott. Bonapace & C., Milano, Italy) was set up with size (2) 16 mm dosing disc and size (2) tamping pins. Milled granules were charged into the encapsulator, along with size 1 white opaque hard gelatin capsule shells. The target capsule fill weight was 317.7 mg, and the average empty capsule shell weight was 75 mg. Tamping pins 1-4 were all set to 9 mm, and the encapsulator was run at speed 2. Weight checks, closure checks, and appearance checks were performed every 15 minutes. Filled capsules were polished in a capsule polishing machine. The net weight of filled, polished capsules was 1225.5 g (approximately 3,183 capsules).

*Example 13(d): Manufacture of meloxicam (7.5 mg) Nanoformulation Capsules* (not according to the invention)

Milled powder (Example 9, Sample Q) was manually encapsulated using a capsule filling device (Cooper plate and capsule loader) into size "4" white-opaque hard-gelatin capsules. Upon encapsulation, each capsule contains 7.5mg active ingredient with a total fill weight of 105mg. The finished capsules were packaged in 40cc HDPE bottles (50 counts per bottle) with the bottles being enclosed using an induction seal.

**Example 14: Dissolution**

*Example 14(a): Dissolution rate of milled metaxalone*

The dissolution of milled metaxalone (100 mg) capsules (Example 13(a)), and a portion (equivalent to 100 mg metaxalone) of commercial Skelaxin® 800 mg (metaxalone) tablets (King Pharmaceuticals®, Inc., USA) were determined using dissolution equipment set up as USP Apparatus II (paddles) with a stirrer speed of 100 rpm. The dissolution media was 1000 ml of 0.01 M HCL (pH 2). The vessel temperature was 37°C. The capsules were weighted down with a wire sinker. Three to six test articles were tested and the data averaged for each time point. At each time point each dissolution vessel was automatically sampled through a 1 μm filter and analyzed in flow through UV/Vis cells. The data in Table 14a below report the percent dissolved of the amount of active in each test article, for the specified time points.

Table 14a. Dissolution profiles of Skelaxin Tablets (100 mg portion) and Metaxalone Nanoformulation Capsules 100 mg.

| Time (min) | Percent of Label Claim Dissolved (%) | |
| --- | --- | --- |
| | Metaxalone Nanoformulation Capsules 100 mg | Skelaxin (100 mg portion) |
| 0 | 0 | 0 |
| 5 | 4 | 0 |
| 9 | 43 | 1 |
| 13 | 75 | 1 |
| 20 | 88 | 2 |
| 30 | 93 | 5 |
| 40 | 93 | 7 |
| 50 | 94 | 9 |
| 60 | 94 | 11 |

The results demonstrate that the milled metaxalone capsules dissolve more quickly and more completely than the commercial reference metaxalone. Those of skill in the art will readily appreciate the advantages conferred by more rapid dissolution -- more active agent is available at any given time point. Put another way, an equal quantity of dissolved metaxalone may be obtained with an initially smaller dosage amount of milled metaxalone, as opposed to the larger initial dose required for the reference metaxalone to reach to the same quantity of dissolved metaxalone. Additionally, as the results make clear, the reference metaxalone does not achieve complete dissolution even by the final time point, while the milled metaxalone achieves about 87% dissolution within 20 minutes. Again, a smaller dose of milled metaxalone yields a quantity of dissolved metaxalone for which a larger dose of reference metaxalone would be required to equal.

In US Patent Application Publication 2005/0063913, nanoparticulate metaxalone formulations with mean particle sizes by weight of 381 nm and 139 nm, respectively, were compared to microparticulate Skelaxin Tablets in an *in-vivo* animal study (see Example 5 thereof). The study showed that the nanoformulations of metaxalone gave a much superior Tmax, Cmax and AUC compared to the Skelaxin. As the dissolution data above indicate that the nanoformulation manufactured with this invention has far superior *in-vitro* dissolution behaviour one skilled in the art would expect such nanoformulations to have similarly superior *in-vivo* performance compared to reference microformulations.

*Example 14(b): Dissolution rate of milled indomethacin* (not according to the invention)

In this example, dissolution rate is compared between 20mg and 40mg naonoformulations of the invention (Example 13(b) and 13(c)), and commercial reference indomethacin USP 25 mg capsules (Mylan Pharmaceuticals Inc). The dissolution was performed using Apparatus I (baskets) according to USP <711>. The dissolution medium (900 ml at 37°C) was100 mM citric acid buffer (pH 5.5 $\pm$ 0.05); the apparatus was stirred at 100 rpm. Sampling times were 5, 10, 20, 30, 45, and 60 min plus an additional time point at 75 min (250 rpm). Samples of 8 mL were taken and filtered through a 0.45 $\mu$m PVDF filter. The samples were assayed by UV-visible spectroscopy with a detection wavelength = 319 nm. The data in Table 14b below report the percent dissolved of the amount of active in each test article, for the specified time points.

Table 14b. Dissolution Profiles of Indomethacin Capsules USP (25 mg) and Indomethacin Nanoformulation Capsules (20 mg and 40 mg)

| Time (min) | Percent of Label Claim Dissolved (%) | | |
| --- | --- | --- | --- |
| | Indomethacin capsules USP, 25 mg | Indomethacin Nanoformulation Capsules 20 mg | Indomethacin Nanoformulation Capsules 40 mg |
| 0 | 0 | 0 | 0 |
| 5 | 20 | 47 | 31 |
| 10 | 28 | 83 | 66 |

(continued)

| Time (min) | Indomethacin capsules USP, 25 mg | Indomethacin Nanoformulation Capsules 20 mg | Indomethacin Nanoformulation Capsules 40 mg |
|---|---|---|---|
| | | Percent of Label Claim Dissolved (%) | |
| 20 | 36 | 99 | 93 |
| 30 | 40 | 100 | 96 |
| 45 | 43 | 100 | 96 |
| 60 | 46 | 101 | 97 |
| 75 | 63 | 101 | 97 |

The results demonstrate that the nanomilled indomethacin capsules dissolve more quickly and more completely than the commercial reference indomethacin. These same capsules were also tested in an *in-vivo* human clinical trial (as described in patent application, *"A novel formulation of indomethacin"*, filed as PCT/AU2010/_____ claiming priority to AU provisional application 2009901740) This trial (fasted leg) demonstrated that the 20 and 40 mg nanomilled indomethacin had faster onset compared to the commercial reference (50 mg) (Tmax = 1.1 hours for 20 mg nano, 1.25 hours for 40 mg nano and 2.0 hours for 50 mg reference) and that 40 mg nanomilled indomethacin had higher a higher Cmax compared to the commercial reference (50 mg) (Cmax = 2995 ng/ml for 40 mg nano and 2652 ng/ml for 50 mg reference). These *in-vivo* data demonstrate that the *in-vitro* dissolution test is indicative of the behaviour of an active pharmaceutical manufactured using this invention.

*Example 14(c): Dissolution rate of milled meloxicam* (not according to the invention)

In this example, dissolution rate is compared between a 7.5 mg nanoformulation of this invention (Example 13(d)), and two commercial reference products Mobicox® 7.5 mg Tablets and Mobic® 7.5 mg Capsules (Both Boehringer Ingelheim). Dissolution was performed using Apparatus II (paddles) according to USP <711>. The dissolution medium was 10 mM phosphate buffer (pH 6.1) with 0.1 % w/w sodium lauryl sulfate (500 ml at 37°C). The apparatus was stirred at 50 rpm. Samples were taken at various time points from 5 to 60 minutes. For each sample 1 mL was taken, filtered through a 0.45 $\mu$m filter and assayed by HPLC using a detection wavelength of 362 nm. The data in Table 14c below report the percent dissolved of the amount of active in each test article, for the specified time points.

Table 14C. Dissolution profiles of Commercial Meloxicam Tablets and Capsules and Meloxicam Nanoformulation Capsules

| Time (min) | Mobicox® Tablets 7.5 mg | Mobic® Capsules 7.5 mg | Meloxicam Nanoformulation Capsules 7.5 mg |
|---|---|---|---|
| | | Percent of Label Claim Dissolved (%) | |
| 0 | 0 | 0 | 0 |
| 5 | 39 | 19 | 44 |
| 10 | 50 | 43 | 68 |
| 15 | 57 | 52 | |
| 20 | | | 82 |
| 30 | 66 | 64 | 86 |
| 45 | | | 89 |
| 60 | 73 | 72 | 93 |

The results demonstrate that the milled meloxicam capsules dissolve more quickly and more completely than the commercial reference meloxicam. The capsules tested in this dissolution study were also tested in an *in-vivo* human clinical trial (as described in patent application, *"A novel formulation of meloxicam"*, PCT/AU2010/_____, claiming priority to AU provisional application 2009901742). This trial (fasted leg) demonstrated that the 7.5 mg nanomilled meloxicam had faster onset compared to the commercial reference (Tmax = 2.0 hours for nano, 5.0 hours reference) and that nanomilled meloxicam had higher a higher Cmax compared to the commercial reference (Cmax = 1087 ng/ml for nano and 628 ng/ml for reference). These *in-vivo* data demonstrate that the *in-vitro* dissolution test is indicative of the behaviour of an active pharmaceutical manufactured using this invention.

**Example 15: Bioavailability of milled metaxalone.**

This Example describes a Single-Dose, 5-Period, 5-Treatment, 5-Way Crossover Bioavailability Study of 2 Metaxalone Nanoformulations (200 mg and 400 mg) and Skelaxin® 800 mg under Fed and Fasted Conditions.

The phase I pharmacokinetic study described in this example uses Metaxalone Nanoformulation Capsules the same as or similar to those described in Example 13(a), and is conducted in accordance with the following protocol.

*INTRODUCTION*

Chemically, metaxalone is 5-[3,5-dimethylphenoxy)methyl]-2-oxazolidone. The empirical formula is $C_{12}H_{15}NO_3$, which corresponds to a molecular weight of 221.25 g/mol. Metaxalone is a white to almost white, odorless crystalline powder freely soluble in chloroform, soluble in methanol and in 96% ethanol, but practically insoluble in ether or water. The mechanism of action of metaxalone in humans has not been established, but may be due to general central nervous system depression. Metaxalone has no direct action on the contractile mechanism of striated muscle, the motor end plate, or the nerve fiber. Although plasma protein binding and absolute bioavailability of metaxalone are not known, the apparent volume of distribution (V/F~ 800 L) and lipophilicity (log P = 2.42) of metaxalone suggest that the drug is extensively distributed in the tissues. Metaxalone is metabolized by the liver and excreted in the urine as unidentified metabolites. Hepatic Cytochrome P450 enzymes play a role in the metabolism of metaxalone. Specifically, CYP1A2, CYP2D6, CYP2E1, and CYP3A4 and, to a lesser extent, CYP2C8, CYP2C9, and CYP2FC19 appear to metabolize metaxalone.

Metaxalone does not significantly inhibit major CYP enzymes such as CYP1A2, CYP2A6, CYP2B6, CYP2C8, CYP2C9, CYP2C19, CYP2D6, CYP2E1, and CYP3A4. Metaxalone does not significantly induce major CYP enzymes such as CYP1A2, CYP2B6, and CYP3A4 *in vitro.*

*OBJECTIVES*

The objective of this single-dose, open-label, randomized, 5-period, 5-treatment crossover study is to evaluate the relative bioavailability and pharmacokinetics of a test formulation of metaxalone 400 mg under fed and fasting conditions, and a test formulation of metaxalone 200 mg under fasting conditions, compared to an 800 mg oral dose of the commercially available reference product, Skelaxin® manufactured by King Pharmaceuticals under fed and fasting conditions.

The primary objectives of the study are:

To determine the relative bioavailability of metaxalone from the 2 x 100 mg and 4 x 100 mg Test capsules versus the 800 mg Reference tablet when administered to healthy subjects under fasted conditions.

To determine the effect of food on the rate and extent of absorption of a single dose of the 4 x 100 mg Test capsule formulation of metaxalone nanoformulation administered to healthy subjects.

To determine the effect of food on the rate and extent of absorption of a single dose of the 800 mg Reference tablet formulation of metaxalone administered to healthy subjects.

To evaluate the dose proportionality between a 200 mg (2 x 100 mg Test capsules) does and a 400 mg (4 x 100 mg capsules) dose of metaxalone nanoformulation administered to healthy subjects under fasting conditions.

*STUDY DESIGN SUMMARY*

This is a single-dose, open-label, randomized, 5-period, 5-treatment crossover study in which up to 40 healthy adult subjects will receive 5 separate single-dose administrations of metaxalone.

Subjects receiving the fed treatments will be administered the study drug after an overnight fast of at least 10 hours, followed by consumption of an FDA standard high-calorie, high-fat breakfast meal beginning 30 minutes prior to each dose.

Subjects receiving the fasting treatments will be administered the study drug following an overnight fast of at least 10 hours.

Subjects will be assigned numbers in an ascending order, based on successful completion of the screening process.

Subjects will receive each of the treatments listed below in randomized fashion during the five treatment periods:

| Treatment A: Fed conditions | Test Formulation Metaxalone Dose = 4 × 100 mg capsule |
|---|---|
| Treatment B: Fasting conditions | Test Formulation Metaxalone Dose = 4 × 100 mg capsule |
| Treatment C Fasting conditions | Test Formulation Metaxalone Dose = 1 × 200 mg capsule |
| Treatment D Fed conditions | Reference Product Skelaxin® Dose = 1 × 800 mg tablet King Pharmaceuticals |
| Treatment E Fasting conditions | Reference Product Skelaxin® Dose = 1 × 800 mg tablet King Pharmaceuticals |

Each drug administration will be separated by a washout period of at least 7 days. Treatments A and D will be orally administered along with 240 mL (8 fl. oz.) of room temperature tap water following a 10-hour overnight fast and standard high-fat, high-calorie breakfast administration. Treatments B, C, and E will be orally administered along with 240 mL (8 fl. oz.) of room temperature tap water following a 10-hour overnight fast.

After dosing, no food will be allowed until 4 hours post-dose. Except for the 240 mL of room temperature tap water provided with the dose, no water may be consumed for 1 hour prior through 1 hour post dose. Water consumption will follow the guidelines in Section 5.4. With the exception of the standard high-fat, high-calorie breakfast meal served with Treatments A and D, meals will be the same and scheduled at approximately the same times relative to dose for each study period.

Subjects who withdraw from the study will not be replaced.

During each study period, 6 mL blood samples will be obtained prior to each dosing and following each dose at selected times through 72 hours post-dose. A total of 115 pharmacokinetic (PK) blood samples will be collected from each subject, 23 samples in each study period. Plasma pharmacokinetic samples will be analyzed for metaxalone using a validated analytical method. Appropriate pharmacokinetic parameters will be calculated for each formulation using non-compartmental methods. In addition, blood will be drawn and urine will be collected for clinical laboratory testing at screening and at the end of the study.

*SUBJECT SELECTION*

*Inclusion Criteria*

All subjects must satisfy the following criteria to be considered for study participation:
Subject must be a male or non-pregnant, non-breastfeeding female.

Subject must be between 18 and 55 years of age (inclusive).

Subject's Body Mass Index (BMI) must be between 18 and 30 kg/m$^2$ (inclusive), and subject must weigh a minimum of 50 kg (110 lbs).

Female subjects must agree to use one of the following forms of birth control from screening until 14 days after completion of the study:

Vasectomized partner (at least 6 months prior to dosing)
Post-menopausal (at least 2 years prior to dosing)
Surgically sterile (bilateral tubal ligation, hysterectomy, bilateral oophorectomy) at least 6 months prior to dosing
Double barrier (diaphragm with spermicide; condoms with spermicide)
IUD (intra-uterine device)

Abstinence (must agree to use a double barrier method if they become sexually active during the study)

Implanted or intrauterine hormonal contraceptives in use for at least 6 consecutive months prior to study dosing and throughout the study duration

Oral, patch, and injected contraceptives in use for at least 3 consecutive months prior to study dosing and throughout the study duration.

Subject must voluntarily consent to participate in this study and provide their written informed consent prior to start of any study-specific procedures.

Subject is willing and able to remain in the study unit for the entire duration of each confinement period and return for outpatient visits.

Subject is willing and able to consume the entire high-calorie, high-fat breakfast meal in the designated timeframe required when assigned to a fed study period study period.

*Exclusion Criteria*

Subjects will be excluded for any of the following:

History or presence of clinically significant cardiovascular, pulmonary, hepatic, renal, hematologic, gastrointestinal, endocrine, immunologic, dermatologic, neurologic, oncologic, or psychiatric disease or any other condition that, in the opinion of the Investigator, would jeopardize the safety of the subject or the validity of the study results.

Specifically, subjects with history or presence of congestive heart failure, coronary artery disease, fluid retention, hypertension, ulcer disease or gastrointestinal bleeding, active kidney disease, or bleeding disorder.

Has a clinically significant abnormal finding on the physical exam, medical history, ECG, or clinical laboratory results at screening.

History or presence of allergic or adverse response to metaxalone or related drugs.

Has been on a significantly abnormal diet during the 4 weeks preceding the first dose of study medication.

Has donated blood or plasma within 30 days prior to the first dose of study medication.

Has participated in another clinical trial within 30 days prior to the first dose of study medication.

Has used any over-the-counter (OTC) medication, including nutritional supplements, within 7 days prior to the first dose of study medication.

Has used any prescription medication, except hormonal contraceptive or hormonal replacement therapy, within 14 days prior to the first dose of study medication.

Subjects that have discontinued the use of implanted, intrauterine, or injected hormonal contraceptives must not have used any for 6 months prior to study start.

Subjects that have discontinued the use of oral or patch hormonal contraceptives must not have used any for 1 month prior to study start.

Has been treated with any known enzyme altering drugs, such as barbiturates, phenothiazines, cimetidine, carbamazepine, etc., within 30 days prior to the first dose of study medication.

Has smoked or used tobacco products within 60 days prior to the first dose of study medication. Has any prior history of substance abuse or treatment (including alcohol) within the past 2 years.

Is a female with a positive pregnancy test result.

Has a positive urine screen for drugs of abuse (amphetamines, barbiturates, benzodiazepines, cocaine, cannabinoids, opiates).

Has had a positive test for, or has been treated for hepatitis B, hepatitis C or HIV.

*Restrictions*

Subject must not take any OTC medication, including nutritional supplements, within 7 days prior to the first dose of study medication until the end-of-study visit without evaluation and approval by the study investigator.

Subject must not take any prescription medication, with the exception of female hormonal contraceptives or hormone replacement therapy, from 14 days prior to the first dose of study medication until the end-of-study visit without evaluation and approval by the study investigator. Subject must not consume beverages and foods containing alcohol, grapefruit, or caffeine/xanthine from 48 hours prior to the first dose of study medication until the end-of-study visit. Subjects will be instructed not to consume any of the above products; however, allowance for an isolated single incidental consumption may be evaluated and approved by the study investigator based on the potential for interaction with the study drug.

Subject must not donate blood or plasma 30 days prior to the first dose of study medication until the end-of-study visit. It is recommended that blood/plasma donations not be made for at least 30 days after the end-of-study visit.

Subject must not use tobacco products from 60 days prior to the first dose of study medication until the end-of-study visit.

Subject must not engage in strenuous exercise from 48 hours prior to the first dose of study medication until the end-of-study visit.

Female subjects must utilize one of the following forms of contraception, if sexually active with a male partner, from screening until 14 days after completion of the study. Approved forms of contraception are:

Vasectomized partner (at least 6 months prior to dosing)
Post-menopausal (at least 2 years prior to dosing)
Surgically sterile (bilateral tubal ligation, hysterectomy, bilateral oophorectomy) at least 6 months prior to dosing
Double barrier (diaphragm with spermicide; condoms with spermicide)
IUD (intra-uterine device)
Abstinence (must agree to use a double barrier method if they become sexually active during the study.)
Implanted or intrauterine hormonal contraceptives must be used for at least 6 consecutive months prior to study dosing and throughout the study duration
Oral, patch, and injected contraceptives must be used for at least 3 consecutive months prior to study dosing and throughout the study duration.

Subjects who have discontinued the use of implanted, intrauterine, or injected hormonal contraceptives must not have used any for 6 months prior to study start.

Subjects who have discontinued the use of oral or patch hormonal contraceptives must not have used any for 1 month prior to study start.

*Screening*

Each potential study participant will have the following assessments by the Investigator or designee within 28 days prior to study start: medical history and demographic data, including sex, age, race, ethnicity, body weight (kg), height (cm), BMI ($kg/m^2$), and smoking habits. Each potential participant will receive a physical examination, electrocardiogram (ECG), and the laboratory tests for hematologic, hepatic, and renal function listed below. ECGs will be performed after subject has been in supine position for a minimum of 5 minutes. All potential subjects will be tested for hepatitis B, hepatitis C, and Human Immunodeficiency Virus (HIV) at screening. Urine drug screen tests will be conducted on all potential subjects. Serum pregnancy tests will be conducted on all female subjects.

Only medically healthy subjects with clinically acceptable laboratory profiles and ECGs will be enrolled in the study. The informed consent documents will be discussed with each potential participant, and each individual will sign an informed consent document for the study prior to any study-specific procedures being performed.

A positive test result for pregnancy, HIV, hepatitis B, hepatitis C, or urine drug screen will end the screening process.

*Laboratory Tests*

A Clinical Laboratory Improvement Amendments (CLIA) certified laboratory will perform the following clinical laboratory tests for this study:

Hematology

The following will be evaluated: hemoglobin, hematocrit, total and differential leukocyte count, red blood cell count (RBC), and platelet count.

Serum Chemistry

The following will be evaluated: albumin, blood urea nitrogen (BUN), creatinine, total bilirubin, alkaline phosphatase (ALP), aspartate transaminase (AST), alanine transaminase (ALT), sodium ($Na^+$), potassium ($K^+$), chloride ($Cl^-$), lactate dehydrogenase (LDH), calcium (Ca), uric acid, and glucose.

Serology

Blood will be tested for Hepatitis B Surface Antigen, Hepatitis C Antibody, and Human Immunodeficiency Virus (HIV).

Urinalysis

The following will be evaluated by an automated or manual urine "dipstick" method: pH, specific gravity, protein, glucose, ketones, bilirubin, blood, nitrite, leukocyte esterase, and urobilinogen. If protein, occult blood, nitrite, or leukocyte esterase values are out of range, a microscopic examination will be performed.

Urine Drug and Alcohol Screens

Urine samples will be tested for drugs of abuse (amphetamines, benzodiazepines, barbiturates, cannabinoids, cocaine, opiates) at screening. Urine samples will be tested for drugs of abuse and alcohol at each check-in.

Pregnancy Test (Female Subject Only)

A serum pregnancy test will be performed on all female subjects at screening. A urine pregnancy test will be performed on all female subjects at each check-in.

*STUDY PROCEDURES*

*Subject Assignment*

Forty subjects will be dosed in this study. Each subject will receive an assigned treatment sequence based on the randomization schedule prepared by the clinical site. Subjects will be randomized to receive either Treatment A, B, C, D, or E during the first study period. After a minimum washout of 7 days, each subject will crossover to receive an alternate treatment. At the completion of the study, each subject will have received a single dose of Treatment A, a single dose of Treatment B, a single dose of Treatment C, a single dose of Treatment D, and a single dose of Treatment E.

| Sequence Number | Period 1 Treatment | Period 2 Treatment | Period 3 Treatment | Period 4 Treatment | Period 5 Treatment |
|---|---|---|---|---|---|
| 1 | A | B | C | D | E |
| 2 | B | C | D | E | A |
| 3 | C | D | E | A | B |
| 4 | D | E | A | B | C |
| 5 | E | A | B | C | D |

The maximum duration of the study from screening to study exit will be approximately 59 days.

*Check-In Procedures*

All subjects will be asked to affirm that the exclusion criteria and restrictions have not been violated since the screening. The subjects' responses will be documented.
A urine sample will be collected from all subjects at each study check-in to screen for drugs of abuse (UDS) and alcohol. If at any time the drug or alcohol test is positive, the subject will be discontinued from study participation.
A urine sample will be collected from all female subjects for a urine pregnancy test at each check-in. This test must be negative for the subject to continue study participation.

*Confinement*

Subjects will be admitted to the research center at an appropriate time the evening prior to study drug administration to ensure a minimum 10-hour fast. Subjects will remain in the research center until completion of the 24-hour procedures for each study period and return for outpatient visits at approximately 36, 48, and 72 hours post-dose in each study period.

*Fasting/Meals/Beverages*

Fed Treatments (A and D)

An optional snack will be served the evening of check-in. All subjects will then be required to fast for at least 10 hours prior to consuming a standard breakfast. Subjects will receive a required FDA standard high-fat, high-calorie breakfast to begin 30 minutes prior to scheduled administration of the dose and to end (last bite taken) within 5 minutes prior to dosing. The subjects will fast for 4 hours thereafter. Standard meals will be provided at approximately 4 and 10 hours after drug administration and at appropriate times thereafter. Meal/snack menus will be the same for all study periods.

The following high-fat (approximately 50% of total caloric content of the meal), high-calorie (approximately 1000 calories) breakfast will be ingested approximately 30 minutes prior to administration of the drug.

> 2 eggs fried in butter
> 2 strips of bacon
> 2 slices of toast with butter
> 4 ounces of hash brown potatoes
> 8 ounces of whole milk

This meal contains approximately 150 protein calories, 250 carbohydrate calories, and 500-600 fat calories. An equivalent meal may be substituted with documentation of the menu and caloric contents.

Water will be allowed *ad lib* during the study except for 1 hour prior through 1 hour post dose.

Fasting Treatments (B, C, and E)

An optional snack will be served the evening of check-in. All subjects will then be required to fast for at least 10 hours prior to scheduled administration of the dose. Standard meals will be provided at approximately 4 and 10 hours after drug administration and at appropriate times thereafter. Meal/snack menus will be the same for all study periods.

Water will be allowed *ad lib* during the study except for 1 hour prior through 1 hour post dose.

*Drug Administration*

Each subject will receive the oral dose of the assigned metaxalone formulation with 240 mL (8 fl. oz.) of room temperature tap water. Subjects must swallow the study medication intact. The medication should NOT be crushed or chewed. A mouth check will be performed immediately after dose to ensure that the medication has been appropriately swallowed.

The subjects will remain seated, except as otherwise required for study procedures or personal needs, for the first 4 hours after dosing. Subjects will not be allowed to lie down, except as directed by clinical staff secondary to adverse events, for the first 4 hours after dosing.

*Blood Sampling, Processing and Shipment*

A total of 690 mL (115 × 6 mL samples) will be collected for PK analysis. In addition, approximately 40 mL of blood will be collected for screening and the end-of-study clinical laboratory evaluations. The total volume of blood collected will not exceed 730 mL.

Blood samples (1 × 6 mL) will be collected in vacutainer tubes containing $K_2$EDTA as a preservative, at 0 (pre-dose) and at 0.25, 0.5, 0.75, 1, 1.25, 1.5, 1.75, 2, 2.25, 2.5, 2.75, 3, 3.5, 4, 5, 8, 12, 16, 24, 36, 48, and 72 hours after dosing. The pre-dose blood sample will be collected within 60 minutes prior to each dose of study drug. Pre-dose blood samples obtained from backup subjects who are randomized into the study may exceed the pre-dose collection window. The time and date of collection for each sample will be recorded.

Blood samples will be centrifuged at approximately 3000 rpm for 10 minutes at 4 degrees Centigrade. The resulting plasma samples will be harvested and transferred into appropriately labeled polypropylene screw-cap tubes. PK samples will be placed in a storage freezer at minus 20 degrees Centigrade or lower within 60 minutes of blood draw. Samples will remain frozen until assayed. A more detailed description of plasma sample preparation requirements may be provided by the analytical laboratory. If such a description is provided, the method of sample preparation provided by the laboratory shall supersede those provided in this protocol and appropriate documentation shall be placed in the study master file.

The samples will be transferred to the analytical laboratory after completion of the study or at mutually agreed upon time points during the clinical conduct of the study. Prior to shipment, the samples will be appropriately packed in a Styrofoam® cooler containing dry ice. Sufficient dry ice will be added to ensure that the samples will remain frozen for at least 24 hours for local shipments and for at least 72 hours for remote shipments. The shipment will be accompanied by documentation containing the following information: name of the study drug product, protocol number, number of subjects, and number of samples included in the shipment.

*End-of-study Procedures*

Vital signs (blood pressure, pulse rate, respiration rate, and temperature) will be measured prior to the collection of the 72-hour blood sample at Study Period 5. Following the collection of the 72-hour blood sample at Study Period 5, all subjects will undergo a physical examination and ECG. The ECG will be performed after subject has been in supine position for a minimum of 5 minutes. Blood and urine will be collected for the same hematology, chemistry, and urinalysis tests performed during screening. When possible, end-of-study procedures will be performed in the event of a subject's early termination from the study.

*Safety Monitoring and Procedures*

At screening, prior to each administration of metaxalone, and at the end-of-study visit (prior to last PK blood collection) the following vital signs will be measured:

> blood pressure
> pulse rate
> respiration rate
> temperature

For purposes of qualifying any given subject for study participation, out-of-range vital signs may be repeated once. At approximately 2, 4, 24 and 72 hours after each dose of study drug the following vital signs will be collected:

> blood pressure
> pulse rate

Additional vital signs measurements may be performed as deemed medically necessary by research personnel. All vital signs measurements will be taken after the subject has completed a minimum 3-minute sit.

Subjects will be closely monitored during each confinement period in the research facility. Subjects will remain seated, except as otherwise required for study procedures or personal needs, for the first four hours after dosing. Should the need to move about occur during the first four hours after each dose, subjects may be escorted to such procedures or activities by research personnel as deemed medically necessary.

Subjects will be instructed to inform the study physician and/or research personnel of any adverse events (AEs) that occur at any time during the study.

Medical emergency personnel trained in advanced cardiac life support will be on site to monitor subjects during the confinement period in the research center. Emergency medical equipment including but not limited to intubation equipment and pulse oximetry shall be maintained on site to administer appropriate medical care should it be required. A physician will remain on site for a minimum of 4 hours after each dose administration and will be available immediately by cell phone or pager thereafter.

*ADVERSE EVENTS*

Subjects will be monitored for any adverse events from the beginning of confinement until the end-of-study visit. The Investigator or a medically qualified designee will review each event and assess its relationship to the study drug. Each sign or symptom will be graded for severity, and the date and time of onset, cessation and resolution will be recorded. Treatment of any adverse reactions will be evaluated and managed by a physician, either at the study site or at a nearby hospital emergency room, as appropriate.

*Definitions*

Adverse event (AE)

An AE is any untoward medical occurrence in a patient or clinical investigation subject administered a pharmaceutical product that does not necessarily have a causal relationship with the product. An AE can therefore be any unfavorable and unintended sign (including a new, clinically important abnormal laboratory finding), symptom, or disease, temporally associated with the product, whether or not related to the product.

Abnormal results of diagnostic procedures, including laboratory findings, are considered to be AEs if the abnormality:

results in study withdrawal
is associated with a serious adverse event (SAE)
is associated with clinical signs or symptoms
is considered by the physician to be of clinical significance

The relationship to the study treatment is characterized as:

| TERM | DEFINITION | CLARIFICATION |
|---|---|---|
| **Unrelated** | This category applies to those adverse events which, after careful consideration, are clearly and incontrovertibly due to extraneous causes (disease, environment, etc.) | |
| **Possibly** | This category applies to those adverse events for which, after careful medical consideration at the time they are evaluated, a connection with the Investigational Medicinal Product (IMP) administration appears unlikely but cannot be ruled out with certainty. | An adverse experience may be considered possibly related if or when (at least two of the following): It follows a reasonable temporal sequence from administration of the Investigational Medicinal Product (IMP). It could not readily have been produced by the subject's clinical state, environmental or toxic factors, or other modes of therapy administered to the subject. It follows a known pattern of response to the IMP. |
| **Probably** | This category applies to those adverse events which, after careful medical consideration at the time they are evaluated, are felt with a high degree of certainty to be related to the IMP. | An adverse experience may be considered probably related if or when (at least three of the following): It follows a reasonable temporal sequence from administration of the IMP. It could not be reasonably explained by the known characteristics of the subject's clinical state, environmental or toxic factors or other modes of therapy administered to the subject. It disappears or decreases on cessation or reduction in dose. There are important exceptions when an adverse event does not disappear upon discontinuation of the drug, yet drug-relatedness clearly exists. It follows a known pattern of response to the IMP. |

Serious Adverse Events (SAE)

A serious AE (SAE) is any untoward medical occurrence that at any dose:

Results in death

Is life threatening

Requires inpatient hospitalization or prolongation of existing hospitalization

Results in persistent or significant disability/incapacity

Is a congenital anomaly

Is an important medical event

Medical and scientific judgment should be exercised in deciding whether it is appropriate to consider other situations serious, such as important medical events that may not be immediately life threatening or result in death or hospitalization but may jeopardize the subject or may require intervention to prevent another of the outcomes listed in the definition above. Examples of such events are intensive treatment in an emergency room or at home for allergic bronchospasm, blood dyscrasias, or convulsions that do not result in hospitalization, or development of drug dependency or drug abuse.

An elective hospital admission to treat a condition present before exposure to the study drug, or a hospital admission for a diagnostic evaluation of an AE, does not qualify the condition or event as an SAE.

A newly diagnosed pregnancy in a subject who has received a study drug is not considered an SAE unless it is suspected that the study drug interacted with a contraceptive method and led to the pregnancy. A congenital anomaly in an infant born to a mother who was exposed to the study drug during pregnancy is an SAE.

The investigator must report all SAEs immediately, and no later than 24 hours after first becoming aware of the event by completing the SAE form.

At the time of first notification of an SAE, the following information should be provided by the study site if available:

Subject's study number and initials
Subject's date of birth
Subject's gender
Date of first dose of study drug(s)
Date of last dose of study drug(s), if applicable
AE term
Time and date of occurrence of the event
A brief description of the event, outcome to date, and any actions taken
The seriousness criteria(on) that were met
Concomitant medication at onset of the event
Relevant medical history information
Relevant laboratory test findings
Investigator's opinion of the relationship to study drug. ("Is there a reasonable possibility that the study drug caused the SAE? Yes or no?").

Whether and when the subject's treatment assignment was unblinded

Any missing or additional relevant information concerning the serious (or unexpected) AE should be provided in a written follow-up report.

The investigator is required to comply with applicable regulations regarding the notification of his/her IRB or IEC.

Pregnancy

All women of reproductive potential who participate in the trial should be counseled on the need to practice adequate birth control and on the importance of avoiding pregnancy during study participation. Women should be instructed to contact the investigator or study staff immediately if pregnancy occurs or is suspected.

*Follow-up of Subjects With an Adverse Event*

Any AE will be followed to a satisfactory resolution, until it becomes stable, or until it can be explained by another known cause(s) (ie, concurrent condition or medication) and clinical judgment indicates that further evaluation is not warranted. All findings relevant to the final outcome of an AE must be reported in the subject's medical record.

*GENERAL CONSIDERATIONS*

*Basic Principles*

This research will be carried out in accordance with the protocol, good clinical practices (GCPs), and applicable regulatory requirements(s) including clinical research guidelines established by the Basic Principles defined in the U.S. 21 CFR Parts 50, 56, and 312 and the principles enunciated in the Declaration of Helsinki (revised version Seoul 2008).

*Institutional Review Board*

This protocol will be reviewed by an appropriate IRB and study enrollment will not commence until the Board has approved the protocol or a modification thereof. The Board is constituted and operates in accordance with the principles and requirements described in the U.S. Code of Federal Regulations (21 CFR Part 56).

*Informed Consent*

Written informed consent will be obtained from each subject prior to performing any baseline study-specific evaluations. The informed consent document is prepared by the Investigator or designee, subject to review and approval by the Sponsor, and forwarded to a qualified IRB for final review and approval. The IRB-approved document must contain, at minimum, the eight basic elements of informed consent. Only the most recently IRB-approved Informed Consent Document must be used to consent prospective study subjects. One copy of the signed and dated informed consent document will be given to the subject and the original retained by the Investigator/site.

*Indications for Subject Withdrawal*

Subjects will be free to withdraw at any time for any reason, or they may be withdrawn if necessary, to protect their health and safety or the integrity of the study data. The final report will include reasons for withdrawals.

*Termination of the Study*

The Principal Investigator reserves the right to terminate the study in the interest of subject safety and welfare. The Sponsor reserves the right to terminate the study at any time for administrative reasons.

*Documentation*

All documents pertaining to the study, including a copy of the approved protocol, copy of the informed consent document and Health Insurance Portability and Accountability Act (HIPAA) documents, completed case report forms (where applicable), drug accountability and retention records, and other study related documents will be retained in the permanent archives of the study site. These will be available for inspection at any time by the Sponsor or the FDA. Per 21 CFR 312, record retention for this study is required for a period of 2 years following the date on which this study agent is approved by the FDA for the marketing purposes that were the subject of this investigation; or, if no application is to be filed or if the application is not approved for such indication, until 2 years following the date on which the entire study (not merely the Investigator's portion of the study, if it involved more than one investigator) is completed, terminated, or discontinued, and the FDA is notified.

PHARMACOKINETIC ANALYSIS

*Analytical Methodology*

A full validation of a sensitive LC-MS-MS assay for metaxalone in plasma, including precision, accuracy, reproducibility, and selectivity, will be provided to the Sponsor. The validation report will include the stability of frozen samples, limit of quantitation, recovery, and Watson LIMS summary tables. The samples from all evaluable subjects completing at least one study period will be analyzed.

*Pharmacokinetic Analysis*

Pharmacokinetic parameters for metaxalone will be calculated using non-compartmental analysis. The following pharmacokinetic parameters will be determined:
The maximum plasma concentration ($C_{max}$) and time to $C_{max}$ ($T_{max}$) will be taken directly from the data. The elimination rate constant, $\lambda_z$, will be calculated as the negative of the slope of the terminal log-linear segment of the plasma concentration-time curve; the range of data to be used will be determined by visual inspection of a semi-logarithmic plot of concentration vs. time. Elimination half-life ($T_{1/2}$) will be calculated according to the following equation:

$$T_{1/2} = 0.693 / \lambda_z$$

Area under the curve to the final sample with a concentration greater than the LOQ ($AUC_{last}$) will be calculated using the linear trapezoidal method and extrapolated to infinity using:

$$AUC_{inf} = AUC_{last} + C_{last}/ \lambda_Z$$

where $C_{last}$ is the final concentration □ LOQ.

All evaluable subjects completing at least one study period will be included in the pharmacokinetic and statistical analysis. Pharmacokinetic calculations will be performed using appropriate software, e.g. WinNonlin (Pharsight Corporation) and/or SAS® for Windows® (SAS Institute).

The relative bioavailability of the test formulation of metaxalone will be assessed under fasting and fed conditions using $AUC_{last}$ and $AUC_{inf}$ after the 4 x 100 mg treatments (Treatment A-fed, Treatment B-fasting), compared to the 1 x 800 mg Skelaxin treatments (Treatment D-fed, Treatment E-fasting). The relative bioavailability will be calculated for individual subjects according to the following equation,

$$F = [Dose(ref)*AUC(test)] / [Dose(test)*AUC(ref)],$$

where Dose(ref) = 800 mg, Dose(test) = 400 mg, AUC(test) = $AUC_{last}$ or $AUC_{inf}$ after administration of the test formulation, and AUC(ref) = $AUC_{last}$ or $AUC_{inf}$ after administration of the reference product. Fasting and fed treatments will be assessed separately and the bioavailability estimates under each condition will be summarized using descriptive statistics.

The dose-proportionality of metaxalone in the test formulation will be assessed using data acquired after administration of Treatment B (4 x 100 mg, fasting) and Treatment C (2 x 100 mg, fasting). The pharmacokinetic exposure parameters $C_{max}$, $AUC_{last}$, and $AUC_{inf}$ for individual subjects will be dose-normalized by dividing through by the administered dose (200 mg or 400 mg). The dose-normalized parameters will then be compared using an ANOVA model, as described in Section 8.3.

Statistical Analysis

Comparison of the log-transformed pharmacokinetic parameters $C_{max}$, $AUC_{last}$, and $AUC_{inf}$ for metaxalone across treatments will be performed using an analysis of variance (ANOVA) model and the two one-sided t-tests procedure. The ANOVA model will include factors for sequence, subject within sequence, treatment, and period. The ratios of the geometric means (test to reference) and 90% confidence intervals will be reported. Statistical analyses will be performed using appropriate software, e.g. WinNonlin (Pharsight Corporation) and/or SAS® for Windows® (SAS Institute).

*DRUG SUPPLIES*

Sufficient quantities of the study drug formulation to allow completion of this study will be supplied. Study drug formulations of metaxalone nanoformulation capsules 100 mg and Skelaxin® 800 mg tablets will be shipped to the clinical research site pursuant to site Standard Operating Procedures (SOPs). Retention samples of investigational metaxalone will not be required. Upon receipt of the study drug products, the supplies will be inventoried and stored in an environmentally controlled and secure, limited access area. The lot numbers of the drugs along with the expiration dates (where available) will be recorded and copies of the Certificate of Analysis (where available) will be maintained on file.

Records will be maintained of the receipt and dispensation of the drugs supplied. At the conclusion of the study, any unused study drug will be returned to the sponsor or destroyed by the site pursuant to written authorization by the sponsor and applicable federal and state regulations.

*ADMINISTRATIVE ISSUES*

The Investigator is referred to the Skelaxin® package insert, information provided during the study initiation visit, information provided by the study monitor, and ICH Guidelines for Good Clinical Practice for information regarding the study drug, details, or general considerations to be followed during the course of this study.

EVENTS SCHEDULE

| PROCEDURE | SCREENING | STUDY | END-OF-STUDY |
|---|---|---|---|
| Informed consent | X | | |
| Medical and medication histories | X | X | |

(continued)

| PROCEDURE | SCREENING | STUDY | END-OF-STUDY |
|---|---|---|---|
| ECG | X | | X |
| Vital signs | X | X | X |
| Physical examination | X | | X |
| Biochemistry, hematology, urinalysis | X | | X |
| Serology | X | | |
| Urine drug screen | X | | |
| Urine drug and alcohol screen | | X | |
| Pregnancy test (female subjects) | X | X | |
| Standard high-fat, high-calorie breakfast[1] | | X | |
| Drug administration | | X | |
| Blood sample collection for pharmacokinetic analysis | | X | |
| Adverse events | | X | X |
| [1] Treatments A and D only. Refer to protocol text for details. | | | |

**Example 16: Efficacy and safety of milled metaxalone**

This Example describes a Phase II, randomized study of Metaxalone Nanoformulation Capsules versus commercial metaxalone tablets for the treatment of acute, painful musculoskeletal conditions.

The phase II efficacy study described in this example uses Metaxalone Nanoformulation Capsules the same or similar to those described in Example 13(a), and is conducted in accordance with the following protocol; however, the dosage of the Metaxalone Nanoformulation Capsules may be adjusted from that described in Example 13(a) based on the results of interim pharmacokinetic studies.

*OBJECTIVES:*

The primary objective of this study is to evaluate the time to onset of relief of discomfort from acute, painful musculoskeletal conditions for Metaxalone Nanoformulation Capsules compared with the standard formulation of metaxalone in subjects with acute, painful musculoskeletal conditions. The secondary objective of this study is to evaluate the analgesic efficacy and safety of Metaxalone Nanoformulation Capsules compared with commercial metaxalone tablets.

*NUMBER OF SUBJECTS:*

Planned enrollment (and/or completion): Approximately 200 subjects (100 in each treatment group) will be enrolled.

*SUBJECT POPULATION:*

*Inclusion Criteria:*

A subject will be eligible for study entry if all of the following inclusion criteria are met:

1. Is male or female $\geq$ 18 and $\leq$ 80 years of age.
2. Has a body weight of $\geq$ 45 kg and a body mass index (BMI) $\leq$ 35 kg/m$^2$.
3. If female and of childbearing potential, is nonlactating and nonpregnant (has negative pregnancy test results at screening [serum] and on the day of commencement of dosing [urine]).
4. If female, is either not of childbearing potential (defined as postmenopausal for at least 1 year or surgically sterile [bilateral tubal ligation, bilateral oophorectomy, or hysterectomy]) or practicing 1 of the following medically acceptable methods of birth control:

> a. Hormonal methods such as oral, implantable, injectable, or transdermal contraceptives for a minimum of 1 full cycle (based on the subject's usual menstrual cycle period) before the study drug administration.
> b. Total abstinence from sexual intercourse (since the last menses before study drug administration).
> c. Intrauterine device (IUD).
> d. Double-barrier method (condoms sponge, diaphragm, or vaginal ring with spermicidal jellies or cream).

5. Is in good health, in the opinion of the investigator.
6. Is able to provide written informed consent to participate in the study and able to understand the procedures and study requirements.
7. Must voluntarily sign and date an informed consent form (ICF) that is approved by an Institutional Review Board (IRB) prior to the conduct of any study procedure.
8. Is willing and able to comply with study requirements, complete the pain evaluations, and return for follow-up 7 $\pm$ 2 days after completion of the study.
9. Is a suitable candidate for the study for any other medically sound reasons.

*Exclusion Criteria:*

A subject will not be eligible for study entry if any of the following exclusion criteria are met:

1. Has a known history of allergic reaction or clinically significant intolerance to metaxalone.
2. Has tested positive either on the urine drug screen or on the alcohol breathalyzer test. Subjects who test positive at screening only and can produce a prescription for the medication from their physician may be considered for study enrollment at the discretion of the investigator.
3. Has known or suspected history of alcoholism or drug abuse or misuse within 2 years of screening or evidence of tolerance or physical dependence before dosing with the study drug.
4. Has received or will require any medication (except hormonal contraceptives, vitamins, or nutritional supplements) within 5 half-lives (or, if half-life is unknown, within 48 hours) before dosing with study drug.
5. Has any clinically significant unstable cardiac, respiratory, neurological, immunological, hematological, or renal disease or any other condition that, in the opinion of the investigator, could compromise the subject's welfare, ability to communicate with the study staff, or otherwise contraindicate study participation.
6. Has a history or current diagnosis of a significant psychiatric disorder that, in the opinion of the investigator, would affect the subject's ability to comply with the study requirements.
7. Is receiving systemic chemotherapy, has an active malignancy of any type, or has been diagnosed with cancer with 5 years of screening (excluding squamous or basal cell carcinoma of the skin).
8. Has a history of clinically significant (investigator opinion) gastrointestinal (GI) event within 6 months before screening or has any history of peptic or gastric ulcers or GI bleeding.
9. Has a surgical or medical condition of the GI or renal system that might significantly alter the absorption, distribution, or excretion of any drug substance.
10. Is considered by the investigator, for any reason (including, but not limited to, the risks described as precautions, warnings, and contraindications in the current version of the Investigator's Brochure [IB] for Metaxalone Nanoformulation Capsules), to be an unsuitable candidate to receive the study drug.
11. Has history of chronic use (defined as daily use for > 2 weeks) of NSAIDs, opiates, or glucocorticoids (except inhaled nasal steroids and topical corticosteroids), for any condition within 6 months before dosing with study drug. Aspirin at a daily dose of $\leq$ 325 mg is allowed for cardiovascular (CV) prophylaxis if the subject has been on a stable dose regimen for $\geq$ 30 days before screening and has not experienced any relevant medical problem.
12. Has a significant renal or hepatic disease, as indicated by the clinical laboratory assessment (results $\geq$ 3 times the upper limit of normal [ULN] for any liver function test, including aspartate aminotransferase [AST], alanine aminotransferase [ALT], and lactate dehydrogenase, or creatinine $\geq$ 1.5 times the ULN) or has any clinically significant laboratory findings at screening that in the investigator's opinion contraindicate study participation.
13. Has significant difficulties swallowing capsules or is unable to tolerate oral medication.
14. Previously participated in another study of Metaxalone Nanoformulation Capsules, or received any investigational drug or device or investigational therapy within 30 days before screening.
15. Is deemed to be unsuitable for participation in the study for any medically sound reason.

*DESIGN:*

This is a phase II study to evaluate the efficacy and safety of Metaxalone Nanoformulation Capsules in subjects with discomforts associated with acute, painful musculoskeletal conditions. Eligible subjects will complete all screening procedures within 28 days before initiation of dosing.

Subjects will assess their baseline pain intensity (VAS) before receiving study drug (predose, Time 0) and their pain intensity (VAS) and pain relief (5-point categorical scale) at appropriate time points after Time 0.

*STUDY DRUG:*

Metaxalone Nanoformulation Capsules

*REFERENCE PRODUCTS:*

Commercially available metaxalone tablets

*TREATMENT REGIMENS*

The treatment regimens will be determined based on the results of pharmacokinetic studies comparing Metaxalone Nanoformulation Capsules and commercial metaxalone tablets.

*STUDY DURATION:*

Up to approximately 12 weeks for each subject, including a 4-week screening period and a posttreatment Follow-up Visit approximately 1 week after dosing with study drug.

*INVESTIGATIVE SITES OR COUNTRIES:*

Two study sites in the United States (US).

*STUDY ENDPOINTS:*

*Efficacy Endpoints:*

The primary efficacy endpoint is time to onset of relief of discomforts associated with acute, painful musculoskeletal conditions (measured as time to perceptible discomfort relief confirmed by meaningful discomfort relief).

The secondary endpoints are the following:

- The sum of total pain relief (TOTPAR) over 0 to 12 hours (TOTPAR-12) after Time 0.
- TOTPAR over 0 to 4 hours (TOTPAR-4) and over 0 to 8 hours (TOTPAR-8) after Time 0.
- VAS pain intensity difference (VASPID) at each scheduled time point after Time 0.
- VAS pain intensity score at each scheduled time point.
- VAS summed pain intensity difference (VASSPID) over 0 to 4 hours (VASSPID-4), over 0 to 8 hours (VASSPID-8), and over 0 to 12 hours (VASSPID-12) after Time 0.
- Summed pain relief and intensity difference (sum of TOTPAR and VASSPID [SPRID]) over 0 to 4 hours (SPRID-4), over 0 to 8 hours (SPRID-8), and over 0 to 12 hours (SPRID-12) after Time 0.
- Pain relief score at each scheduled time point after Time 0.
- Peak pain relief.
- Time to peak pain relief.
- Time to first perceptible pain relief.
- Time to meaningful pain relief.
- Proportion of subjects using rescue medication.
- Time to first use of rescue medication (duration of analgesia).
- Patient's global evaluation of study drug.
- Any other clinically relevant endpoints.

*Safety Endpoints:*

The safety endpoints are the incidence of treatment-emergent AEs (TEAEs) and changes in vital sign measurements.

*STATISTICAL METHODS SUMMARY:*

*Analysis Populations:*

The analysis populations include the following:

- The intent-to-treat (ITT) population will consist of all subjects who are treated with study drug and who have at least 1 pain relief assessment after Time 0. The ITT population is the primary population for the efficacy analysis.
- The per-protocol (PP) population will consist of all ITT subjects who remain in the study for at least 12 hours of treatment and who do not incur a major protocol violation that would challenge the validity of their data. This population will be utilized to evaluate the sensitivity of the primary efficacy analysis.
- The safety population will include all subjects who are treated with study drug. The safety population is the population for all safety assessments.

*Subject Characteristics:*

Demographic and baseline characteristics (including age, sec, race, weight, height, BMI, medical history, study duration, and baseline values of efficacy variables) will be summarized for each treatment group and for the overall population by descriptive statistics. No formal statistical analyses will be performed.

*Efficacy Analyses:*

The null hypothesis in this study is that the time to onset of relief of discomfort for commercially available metaxalone is equal to the time to onset of analgesia for Metaxalone Nanoformulation Capsules. It will be analyzed using analysis of covariance (ANCOVA) models, which include treatment effect and significant covariates. The effect of potential covariates, such as sex and baseline pain intensity, will be assessed using appropriate ANCOVA models. The analysis will be based on a 2-sided test as the significance level of 0.05.
Other comparisons between the treatment regimens will be considered secondary. No P value adjustment will be made for multiple endpoints or multiple comparisons. Each efficacy endpoint will be summarized descriptively by treatment group.

For continuous secondary endpoints such as pain intensity score, VASPID at each scheduled time point, peak pain intensity, TOTPAR-4, TOTPAR-8, TOTPAR-12, VASSPID-4, VASSPID-8, VASSPID-12, SPRID-4, SPRID-8, and SPRID-12,descriptive statistics (such as mean, standard error, median, minimum, and maximum) will be provided for each treatment regimen. Nominal P values from 2-sample tests comparing the placebo group with other treatment groups will be provided, but no formal statistical inferences will be drawn on the basis of these tests.

For ordinal secondary endpoints, such as pain relief at each scheduled time point, peak pain relief, and global evaluation of study drug, descriptive summaries will be provided to include the number and percentage of subjects within each category for each treatment group. Nominal P values from Fisher's exact tests (or chi-square tests, as appropriate) comparing the placebo group with other treatment groups will be provided, but no formal statistical inferences will be drawn on the basis of these tests.

For each time-to-event endpoint, the Kaplan-Meier method will be used to evaluate the treatment effect. Time to onset of relief of discomforts (measured as time to perceptible discomfort relief confirmed by meaningful discomfort relief) will be based on data collected using the 2-stopwatch method. Time to onset of discomfort relief will be right-censored at 12 hours for subjects who do not experience both perceptible discomfort relief and meaningful discomfort relief during the 12-hour interval after Time 0. The summary table will provide the number of subjects analyzed, the number of subjects censored, estimates for the quartiles, and 95% confidence intervals (CIs) for the estimated median and the restricted mean estimate. P values form the Wilcoxon or log-rank tests (as appropriate) will also be used to examine treatment effect. Cox proportional hazard models will be used to explore such potential covariates as sex and baseline pain intensity, if appropriate.
For the proportion of subjects using rescue medication, a logistic regression model that adjusts for baseline pain intensity, if appropriate, will be used to evaluate the treatment effect. Subgroup analysis by sex may be performed if it is confirmed to be a statistically significant covariate for TOTPAR-12. Baseline values are defined as the last measurements taken before dosing with a study drug.

For pain intensity, missing observations will be imputed using baseline-observation-carried-forward (BOCF) for subjects who withdraw from the study due to lack of efficacy or an AE/intolerance to study drug. The BOCF imputation will be applied in place of all scheduled assessments after the time of early termination due to lack of efficacy or an AE/intolerance to study drug using the baseline observation taken before Time 0.

For subjects who withdraw from the study due to reasons other than lack of efficacy or an AE/intolerance to study drug, missing observations for pain intensity and pain relief will be imputed using last-observation-carried-forward (LOCF). The LOCF imputation will be applied in place of all scheduled assessments after the time of early termination due to reasons other than lack of efficacy or an AE/intolerance to the drug.

For subjects who take any dose of rescue medication, subsequent measures after the first dose of rescue medication will be disregarded. Instead, all scheduled assessments after the first dose of rescue medication will be imputed using BOCF using the baseline observation taken before Time 0. Single missing data points will be imputed using linear interpolation, if they do not occur at the end of the study. For other conditions before early termination or rescue medication, missing data will be imputed using LOCF.

*Safety Analysis:*

Data listings will be provided for protocol-specified safety data. The Medical Dictionary for Regulatory Activities (MedDRA) (Version 9.1 or higher) will be used to classify all AEs with respect to system organ class and preferred term. Adverse event summaries will include only TEAEs, which will be summarized for each treatment group. Fisher's 2-sided exact test will be used to compare the rates of occurrence between the commercially available metaxalone tablets and Metaxalone Nanoformulation Capsule groups for all TEAEs.

*Sample Size:*

The sample size will be sufficient to determine statistically significant differences between Metaxalone Nanoformulation Capsules and commercially available metaxalone tablets in the primary efficacy endpoint.

**Claims**

1. A method for producing a composition, comprising the steps of:
   dry milling a solid biologically active material and a millable grinding matrix in a mill comprising a plurality of milling bodies, for a time period sufficient to produce particles of the biologically active material dispersed in an at least partially milled grinding matrix,
   wherein the biologically active material is metaxalone, and
   wherein the particles have a median particle size, determined on a particle volume basis and by laser diffraction, equal to or less than 500 nm.

2. The method of claim 1 where the composition produced by said method comprises particles of the biologically active compound at or above a volume fraction of 25 v/v%.

3. The method of claim 1 or claim 2, wherein the particles have a median particle size, determined on a particle volume basis, equal to or less than a size selected from the group consisting of: 400 nm, 300 nm, 200 nm and 100 nm.

4. The method of claim 3, wherein the Dx of the particle size distribution, as measured on a particle volume basis, is selected from the group consisting of less than or equal to 400 nm, 300nm, 200nm, and 100 nm; wherein x is greater than or equal to 90.

5. The method of any preceding claim, wherein the milling time period is a range selected from the group consisting of: between 10 minutes and 2 hours, between 10 minutes and 90 minutes, between 10 minutes and 1 hour, between 10 minutes and 45 minutes, between 10 minutes and 30 minutes, between 5 minutes and 30 minutes, between 5 minutes and 20 minutes, between 2 minutes and 10 minutes, between 2 minutes and 5 minutes, between 1 minute and 20 minutes, between 1 minute and 10 minutes, and between 1 minute and 5 minutes.

6. The method of any preceding claim, wherein the dry milling is undertaken in a mechanically agitated attritor mill (horizontal or vertical), vibratory mill or nutating mill.

7. The method of any preceding claim, wherein the total combined amount of biologically active material and grinding matrix in the mill at any given time is equal to or greater than a mass selected from the group consisting of: 200 grams, 500 grams, 1 kg, 2kg, 5 kg, 10 kg, 20 kg, 30 kg, 50 kg, 75 kg, 100kg, 150 kg and 200 kg.

8. The method of any preceding claim, wherein the grinding matrix is a single material or is a mixture of two or more materials in any proportion, wherein the single material or the mixture of two or more materials is selected from the group consisting of: mannitol, sorbitol, Isomalt, xylitol, maltitol, lactitol, erythritol, arabitol, ribitol, glucose, fructose, mannose, galactose, anhydrous lactose, lactose monohydrate, sucrose, maltose, trehalose, maltodextrins, dextrin, Inulin, dextrates, polydextrose, starch, wheat flour, corn flour, rice flour, rice starch, tapioca flour, tapioca starch, potato flour, potato starch, other flours and starches, milk powder, skim milk powders, other milk solids, soy flour, soy meal or other soy products, cellulose, microcrystalline cellulose, microcystalline cellulose based co blended materials, pregelatinized (or partially) starch, HPMC, CMC, HPC, citric acid, tartaric acid, malic acid, maleic acid fumaric acid , ascorbic acid, succinic acid, sodium citrate, sodium tartrate, sodium malate, sodium ascorbate, potassium citrate, potassium tartrate, potassium malate, potassium ascorbate, sodium carbonate, potassium carbonate, magnesium carbonate, sodium bicarbonate, potassium bicarbonate and calcium carbonate. dibasic calcium phosphate, tribasic calcium phosphate, sodium sulfate, sodium chloride, sodium metabisulphite, sodium thiosulfate, ammonium chloride, Glauber's salt, ammonium carbonate, sodium bisulfate, magnesium sulfate, potash alum, potassium chloride, sodium hydrogen sulfate, sodium hydroxide, crystalline hydroxides, hydrogen carbonates, methylamine hydrochloride, ammonium bromide, silica, thermal silica, alumina, titanium dioxide, talc, chalk, mica, kaolin, bentonite, hectorite, magnesium trisilicate, clay based materials or aluminium silicates, sodium lauryl sulfate, sodium stearyl sulfate, sodium cetyl sulfate, sodium cetostearyl sulfate, sodium docusate, sodium deoxycholate, N-lauroylsarcosine sodium salt, glyceryl monostearate , glycerol distearate glyceryl palmitostearate, glyceryl behenate, glyceryl caprylate, glyceryl oleate, benzalkonium chloride, CTAB, CTAC, Cetrimide, cetylpyridinium chloride, cetylpyridinium bromide, benzethonium chloride, PEG 40 stearate, PEG 100 stearate, poloxamer 188, , poloxamer 338, poloxamer 407 polyoxyl 2 stearyl ether, polyoxyl 100 stearyl ether, polyoxyl 20 stearyl ether, polyoxyl 10 stearyl ether, polyoxyl 20 cetyl ether, polysorbate 20, polysorbate 40, polysorbate 60, polysorbate 61, polysorbate 65, polysorbate 80, polyoxyl 35 castor oil, polyoxyl 40 castor oil, polyoxyl 60 castor oil, polyoxyl 100 castor oil, polyoxyl 200 castor oil, polyoxyl 40 hydrogenated castor oil, polyoxyl 60 hydrogenated castor oil, polyoxyl 100 hydrogenated castor oil, polyoxyl 200 hydrogenated castor oil, cetostearyl alcohol, macrogel 15 hydroxystearate, sorbitan monopalmitate, sorbitan monostearate, sorbitan trioleate, sucrose palmitate, sucrose stearate, sucrose distearate, sucrose laurate, glycocholic acid, sodium glycholate, cholic acid, sodium cholate, sodium deoxycholate, deoxycholic acid, sodium taurocholate, taurocholic acid, sodium taurodeoxycholate, taurodeoxycholic acid, soy lecithin, phosphatidylcholine, phosphatidylethanolamine, phosphatidylserine, phosphatidylinositol, PEG4000, PEG6000, PEG8000, PEG 10000, PEG20000, alkyl naphthalene sulfonate condensate/lignosulfonate blend, calcium dodecylbenzene sulfonate, sodium dodecylbenzene sulfonate, diisopropyl naphthaenesulphonate, erythritol distearate, naphthalene sulfonate formaldehyde condensate, nonylphenol ethoxylate (POE-30), tristyrylphenol ethoxylate, polyoxyethylene (15) tallowalkylamines, sodium alkyl naphthalene sulfonate, sodium alkyl naphthalene sulfonate condensate, sodium alkylbenzene sulfonate, sodium isopropyl naphthalene sulfonate, sodium methyl naphthalene formaldehyde sulfonate, sodium n-butyl naphthalene sulfonate, tridecyl alcohol ethoxylate (POE-18), triethanolamine isodecanol phosphate ester, triethanolamine tristyrylphosphate ester, tristyrylphenol ethoxylate sulfate and bis(2-hydroxyethyl)tallowalky lamines.

9. The method of any preceding claim, wherein a milling aid or combination of milling aids is used, where the milling aid is selected from the group consisting of: colloidal silica, a solid or semi solid surfactant, a liquid surfactant, a surfactant that can be manufactured into a solid or semisolid, a polymer, and a stearic acid, optionally wherein the surfactant is selected from the group consisting of: polyoxyethylene alkyl ethers, polyoxyethylene stearates, poloxamers, sarcosine based surfactants, polysorbates, alkyl sulfates and other sulfate surfactants, ethoxylated castor oil, polyvinylpyrrolidones, deoxycholate based surfactants, trimethyl ammonium based surfactants, lecithin and other phospholipids and bile salts.

10. The method of claim 9, wherein the surfactant is selected from the group consisting of: sodium lauryl sulfate, sodium docusate, sodium deoxycholate, N-lauroylsarcosine sodium salt, benzalkonium chloride, cetylpyridinium chloride, cetylpyridinium bromide, benzethonium chloride, PEG 40 stearate, PEG 100 stearate, poloxamer 188, polyoxyethylene (2) stearyl ether, polyethylene (100) stearyl ether, polyoxyethylene (20) stearyl ether, lecithin, poloxamer 407, polyethyleneglycol 3000, polyethyleneglycol 8000, polyvinylpyrrolidone, sodium dodecylbenzenesulphonic acid, sodium octadecyl sulphate, sodium pentane sulphonate, polysorbate 80, and polysorbate 61.

11. The method of claim 9 or claim 10, wherein the milling aid has a concentration selected from the group consisting of: 0.1 -10% w/w, 0.1 -5% w/w, 0.1 -2.5% w/w, of 0.1 - 2% w/w, 0.1 -1%, 0.5 -5% w/w, 0.5 -3% w/w, 0.5 - 2% w/w, 0.5 - 1.5%, 0.5 -1% w/w, of 0.75 - 1.25 % w/w, 0.75 -1% and 1% w/w.

12. The method of any preceding claim, wherein a facilitating agent is used or combination of facilitating agents is used, where the facilitating agent is selected from the group consisting of: surfactants, polymers, binding agents, filling agents, lubricating agents, sweeteners, flavouring agents, preservatives, buffers, wetting agents, disintegrants, effervescent agents, agents that may form part of a medicament, including a solid dosage form.

13. The method of claim 12, wherein the facilitating agent is added during dry milling at a time selected from the group consisting of: with 100% of the total milling time remaining, with 1-5 % of the total milling time remaining, with 1-10 % of the total milling time remaining, with 1-20 % of the total milling time remaining, with 1-30 % of the total milling time remaining, with 2-5% of the total milling time remaining, with 2-10% of the total milling time remaining, and with 5-20% of the total milling time remaining, optionally wherein the facilitating agent is selected from the group consisting of: crosslinked PVP (crospovidone), cross linked carmellose (croscarmellose), sodium starch glycolate, Povidone (PVP), Povidone K12, Povidone K17, Povidone K25, Povidone K29/32 and Povidone K30.

**Patentansprüche**

1. Verfahren zur Herstellung einer Zusammensetzung, umfassend die Schritte:

   Trockenmahlen eines festen biologisch aktiven Materials und einer mahlbaren Mahlmatrix in einer Mühle, die eine Vielzahl von Mahlkörpern umfasst, über einen Zeitraum, der ausreicht, um Partikel des biologisch aktiven Materials in einer wenigstens teilweise gemahlenen Mahlmatrix zu erhalten,
   wobei das biologisch aktive Material Metaxolon ist und
   wobei die Partikel eine mediane Partikelgröße, bestimmt auf Partikelvolumenbasis und durch Laserdiffraktion, von gleich oder kleiner als 500 nm aufweisen.

2. Verfahren gemäß Anspruch 1, wobei die durch das Verfahren hergestellte Zusammensetzung Partikel der biologisch aktiven Verbindung in oder über einem Volumenanteil von 25 Vol./Vol.-% umfasst.

3. Verfahren gemäß Anspruch 1 oder Anspruch 2, wobei die Partikel eine mediane Partikelgröße, bestimmt auf Partikelvolumenbasis, von gleich oder kleiner als eine Größe ausgewählt aus der Gruppe bestehend aus: 400 nm, 300 nm, 200 nm und 100 nm aufweisen.

4. Verfahren gemäß Anspruch 3, wobei der Dx-Wert der Partikelgrößenverteilung, wie gemessen auf Partikelvolumenbasis, ausgewählt ist aus der Gruppe bestehend aus kleiner als oder gleich 400 nm, 300 nm, 200 nm und 100 nm; wobei x größer als oder gleich 90 ist.

5. Verfahren gemäß einem der vorstehenden Ansprüche, wobei die Mahldauer in einem Bereich ausgewählt aus der Gruppe bestehend aus: zwischen 10 Minuten und 2 Stunden, zwischen 10 Minuten und 90 Minuten, zwischen 10 Minuten und 1 Stunde, zwischen 10 Minuten und 45 Minuten, zwischen 10 Minuten und 30 Minuten, zwischen 5 Minuten und 30 Minuten, zwischen 5 Minuten und 20 Minuten, zwischen 2 Minuten und 10 Minuten, zwischen 2 Minuten und 5 Minuten, zwischen 1 Minute und 20 Minuten, zwischen 1 Minuten und 10 Minuten, zwischen 1 Minute und 5 Minuten liegt.

6. Verfahren gemäß einem der vorstehenden Ansprüche, wobei das Trockenmahlen in einer mechanisch bewegten Attritormühle (horizontal oder vertikal), Schwingmühle oder Taumelmühle durchgeführt wird.

7. Verfahren gemäß einem der vorstehenden Ansprüche, wobei die kombinierte Gesamtmenge von biologisch aktivem Material und Mahlmatrix in der Mühle zu jedem gegebenen Zeitpunkt gleich einer oder größer als eine Masse ausgewählt aus der Gruppe bestehend aus: 200 Gramm, 500 Gramm, 1 kg, 2 kg, 5 kg, 10 kg, 20 kg, 30 kg, 50 kg, 75 kg, 100 kg, 150 kg und 200 kg ist.

8. Verfahren gemäß einem der vorstehenden Ansprüche, wobei die Mahlmatrix ein einziges Material ist oder ein Gemisch von zwei oder mehr Materialien in beliebigem Verhältnis ist, wobei das einzige Material oder das Gemisch von zwei oder mehr Materialien ausgewählt ist aus der Gruppe bestehend aus: Mannit, Sorbit, Isomalt, Xylit, Maltit, Lactit, Erythrit, Arabit, Ribit, Glucose, Fructose, Mannose, Galactose, wasserfreier Lactose, Lactosemonohydrat, Saccharose, Maltose, Trehalose, Maltodextrinen, Dextrin, Inulin, Dextraten, Polydextrose, Stärke, Weizenmehl, Maismehl, Reismehl, Reisstärke, Tapiokamehl, Tapiokastärke, Kartoffelmehl, Kartoffelstärke, anderen Mehlen und Stärken, Milchpulver, Magermilchpulvern, anderen Milchfeststoffen, Sojamehl, Sojagries und anderen Sojaprodukten, Cellulose, mikrokristalliner Cellulose, Materialien auf der Basis von oder gemischt mit mikrokristalliner Cellulose, (gegebenenfalls teilweise) vorgelierter Stärke, HPMC, CMC, HPC, Citronensäure, Weinsäure, Apfelsäure, Maleinsäure, Fumarsäure, Ascorbinsäure, Bernsteinsure, Natriumcitrat, Natriumtartrat, Natriummalat, Natriumascorbat, Kaliumcitrat, Kaliumtartrat, Kaliummalat, Kaliumascorbat, Natriumcarbonat, Kaliumcarbonat, Magnesiumcarbonat, Natriumbicarbonat, Kaliumbicarbonat und Calciumcarbonat, dibasischem Calciumphosphat, tribasischem Calciumphosphat, Natriumsulfat, Natriumchlorid, Natriummetabisulfit, Natriumthiosulfat, Ammoniumchlorid, Glaubersalz, Ammoniumcarbonat, Natriumbisulfat, Magnesiumsulfat, Kaliumalaun, Kaliumchlorid, Natriumhydrogensulfat, Natriumhydroxid, kristallinen Hydroxiden, Hydrogencarbonaten, Methylaminhydrochlorid, Ammoniumbromid, Siliciumdioxid, thermischem Siliciumdioxid, Aluminiumoxid, Titandioxid, Talkum, Kalk, Glimmer, Kaolin, Bentonit, Hektorit, Magnesiumtrisilicat, Materialien auf Tonbasis oder Aluminiumsilicaten, Natriumlaurylsulfat, Natriumstearylsulfat, Natriumcetylsulfat, Natriumcetostearylsulfat, Natriumdocusat, Natriumdesoxycholat, N-Lauroylsarcosin-Natriumsalz, Glycerylmonostearat, Glyceroldistearat, Glycerylpalmitostearat, Glycerylbehenat, Glycerylcaprylat, Glyceryloleat, Benzalkoniumchlorid, CTAB, CTAC, Cetrimid, Cetylpyridiniumchlorid, Cetylpyridiniumbromid, Benzethoniumchlorid, PEG-40-Stearat, PEG-100-Stearat, Poloxamer 188, Poloxamer 338, Poloxamer 407, Polyoxyl-2-stearylether, Polyoxyl-100-stearylether, Polyoxyl-20-stearylether, Polyoxyl-10-stearylether, Polyoxyl-20-cetylether, Polysorbat 20, Polysorbat 40, Polysorbat 60, Polysorbat 61, Polysorbat 65, Polysorbat 80, Polyoxyl-35-Rizinusöl, Polyoxyl-40-Rizinusöl, Polyoxyl-60-Rizinusöl, Polyoxyl-100-Rizinusöl, Polyoxyl-200-Rizinusöl, Polyoxyl-40-hydriertes-Rizinusöl, Polyoxyl-60-hydriertes-Rizinusöl, Polyoxyl-100-hydriertes-Rizinusöl, Polyoxyl-200-hydriertes-Rizinusöl, Cetostearylalkohol, Makrogel-15-hydroxystearat, Sorbitanmonopalmitat, Sorbitanmonostearat, Sorbitantrioleat, Saccharosepalmitat, Saccharosestearat, Saccharosedistearat, Saccharoselaurat, Glycocholsäure, Natriumglycholat, Cholsäure, Natriumcholat, Natriumdesoxycholat, Desoxycholsäure, Natriumtaurocholat, Taurocholsäure, Natriumtaurodesoxycholat, Taurodesoxycholsäure, Sojalecithin, Phosphatidylcholin, Phosphatidylethanolamin, Phosphatidylserin, Phosphatidylinositol, PEG 4000, PEG 6000, PEG 8000, PEG 10000, PEG 20000, Alkylnaphthalinsulfonat-Kondensat/Lignosulfonat-Gemisch, Calciumdodecylbenzolsulfonat, Natriumdodecylbenzolsulfonat, Diisopropylnaphthalinsulfonat, Erythritdistearat, Naphthalinsulfonat-Formaldehyd-Kondensat, Nonylphenolethoxylat (POE-30), Tristyrylphenolethoxylat, Polyoxyethylen(15)talgalkylaminen, Natriumalkylnaphthalinsulfonat, Natriumalkylnaphthalinsulfonat-Kondensat, Natriumalkylbenzolsulfonat, Natriumisopropylnaphthalinsulfonat, Natriummethylaphthalin-Formaldehyd-Sulfonat, Natrium-n-butylnaphthalinsulfonat, Tridecylalkoholethoxylat (POE-18), Triethanolaminisodecanolphosphatester, Triethanolamintristyrylphosphatester, Tristyrylphenolethoxylatsulfat und Bis(2-hydroxyethyl)talgalkylaminen.

9. Verfahren gemäß einem der vorstehenden Ansprüche, wobei ein Mahlhilfsmittel oder eine Kombination von Mahlhilfsmitteln verwendet wird, wobei das Mahlhilfsmittel ausgewählt ist aus der Gruppe bestehend aus: kolloidalem Siliciumdioxid, einem festen oder halbfesten grenzflächenaktiven Mittel, einem flüssigen grenzflächenaktiven Mittel, einem grenzflächenaktiven Mittel, das zu einem Feststoff oder halbfesten Stoff gemacht werden kann, einem Polymer und einer Stearinsäure, wobei das grenzflächenaktive Mittel gegebenenfalls ausgewählt ist aus der Gruppe bestehend aus: Polyoxyethylenalkylethern, Polyoxyethylenstearaten, Poloxameren, grenzflächenaktiven Mitteln auf Sarcosinbasis, Polysorbaten, Alkylsulfaten und anderen Sulfattensiden, ethoxyliertem Rizinusöl, Polyvinylpyrrolidonen, grenzflächenaktiven Mitteln auf Desoxycholatbasis, grenzflächenaktiven Mitteln auf Trimethylammoniumbasis, Lecithin und anderen Phospholipiden und Gallensalzen.

10. Verfahren gemäß Anspruch 9, wobei das grenzflächenaktive Mittel ausgewählt ist aus der Gruppe bestehend aus: Natriumlaurylsulfat, Natriumdocusat, Natriumdesoxycholat, N-Lauroylsarcosin-Natriumsalz, Benzalkoniumchlorid, Cetylpyridiniumchlorid, Cetylpyridiniumbromid, Benzethoniumchlorid, PEG-40-Stearat, PEG-100-Stearat, Poloxamer 188, Polyoxyethylen(2)stearylether, Polyethylen(100) stearylether, Polyoxyethylen(20)stearylether, Lecithin, Poloxamer 407, Polyethyleneglycol 3000, Polyethyleneglycol 8000, Polyvinylpyrrolidon, Natriumdodecylbenzolsulfonsäure, Natriumoctadecylsulfat, Natriumpentansulfonat, Polysorbat 80 und Polysorbat 61.

11. Verfahren gemäß Anspruch 9 oder Anspruch 10, wobei das Mahlhilfsmittel eine Konzentration ausgewählt aus der Gruppe bestehend aus: 0,1-10 Gew./Gew.-%, 0,1-5 Gew./Gew.-%, 0,1-2,5 Gew./Gew.-%, 0,1-2 Gew./Gew.-%, 0,1-1 Gew./Gew.-%, 0,5-5 Gew./Gew.-%, 0,5-3 Gew./Gew.-%, 0,5-2 Gew./Gew.-%, 0,5-1,5 Gew./Gew.-%, 0,5-1 Gew./Gew.-%, 0,75-1,25 Gew./Gew.-%, 0,75-1 Gew./Gew.-% und 1 Gew./Gew.-% aufweist.

12. Verfahren gemäß einem der vorstehenden Ansprüche, wobei ein Hilfsmittel verwendet wird oder eine Kombination von Hilfsmitteln verwendet wird, wobei das Hilfsmittel ausgewählt ist aus der Gruppe bestehend aus: grenzflächenaktiven Mitteln, Polymeren, Bindemitteln, Füllstoffen, Gleitmitteln, Süßstoffen, Aromastoffen, Konservierungsmitteln, Puffern, Netzmitteln, Sprengmitteln, Brausemitteln, Mitteln, die einen Teil eines Medikaments, einschließlich einer festen Darreichungsform, bilden können.

**13.** Verfahren gemäß Anspruch 12, wobei das Hilfsmittel während des Trockenmahlens über einen Zeitraum zugegeben wird, der ausgewählt ist aus der Gruppe bestehend aus: mit 100 % der verbleibenden Gesamtmahlzeit, mit 1-5 % der verbleibenden Gesamtmahlzeit, mit 1-10 % der verbleibenden Gesamtmahlzeit, mit 1-20 % der verbleibenden Gesamtmahlzeit, mit 1-30 % der verbleibenden Gesamtmahlzeit, mit 2-5 % der verbleibenden Gesamtmahlzeit, mit 2-10 % der verbleibenden Gesamtmahlzeit und mit 5-20 % der verbleibenden Gesamtmahlzeit, wobei das Hilfsmittel gegebenenfalls ausgewählt ist aus der Gruppe bestehend aus: vernetztem PVP (Crospovidon), vernetzter Carmellose (Croscarmellose), Natriumstärkeglycolat, Povidon (PVP), Povidon K12, Povidon K17, Povidon K25, Povidon K29/32 und Povidon K30.

## Revendications

**1.** Procédé pour la production d'une composition, comprenant les étapes de :
broyage à sec d'un matériau biologiquement actif solide et d'une matrice de meulage broyable dans un broyeur comprenant une pluralité de corps de broyage, pendant une période de temps suffisante pour produire des particules du matériau biologiquement actif dispersées dans une matrice de meulage au moins partiellement broyée,

le matériau biologiquement actif étant la métaxalone et
les particules possédant une grosseur moyenne de particule, déterminée sur une base de volume de particules et par diffraction laser, inférieure ou égale à 500 nm.

**2.** Procédé selon la revendication 1, la composition produite selon ledit procédé comprenant des particules du composé biologiquement actif selon une fraction en volume supérieure ou égale à 25% v/v.

**3.** Procédé selon la revendication 1 ou la revendication 2, les particules possédant une grosseur moyenne de particule, déterminée sur une base de volume de particules, inférieure ou égale à une grosseur choisie dans le groupe constitué par : 400 nm, 300 nm, 200 nm et 100 nm.

**4.** Procédé selon la revendication 3, le Dx de la distribution de grosseur de particule, tel que mesuré sur une base de volume de particules, étant choisi dans le groupe constitué par : inférieur ou égal à 400 nm, inférieur ou égal à 300 nm, inférieur ou égal à 200 nm et inférieur ou égal à 100 nm ; x étant supérieur ou égal à 90.

**5.** Procédé selon l'une quelconque revendication précédente, la période de temps de broyage étant une plage choisie dans le groupe constitué par : entre 10 minutes et 2 heures, entre 10 minutes et 90 minutes, entre 10 minutes et 1 heure, entre 10 minutes et 45 minutes, entre 10 minutes et 30 minutes, entre 5 minutes et 30 minutes, entre 5 minutes et 20 minutes, entre 2 minutes et 10 minutes, entre 2 minutes et 5 minutes, entre 1 minute et 20 minutes, entre 1 minute et 10 minutes et entre 1 minute et 5 minutes.

**6.** Procédé selon l'une quelconque revendication précédente, le broyage à sec étant réalisé dans un broyeur par frottement à agitation mécanique (horizontal ou vertical), dans un broyeur vibrant ou dans un broyeur à nutation.

**7.** Procédé selon l'une quelconque revendication précédente, la quantité totale combinée de matériau biologiquement actif et de matrice de meulage dans le broyeur étant à tout moment supérieure ou égale à une masse choisie dans le groupe constitué par : 200 grammes, 500 grammes, 1 kg, 2 kg, 5 kg, 10 kg, 20 kg, 30 kg, 50 kg, 75 kg, 100 kg, 150 kg et 200 kg.

**8.** Procédé selon l'une quelconque revendication précédente, la matrice de meulage étant un matériau unique ou étant un mélange de deux matériaux ou plus en une quelconque proportion, le matériau unique ou le mélange de deux matériaux ou plus étant choisi dans le groupe constitué par : le mannitol, le sorbitol, l'isomalt, le xylitol, le maltitol, le lactitol, l'érythritol, l'arabitol, le ribitol, le glucose, le fructose, le mannose, le galactose, le lactose anhydre, le monohydrate de lactose, le saccharose, le maltose, le tréhalose, les maltodextrines, la dextrine, l'inuline, les dextrates, le polydextrose, l'amidon, la farine de blé, la farine de maïs, la farine de riz, l'amidon de riz, la farine de tapioca, l'amidon de tapioca, la farine de pomme de terre, l'amidon de pomme de terre, d'autres farines et amidons, la poudre de lait, les poudres de lait écrémé, d'autres solides lactés, la farine de soja, le tourteau de soja ou d'autres produits de soja, la cellulose, la cellulose microcristalline, les matériaux co-mélangés à base de cellulose microcristalline, l'amidon prégélatinisé (ou partiellement gélatinisé), la HPMC (hydroxypropylméthylcellulose), la CMC (carboxyméthylcellulose), la HPC (hydroxypropylcellulose), l'acide citrique, l'acide tartrique, l'acide malique, l'acide maléique, l'acide fumarique, l'acide ascorbique, l'acide succinique, le citrate de sodium, le tartrate de sodium, le malate de sodium, l'ascorbate de sodium, le citrate de potassium, le tartrate de potassium, le malate de potassium, l'ascorbate de potassium, le carbonate de sodium, le carbonate de potassium, le carbonate de magnésium, le bicarbonate de sodium, le bicarbonate de potassium et le carbonate de calcium, le phosphate de calcium dibasique, le phosphate de calcium tribasique, le sulfate de sodium, le chlorure de sodium, le métabisulfite de sodium, le thiosulfate de sodium, le chlorure d'ammonium, le sel de Glauber, le carbonate d'ammonium, le bisulfate de sodium, le sulfate de magnésium, l'alun de potasse, le chlorure de potassium, l'hydrogénosulfate de sodium, l'hydroxyde de sodium, les hydroxydes cristallins, les hydrogénocarbonates, le chlorhydrate de méthylamine, le bromure d'ammonium, la silice, la silice thermique, l'alumine, le dioxyde de titane, le talc, la craie, le mica, le kaolin, la bentonite, l'hectorite, le trisilicate de magnésium, les matériaux à base d'argile ou les silicates d'aluminium, le laurylsulfate de sodium, le stéarylsulfate de sodium, le cétylsulfate de sodium, le cétostéarylsulfate de sodium, le docusate de sodium, le désoxycholate de sodium, le sel de sodium de la N-lauroylsarcosine, le monostéarate de glycéryle, le distéarate de glycérol, le palmitostéarate de glycéryle, le béhénate de glycéryle, le caprilate de glycéryle, l'oléate de glycéryle, le chlorure de benzalkonium, le CTAB (bromure de cétrimonium), le CTAC (chlorure de cétrimonium), le cétrimide, le chlorure de cétylpyridinium, le bromure de cétylpyridinium, le chlorure de benzéthonium, le stéarate de PEG 40, le stéarate de PEG 100, le poloxamère 188, le poloxamère 338, le poloxamère 407, l'éther stéarylique de polyoxyl-2, l'éther stéarylique de polyoxyl-100, l'éther stéarylique de polyoxyl-20, l'éther stéarylique de polyoxyl-10, l'éther cétylique de polyoxyl-20, le polysorbate 20, le polysorbate 40, le polysorbate 60, le polysorbate 61, le polysorbate 65, le polysorbate 80, l'huile de ricin de polyoxyl-35, l'huile de ricin de polyoxyl-40, l'huile de ricin de polyoxyl-60, l'huile de ricin de polyoxyl-100, l'huile de ricin de polyoxyl-200, l'huile de ricin hydrogénée de polyoxyl-40, l'huile de ricin hydrogénée de polyoxyl-60, l'huile de ricin hydrogénée de polyoxyl-100, l'huile de ricin hydrogénée de polyoxyl-200, l'alcool cétostéarylique, l'hydroxystéarate de macrogel 15, le monopalmitate de sorbitane, le monostéarate de sorbitane, le trioléate de sorbitane, le palmitate de saccharose, le stéarate de saccharose, le distéarate de saccharose, le laurate de saccharose, l'acide glycocholique, le glycolate de sodium, l'acide cholique, le cholate de sodium, le désoxycholate de sodium, l'acide désoxycholique, le taurocholate de sodium, l'acide taurocholique, le taurodésoxycholate de sodium, l'acide taurodésoxycholique, la lécithine de soja, la phosphatidylcholine, la phosphatidyléthanolamine, la phosphatidylsérine, le phosphatidylinositol, le PEG 4000, le PEG 6000, le PEG 8000, le PEG 10.000, le PEG 20.000, un mélange condensat d'alkylnaphtalènesulfonate/lignosulfonate, le dodécylbenzènesulfonate de calcium, le dodécylbenzènesulfonate de sodium, le diisopropylnaphtalènesulfonate, le distéarate d'érythritol, un condensat de naphtalènesulfonate et de formaldéhyde, l'éthoxylate de nonylphénol (POE-30), l'éthoxylate de tristyrylphénol, les alkylamines de suif de poly(oxyde d'éthylène) (15), l'alkylnaphtalènesulfonate de sodium, un condensat d'alkylnaphtalènesulfonate de sodium, l'alkylbenzènesulfonate de sodium, l'isopropylnaphtalènesulfonate de sodium, le sel de sodium de méthylnaphtalènesulfonate-formaldéhyde, le n-butylnaphtalènesulfonate de sodium, l'éthoxylate d'alcool tridécylique (POE-18), l'ester de phosphate d'isodécanol triéthanolamine, l'ester de tristyrylphosphate triéthanolamine, le sulfate d'éthoxylate de tristyrylphénol, les alkylamines de suif de bis(2-hydroxyéthyle).

9. Procédé selon l'une quelconque revendication précédente, un auxiliaire de broyage ou une combinaison d'auxiliaires de broyage étant utilisés, l'auxiliaire de broyage étant choisi dans le groupe constitué par : la silice colloïdale, un tensioactif solide ou semi-solide, un tensioactif liquide, un tensioactif pouvant être produit sous forme solide ou semi-solide, un polymère et un acide stéarique, éventuellement le tensioactif étant choisi dans le groupe constitué par : les éthers d'alkyle de poly(oxyde d'éthylène), les stéarates de poly(oxyde d'éthylène), les poloxamères, les tensioactifs à base de sarcosine, les polysorbates, les sulfates d'alkyle et d'autres tensioactifs de type sulfate, l'huile de ricin éthoxylée, les polyvinylpyrrolidones, les tensioactifs à base de désoxycholate, les tensioactifs à base de triméthylammonium, la lécithine et les autres phospholipides et les sels biliaires.

10. Procédé selon la revendication 9, le tensioactif étant choisi dans le groupe constitué par : le laurylsulfate de sodium, le docusate de sodium, le désoxycholate de sodium, le sel de sodium de la N-lauroylsarcosine, le chlorure de benzalkonium, le chlorure de cétylpyridinium, le bromure de cétylpyridinium, le chlorure de benzéthonium, le stéarate de PEG 40, le stéarate de PEG 100, le poloxamère 188, l'éther stéarylique de poly(oxyde d'éthylène) (2), l'éther stéarylique de polyéthylène (100), l'éther stéarylique de poly(oxyde d'éthylène) (20), la lécithine, le poloxamère 407, le polyéthylèneglycol 3000, le polyéthylèneglycol 8000, la polyvinylpyrrolidone, le dodécylbenzènesulfonate de sodium, l'octadécylsulfate de sodium, le pentanesulfonate de sodium, le polysorbate 80 et le polysorbate 61.

11. Procédé selon la revendication 9 ou la revendication 10, l'auxiliaire de broyage possédant une concentration choisie dans le groupe constitué par : 0,1-10% p/p, 0,1-5% p/p, 0,1-2,5% p/p, 0,1-2% p/p, 0,1-1%, 0,5-5% p/p, 0,5-3% p/p, 0,5-2% p/p, 0,5-1,5%, 0,5-1% p/p, 0,75-1,25 p/p, 0,75-1% et 1% p/p.

12. Procédé selon l'une des revendications précédentes, un agent facilitant étant utilisé ou une combinaison d'agents facilitants étant utilisée, l'agent facilitant étant choisi dans le groupe constitué par : les surfactants, les polymères, les agents liants, les agents de charge, les agents lubrifiants, les édulcorants, les agents aromatisants, les conservateurs, les tampons, les agents mouillants, les désintégrants, les agents effervescents, les agents pouvant faire partie d'un médicament, y compris une forme de dosage solide.

13. Procédé selon la revendication 12, l'agent facilitant étant ajouté pendant le broyage à sec à un temps choisi dans le groupe constitué par : à 100% du temps de broyage total restant, à 1-5% du temps de broyage total restant, à 1-10% du temps de broyage total restant, à 1-20% du temps de broyage total restant, à 1-30% du temps de broyage total restant, à 2-5% du temps de broyage total restant, à 2-10% du temps de broyage total restant, et à 5-20% du temps de broyage total restant, éventuellement l'agent facilitant étant choisi dans le groupe constitué par : la PVP réticulée (crospovidone), la carmellose réticulée (croscarmellose), le glycolate d'amidon sodique, la povidone (PVP), la povidone K12, la povidone K17, la povidone K25, la povidone K29/32 et la povidone K30.

| Sample No. | Active material | | | | Primary Matrix | | | Surfactant #1 | | | Surfactant #2 | | | Time (mins.) | Particle Size | | | | | | Yield (%) | Variations |
|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | | D(0.5) μm | % <0.20 μm | % <0.30 μm | % < 0.5 μm | % < 1.0 μm | % < 2.0 μm | | |
| A | IND | 1.20 | 12 | | LAC | 8.80 | 88 | | | | | | | 30 | 0.223 | 45 | 61 | 71 | 77 | 89 | | |
| B | IND | 1.20 | 12 | | LAC | 8.70 | 87 | SPS | 0.1 | 1 | | | | 30 | 0.215 | 47 | 64 | 84 | 83 | 93 | | |
| C | IND | 1.20 | 12 | | LAC | 8.70 | 87 | SDS | 0.1 | 1 | | | | 30 | 0.189 | 53 | 73 | 88 | 95 | 99 | | |
| D | IND | 1.20 | 12 | | LAC | 8.70 | 87 | SOS | 0.1 | 1 | | | | 30 | 0.203 | 49 | 69 | 84 | 92 | 97 | | |
| E | IND | 1.20 | 12 | | LAC | 8.70 | 87 | B700 | 0.1 | 1 | | | | 30 | 0.167 | 60 | 80 | 93 | 97 | 99 | | |
| F | IND | 1.20 | 12 | | LAC | 8.70 | 87 | B76 | 0.1 | 1 | | | | 30 | 0.192 | 52 | 72 | 89 | 96 | 99 | | |
| G | IND | 1.20 | 12 | | LAC | 8.70 | 87 | SDC | 0.1 | 1 | | | | 30 | 0.191 | 52 | 67 | 77 | 83 | 93 | | |
| H | IND | 1.20 | 12 | | LAC | 8.70 | 87 | SNS | 0.1 | 1 | | | | 30 | 0.225 | 44 | 63 | 79 | 88 | 96 | | |
| I | IND | 1.20 | 12 | | LAC | 8.70 | 87 | LEC | 0.1 | 1 | | | | 30 | 0.230 | 44 | 61 | 75 | 85 | 95 | | |
| J | IND | 0.5 | 10 | | LAC | 4.50 | 90 | | | | | | | 20 | 0.237 | 44 | 57 | 65 | 73 | 85 | | |
| K | IND | 0.5 | 10 | | LAC | 4.45 | 89 | P40S | 0.05 | 1 | | | | 20 | 0.169 | 58 | 72 | 80 | 89 | 97 | | |
| L | IND | 0.5 | 10 | | LAC | 4.45 | 89 | DS | 0.05 | 1 | | | | 20 | 0.249 | 42 | 56 | 68 | 84 | 98 | | |
| M | IND | 0.5 | 10 | | LAC | 4.45 | 89 | AS | 0.05 | 1 | | | | 20 | 0.190 | 52 | 67 | 76 | 84 | 92 | | |
| N | IND | 1.0 | 20 | | LAC | 3.95 | 79 | SDS | 0.05 | 1 | | | | 30 | 0.435 | 24 | 38 | 53 | 67 | 83 | | |
| O | IND | 1.0 | 20 | | | | | SDS | 4.00 | 80 | | | | 30 | 2.612 | 0 | 0 | 0 | 6 | 34 | | |
| P | IND | 4.95 | 99 | | | | | SDS | 0.05 | 1 | | | | 30 | 1094 | 0 | 0 | 0 | 0 | 2 | | |
| Q | IND | 1.0 | 20 | | LAC | 4.00 | 80 | | | | | | | 30 | 5.128 | 0 | 0 | 0 | 0 | 8 | | |
| R | DIC | 1.0 | 20 | | LAC | 3.95 | 79 | SDS | 0.05 | 1 | | | | 30 | 0.153 | 66 | 84 | 95 | 98 | 99 | | |
| S | DIC | 1.0 | 20 | | | | | SDS | 4.00 | 80 | | | | 30 | 3.173 | 0 | 0 | 0 | 3 | 24 | | |

**Figure 1A**

| Sample No. | Active material | | | | Primary Matrix | | | Surfactant #1 | | | Surfactant #2 | | | Time (mins.) | Particle Size | | | | | | Yield (%) | Variations |
|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | | D(0.5) μm | % <0.20 μm | % <0.30 μm | % < 0.5 μm | % < 1.0 μm | % < 2.0 μm | | |
| T | DIC | 4.95 | 99 | | | | | SDS | 0.05 | 1 | | | | 30 | 117 | 0 | 0 | 0 | 1 | 4 | | |
| U | DIC | 1.00 | 20 | | LAC | 4.00 | 80 | | | | | | | 30 | 0.178 | 56 | 74 | 86 | 92 | 97 | | |
| V | DIC | 2.00 | 20 | | MAN | 8.00 | 80 | | | | | | | 30 | 0.2 | 50 | 69 | 84 | 91 | 97 | | |
| W | DIC | 2.00 | 20 | | MAN | 7.90 | 79 | SDS | 0.1 | 1 | | | | 30 | 0.201 | 50 | 69 | 83 | 91 | 97 | | |
| X | DIC | 2.00 | 20 | | MAN | 7.90 | 79 | SOS | 0.1 | 1 | | | | 30 | 0.195 | 51 | 71 | 85 | 92 | 97 | | |
| Y | NAA | 1.75 | 35 | | LAC | 3.2 | 65 | | | | | | | 20 | 2.9 | 18 | 23 | 25 | 26 | 38 | | |
| Z | NAA | 1.75 | 35 | | LAC | 3.25 | 64 | P40S | 0.05 | 1 | | | | 20 | 0.373 | 33 | 45 | 56 | 70 | 87 | | |
| AA | NAA | 1.75 | 35 | | LAC | 3.25 | 64 | SDS | 0.05 | 1 | | | | 20 | 0.293 | 38 | 50 | 60 | 65 | 75 | | |
| AB | NAA | 4.0 | 40 | | LAC | 5.9 | 59 | P40S | 0.1 | 1 | | | | 120 | 0.285 | 37 | 52 | 66 | 75 | 82 | | |
| AC | NAA | 4.0 | 40 | | LAC | 6.0 | 60 | | | | | | | 120 | 6.1 | 0 | 0 | 0 | 0 | 8 | | |
| AD | NAA | 1.40 | 35 | | MAN | 2.60 | 65 | | | | | | | 20 | 0.171 | 58 | 73 | 82 | 86 | 88 | | |
| AE | NAA | 1.40 | 35 | | MAN | 2.52 | 63 | SDS | 0.08 | 2 | | | | 20 | 0.131 | 76 | 90 | 95 | 96 | 98 | | |
| AF | NAA | 1.2 | 30 | | MAN | 2.8 | 70 | | | | | | | 20 | 0.208 | 48 | 64 | 75 | 79 | 84 | | |
| AG | NAA | 1.2 | 30 | | MAN | 2.76 | 69.0 | SDS | 0 | 1.0 | | | | 20 | 0.173 | 58 | 75 | 86 | 91 | 96 | | |
| AH | NAA | 1.2 | 30.0 | | LAC | 2.8 | 70.0 | | | | | | | 20 | 0.396 | 33 | 44 | 53 | 58 | 70 | | |
| AI | NAA | 1.2 | 30.0 | | TCD | 2.8 | 70.0 | | | | | | | 20 | 3.1 | 18 | 24 | 27 | 27 | 37 | | |
| AJ | NAA | 1.2 | 30.0 | | CAC | 2.8 | 70.0 | | | | | | | 20 | 28 | 3 | 4 | 5 | 6 | 10 | | |
| AK | NAA | 1 | 25.0 | | LAA | 3 | 75.0 | | | | | | | 20 | 1.07 | 31 | 41 | 46 | 49 | 67 | | |
| AL | NAA | 1 | 25.0 | | XYL | 3 | 75.0 | | | | | | | 20 | 0.18 | 57 | 75 | 87 | 92 | 95 | | |

Figure 1B

| Sample No. | Active material | | | | Primary Matrix | | | Surfactant #1 | | | Surfactant #2 | | | Time (mins.) | Particle Size | | | | | | Yield (%) | Variations |
|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | | D(0.5) µm | % <0.20 µm | % <0.30 µm | % < 0.5 µm | % < 1.0 µm | % < 2.0 µm | | |
| AM | NAA | 1 | 25.0 | | MAA | 3 | 75.0 | | | | | | | 20 | 0.153 | 66 | 85 | 96 | 98 | 99 | | |
| AN | NAA | 1 | 25.0 | | TCD | 3 | 75.0 | | | | | | | 20 | 0.331 | 35 | 48 | 57 | 62 | 72 | | |
| AO | HAL | 1 | 10.0 | | LAC | 9 | 90.0 | | | | | | | 40 | 2.123 | 0 | 0 | 0 | 0 | 5 | | |
| AP | HAL | 1 | 10.0 | | LAC | 8.9 | 89.0 | LEC | 0.1 | 1.0 | | | | 40 | 0.135 | 74 | 90 | 97 | 98 | 99 | | |
| AQ | MET | 1 | 10.0 | | LAC | 9 | 90.0 | | | | | | | 40 | 4.727 | 0 | 0 | 0 | 0 | 4 | | |
| AR | MET | 1 | 10.0 | | LAC | 8.9 | 89.0 | SDS | 0.1 | 1.0 | | | | 40 | 0.129 | 80 | 93 | 96 | 97 | 98 | | |
| AS | TRI | 1 | 10.0 | | LAC | 9 | 90.0 | | | | | | | 40 | 2.622 | 0 | 0 | 0 | 0 | 25 | | |
| AT | TRI | 1 | 10.0 | | LAC | 8.9 | 89.0 | B700 | 0.1 | 1.0 | | | | 40 | 0.128 | 82 | 96 | 98 | 98 | 99 | | |
| AU | SUL | 1 | 10.0 | | LAC | 9 | 90.0 | | | | | | | 40 | 0.388 | 27 | 42 | 56 | 69 | 86 | | |
| AV | SUL | 1 | 10.0 | | LAC | 8.9 | 89.0 | SDS | 0.1 | 1.0 | | | | 40 | 0.455 | 6 | 26 | 55 | 78 | 96 | | |
| AW | MAN | 1 | 10.0 | | LAC | 9 | 90.0 | | | | | | | 40 | 0.198 | 50 | 71 | 88 | 97 | 97 | | |
| AX | MAN | 1 | 10.0 | | LAC | 8.9 | 89.0 | B700 | 0.1 | 1.0 | | | | 40 | 0.17 | 60 | 82 | 96 | 100 | 100 | | |
| AY | MAN | 1 | 10.0 | | LAC | 8.9 | 89.0 | SDS | 0.1 | 1.0 | | | | 40 | 0.171 | 60 | 82 | 97 | 100 | 100 | | |
| AZ | MAN | 1 | 10.0 | | LAC | 8.9 | 89.0 | LEC | 0.1 | 1.0 | | | | 40 | 0.181 | 56 | 78 | 95 | 100 | 100 | | |
| BA | MAN | 2 | 20.0 | | LAC | 7.9 | 79.0 | SDS | 0.1 | 1.0 | | | | 40 | 0.212 | 47 | 68 | 86 | 96 | 98 | | |
| BB | MAN | 3 | 30.0 | | LAC | 6.9 | 69.0 | SDS | 0.1 | 1.0 | | | | 40 | 0.258 | 36 | 58 | 81 | 94 | 97 | | |
| BC | MTX | 1.5 | 30.0 | | LAC | 3.5 | 69.0 | P407 | 0.1 | 1.0 | | | | 60 | 0.16 | 63 | 77 | 84 | 89 | 93 | | 2 |
| BD | MTX | 1.5 | 30.0 | | LAC | 3.5 | 70.0 | | | | | | | 60 | 0.28 | 40 | 52 | 59 | 59 | 71 | | 2 |
| BE | MTX | 2.5 | 50.0 | | LAC | 2.35 | 47.0 | SDS | 0.8 | 2.0 | P407 | 0.1 | 2 | 60 | 0.148 | 67 | 83 | 92 | 98 | 99 | | 2 |

Figure 1C

| Sample No. | Active material | | | | Primary Matrix | | | Surfactant #1 | | | Surfactant #2 | | | Time (mins.) | Particle Size | | | | | | Yield (%) | Variations |
|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | | D(0.5) μm | % <0.20 μm | % <0.30 μm | % < 0.5 μm | % < 1.0 μm | % < 2.0 μm | | |
| BF | NAA | 1 | 25 | 30 | MAN | 3 | 75 | | | | | | | 20 | 0.181 | 55 | 74 | 87 | 94 | 97 | | |
| BG | NAA | 1 | 25 | 30 | XYL | 3 | 75 | | | | | | | 20 | 0.177 | 56 | 74 | 85 | 91 | 95 | | |
| BH | NAS | 1.25 | 25 | 30 | TA | 3.75 | 75 | | | | | | | 20 | 0.311 | 37 | 49 | 59 | 64 | 75 | | |
| BI | NAS | 1.25 | 25 | 30 | TA | 3.75 | 74 | P40S | 0.1 | 1 | | | | 30 | 0.303 | 36 | 50 | 62 | 77 | 89 | | |
| BJ | DIC | 3 | 30 | 31 | LAC | 6.9 | 69 | SDS | 0.1 | 1 | | | | 90 | 0.202 | 49 | 69 | 83 | 88 | 92 | | |
| BK | 2,4D | 1 | 20 | | LAC | 4 | 80 | | | | | | | 30 | 1.205 | 17 | 23 | 29 | 43 | 72 | 93 | 1 |
| BL | 2,4D | 1 | 20 | | LAC | 3.95 | 79 | SDA | 0.05 | 1 | | | | 30 | 0.473 | 20 | 33 | 52 | 75 | 82 | 91 | 1 |
| BM | 2,4D | 1 | 20 | | LAC | 3.95 | 79 | T3785 | 0.05 | 1 | | | | 30 | 0.414 | 24 | 38 | 57 | 78 | 94 | 93 | 1 |
| BN | 2,4D | 1 | 20 | | LAC | 3.95 | 79 | D920 | 0.05 | 1 | | | | 30 | 0.402 | 26 | 40 | 57 | 78 | 91 | 93 | 1 |
| BO | 2,4D | 1 | 20 | | LAC | 3.95 | 79 | SDS | 0.05 | 1 | | | | 30 | 0.276 | 36 | 54 | 74 | 92 | 96 | | 1 |
| BP | 2,4D | 1 | 20 | | LAC | 3.95 | 79 | B700 | 0.05 | 1 | | | | 30 | 0.269 | 38 | 54 | 69 | 86 | 95 | 92 | 1 |
| BQ | 2,4D | 1 | 20 | | LAC | 3.95 | 79 | K1251 | 0.05 | 1 | | | | 30 | 0.252 | 41 | 56 | 71 | 89 | 96 | 91 | 1 |
| BR | 2,4D | 1 | 20 | | LAC | 3.95 | 79 | T305 | 0.05 | 1 | | | | 30 | 0.231 | 44 | 59 | 73 | 87 | 96 | 94 | 1 |
| BS | GLY | 1 | 20 | | LAC | 3.95 | 79 | T2700 | 0.05 | 1 | | | | 30 | 0.976 | 25 | 35 | 43 | 50 | 64 | 59 | 4 |
| BT | GLY | 1 | 20 | | LAC | 3.95 | 79 | B700 | 0.05 | 1 | | | | 30 | 1.449 | 21 | 27 | 33 | 42 | 57 | 84 | 4 |
| BU | GLY | 1 | 20 | | LAC | 3.9 | 78 | B700 | 0.05 | 1 | T2700 | 0.05 | 1 | 30 | 0.311 | 37 | 49 | 58 | 66 | 79 | 81 | 4 |
| BV | GLY | 1 | 20 | | LAC | 3.9 | 78 | B700 | 0.05 | 1 | K1251 | 0.05 | 1 | 30 | 1.085 | 26 | 34 | 41 | 49 | 66 | 82 | 4 |
| BW | GLY | 1 | 20 | | LAC | 3.9 | 78 | B700 | 0.05 | 1 | P188 | 0.05 | 1 | 30 | 1.48 | 8 | 11 | 16 | 33 | 62 | 86 | 4 |
| BX | GLY | 1 | 20 | | LAC | 3.9 | 78 | B700 | 0.05 | 1 | T2700 | 0.05 | 1 | 60 | 0.176 | 57 | 74 | 86 | 94 | 96 | 79 | 4 |

Figure 1D

## Figure 1E

| Sample No. | Active material Name | Active material Mass (g) | Active material % w/w | Active material % v/v | Primary Matrix Name | Primary Matrix Mass (g) | Primary Matrix % w/w | Surfactant #1 Name | Surfactant #1 Mass (g) | Surfactant #1 % w/w | Surfactant #2 Name | Surfactant #2 Mass (g) | Surfactant #2 % w/w | Time (mins.) | D(0.5) μm | % <0.20 μm | % <0.30 μm | % <0.5 μm | % <1.0 μm | % <2.0 μm | Yield (%) | Variations |
|---|---|---|---|---|---|---|---|---|---|---|---|---|---|---|---|---|---|---|---|---|---|---|
| BY | GLY | 1 | 20 | | LAC | 3.9 | 78 | B700 | 0.05 | 1 | K1251 | 0.05 | 1 | 60 | 0.658 | 0 | 0 | 21 | 93 | 100 | 81 | 4 |
| BZ | GLY | 1 | 20 | | LAC | 3.9 | 78 | B700 | 0.05 | 1 | T2700 | 0.05 | 1 | 60 | 0.159 | 63 | 78 | 88 | 94 | 95 | 79 | 4 |
| CA | GLY | 1 | 20 | | LAC | 3.9 | 78 | B700 | 0.05 | 1 | K1251 | 0.05 | 1 | 60 | 0.297 | 34 | 50 | 70 | 95 | 100 | 81 | 4 |
| CB | MEL | 0.5 | 10 | | LAC | 4.4 | 88 | CEL | 0.1 | 2 | | 0 | 0 | 25 | 1.128 | 31 | 39 | 42 | 48 | 68 | 68 | 1 |
| CC | MEL | 0.5 | 10 | | LAC | 4.3 | 87 | P188 | 0.2 | 3 | BC | 0 | 1 | 25 | 0.27 | 38 | 53 | 59 | 62 | 81 | 73 | 1 |
| CD | MEL | 0.5 | 10 | | LAC | 4.3 | 87 | P188 | 0.2 | 3 | CEL | 0 | 1 | 25 | 0.278 | 40 | 52 | 58 | 62 | 76 | 74 | 1 |
| CE | MEL | 0.5 | 10 | | LAC | 4.3 | 87 | P188 | 0.2 | 3 | DS | 0 | 1 | 25 | 0.12 | 82 | 96 | 100 | 100 | 100 | 88 | 1 |
| CF | MEL | 0.5 | 10 | | LAC | 4.4 | 89 | P188 | 0.1 | 1 | K25 | 0 | 1 | 25 | 0.249 | 42 | 55 | 59 | 61 | 74 | 69 | 1 |
| CG | MEL | 0.25 | 5 | | MAN | 4.6 | 92 | P188 | 0.2 | 3 | LEC | 0.02 | 0.5 | 25 | 0.123 | 81 | 96 | 100 | 100 | 100 | 58 | 1 |
| CH | MEL | 0.5 | 10 | | LAC | 4.3 | 87 | P188 | 0.2 | 3 | LEC | 0.02 | 0.5 | 25 | 0.144 | 71 | 88 | 94 | 94 | 97 | 68 | 1 |
| CI | MEL | 0.5 | 10 | | LAC | 4.3 | 87 | P188 | 0.2 | 3 | SDC | 0.02 | 0.5 | 25 | 0.184 | 54 | 70 | 79 | 81 | 87 | 63 | 1 |
| CJ | MEL | 0.5 | 10 | | LAC | 4.3 | 87 | P188 | 0.2 | 3 | T80 | 0 | 1 | 25 | 0.224 | 45 | 61 | 70 | 75 | 87 | 68 | 1 |
| CK | MEL | 0.25 | 5 | | LAC | 4.7 | 94 | P188 | 0.1 | 1 | | | | 25 | 0.158 | 63 | 81 | 90 | 90 | 93 | 48 | 1 |
| CL | MEL | 0.5 | 10 | | LAC | 4.4 | 87 | P188 | 0.2 | 3 | | | | 25 | 0.169 | 59 | 76 | 85 | 87 | 93 | 58 | 1 |
| CM | MEL | 0.25 | 5 | | LAC | 4.6 | 92 | P188 | 0.2 | 3 | | | | 25 | 0.221 | 46 | 60 | 68 | 69 | 75 | 68 | 1 |
| CN | MEL | 0.5 | 10 | | LAC | 4.3 | 85 | P188 | 0.3 | 5 | | | | 25 | 0.309 | 39 | 49 | 55 | 56 | 66 | 74 | 1 |
| CO | MEL | 0.5 | 9.5 | | MAN | 4.6 | 88 | P188 | 0.2 | 3 | | | | 25 | 0.251 | 43 | 55 | 61 | 62 | 68 | 55 | 1 |
| CP | MEL | 0.5 | 10 | | MAN | 4.5 | 89 | P3000 | 0.1 | 1 | | | | 25 | 1.343 | 29 | 36 | 39 | 43 | 63 | 61 | 1 |
| CQ | MEL | 0.5 | 10 | | MAN | 4.5 | 89 | SDC | 0.1 | 1 | | | | 25 | 1.699 | 25 | 31 | 32 | 37 | 56 | 77 | 1 |

64

| Sample No. | Active material | | | | Primary Matrix | | | Surfactant #1 | | | Surfactant #2 | | | Time (mins.) | Particle Size | | | | | | Yield (%) | Variations |
|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | | D(0.5) μm | % <0.20 μm | % <0.30 μm | % < 0.5 μm | % < 1.0 μm | % < 2.0 μm | | |
| CR | MEL | 0.5 | 10 | | LAC | 4.4 | 88 | T80 | 0.1 | 2 | | | | 25 | 1.279 | 28 | 35 | 38 | 44 | 65 | 68 | 1 |
| CS | MAN | 2.5 | 50 | 45 | LAC | 2.35 | 47 | SDS | 0.15 | 3 | | | | 15 | 0.318 | 31 | 48 | 65 | 80 | 84 | | 5 |
| CT | MAN | 2.5 | 50 | 45 | LAC | 2.45 | 49 | P188 | 0.05 | 1 | | | | 15 | 0.33 | 30 | 46 | 64 | 77 | 82 | | 5 |
| CU | MAN | 2.5 | 50 | 45 | LAC | 2.45 | 49 | P40S | 0.05 | 1 | | | | 15 | 0.333 | 30 | 46 | 63 | 75 | 80 | | 5 |
| CV | MAN | 2.5 | 50 | 45 | LAC | 2.45 | 49 | B700 | 0.05 | 1 | | | | 15 | 0.337 | 29 | 46 | 63 | 76 | 81 | | 5 |
| CW | MAN | 2.5 | 50 | 45 | LAC | 2.45 | 49 | P407 | 0.05 | 1 | | | | 15 | 0.342 | 28 | 45 | 63 | 76 | 82 | | 5 |
| CX | MAN | 2.5 | 50 | 45 | LAC | 2.45 | 49 | T1221 | 0.05 | 1 | | | | 15 | 0.411 | 24 | 40 | 56 | 69 | 75 | | 5 |
| CY | MAN | 2.5 | 50 | 45 | LAC | 2.45 | 49 | DS | 0.05 | 1 | | | | 15 | 0.462 | 22 | 37 | 52 | 65 | 71 | | 5 |
| CZ | MAN | 2.5 | 50 | 45 | LAC | 2.45 | 49 | SDS | 0.05 | 1 | | | | 15 | 1.369 | 1 | 6 | 20 | 43 | 56 | | 5 |
| DA | MAN | 2.5 | 50 | 45 | LAC | 2.45 | 49 | SDA | 0.05 | 1 | | | | 15 | 1.766 | 0 | 2 | 14 | 38 | 52 | | 5 |
| DB | MAN | 2.5 | 50 | 45 | LAC | 2.45 | 49 | CEL | 0.05 | 1 | | | | 15 | 1.86 | 0 | 2 | 14 | 37 | 51 | | 5 |
| DC | MAN | 2.5 | 50 | 45 | LAC | 2.5 | 50 | | | | | | | 15 | 2.578 | 0 | 1 | 11 | 31 | 45 | | 5 |
| DD | CEL | 0.5 | 10 | | LAC | 4.3 | 86 | SDS | 0.1 | 2 | P40S | 0.1 | 2 | 15 | 0.134 | 76 | 88 | 91 | 91 | 93 | 88 | 1 |
| DE | CEL | 0.5 | 10 | | LAC | 4.3 | 86 | SDS | 0.1 | 2 | P407 | 0.1 | 2 | 15 | 0.14 | 75 | 83 | 83 | 83 | 86 | 90 | 1 |
| DF | CEL | 0.5 | 10 | | LAC | 4.3 | 86 | SDS | 0.1 | 1 | LEC | 0.15 | 3 | 15 | 0.181 | 55 | 70 | 79 | 83 | 89 | 90 | 1 |
| DG | CEL | 0.5 | 10 | | LAC | 4.3 | 86 | SDS | 0.1 | 1 | B700 | 0.15 | 3 | 15 | 1.903 | 28 | 37 | 44 | 46 | 51 | 90 | 1 |
| DH | CEL | 0.5 | 10 | | LAC | 4.5 | 90 | | | | | | | 15 | 5.296 | 8 | 11 | 13 | 13 | 16 | 85 | 1 |
| DI | CEL | 0.5 | 10 | | LAC | 4.3 | 86 | SDS | 0.1 | 1 | P3000 | 0.15 | 3 | 15 | 0.397 | 33 | 45 | 53 | 59 | 71 | 88 | 1 |
| DJ | CEL | 0.5 | 10 | | LAC | 4.4 | 88 | SDS | 0.1 | 1 | P8000 | 0.05 | 1 | 15 | 0.234 | 44 | 58 | 69 | 77 | 87 | 87 | 1 |

Figure 1F

| Sample No. | Active material | | | | Primary Matrix | | | Surfactant #1 | | | Surfactant #2 | | | Time (mins.) | Particle Size | | | | | | Yield (%) | Variations |
|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | | D(0.5) µm | % <0.20 µm | % <0.30 µm | % < 0.5 µm | % < 1.0 µm | % < 2.0 µm | | |
| DK | CEL | 0.5 | 10 | | LAC | 4.3 | 86 | DS | 0.1 | 2 | P40S | 0.1 | 2 | 15 | 0.319 | 35 | 48 | 61 | 69 | 74 | 88 | 1 |
| DL | CEL | 0.5 | 10 | | SOR | 4.5 | 90 | | | | | | | 15 | 16.031 | 0 | 0 | 0 | 0 | 0.8 | 46 | 1 |
| DM | CEL | 0.5 | 10 | | SOR | 4.45 | 89 | SDS | 0.1 | 1 | | | | 15 | 0.173 | 57 | 72 | 79 | 80 | 86 | 52 | 1 |
| DN | CYA | 0.5 | 10 | | LAC | 4.45 | 89 | SDS | 0.1 | 1 | | | | 20 | 0.159 | 65 | 84 | 95 | 100 | 100 | 79 | 5 |
| DO | CYA | 0.5 | 10 | | MAN | 4.45 | 89 | SDS | 0.1 | 1 | | | | 20 | 0.194 | 52 | 68 | 79 | 84 | 84 | 87 | 5 |
| DP | PRO | 0.5 | 10 | | LAC | 4.45 | 89 | SDS | 0.1 | 1 | | | | 20 | 0.229 | 43 | 63 | 83 | 97 | 98 | 87 | 5 |
| DQ | PRO | 0.5 | 10 | | MAN | 4.45 | 89 | SDS | 0.1 | 1 | | | | 20 | 0.553 | 15 | 27 | 45 | 77 | 94 | 89 | 5 |
| DR | PRO | 0.5 | 10 | | LAC | 4.45 | 89 | C40 | 0.1 | 1 | | | | 20 | 0.546 | 10 | 23 | 45 | 76 | 89 | 72 | 5 |
| DS | SAL | 0.51 | 10 | | LAC | 4.5 | 89.5 | LEC | 0.05 | 1 | | | | 20 | 0.128 | 84 | 98 | 100 | 100 | 100 | | |
| DT | SAL | 0.51 | 10 | | LAC | 4.5 | 89.5 | LEC | 0.05 | 1 | | | | 20 | 0.42 | 31 | 42 | 53 | 57 | 57 | | |
| DU | CIP | 0.76 | 15 | | MAL | 4.1 | 83 | T80 | 0.05 | 1 | DS | | | 20 | 0.22 | 40 | 74 | 85 | 85 | 92 | 96 | 6 |
| DV | CIP | 0.76 | 15 | | LAC | 4.2 | 85 | | | | | | | 20 | 25.909 | 1 | 2 | 3.1 | 4.8 | 7 | 89 | 6 |
| DW | CIP | 0.76 | 15 | | MAL | 4.3 | 85 | | | | | | | 20 | 0.238 | 43 | 56 | 58 | 58 | 61 | 93 | 6 |
| DX | CIP | 0.75 | 15 | | LAA | 4.3 | 85 | | | | | | | 20 | 0.205 | 49 | 62 | 65 | 65 | 71 | 97 | 6 |
| DY | CIP | 0.75 | 15 | | LAA | 4.2 | 84 | T80 | 0.06 | 1 | | | | 20 | 0.14 | 75 | 91 | 94 | 94 | 96 | 96 | 6 |
| DZ | CIP | 0.75 | 15 | | LAA | 4.2 | 84 | SOL | 0.05 | 1 | | | | 20 | 0.237 | 35 | 66 | 78 | 78 | 84 | 97 | 6 |
| EA | CIP | 0.75 | 15 | | LAA | 4.2 | 84 | CEL | 0.06 | 1 | | | | 20 | 0.23 | 37 | 69 | 81 | 81 | 87 | 87 | 6 |
| EB | CIP | 0.75 | 15 | | LAA | 4.2 | 84 | DS | 0.05 | 1 | | | | 20 | 0.216 | 42 | 74 | 83 | 83 | 91 | 96 | 6 |
| EC | CIP | 0.75 | 15 | | LAA | 4.2 | 84 | P8000 | 0.05 | 1 | | | | 20 | 0.243 | 33 | 64 | 75 | 75 | 82 | 97 | 6 |

**Figure 1G**

**Figure 1H**

| Sample No. | Active material | | | | Primary Matrix | | | Surfactant #1 | | | Time (mins.) | Particle Size | | | | | | Yield (%) | Variations |
|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | | D(0.5) µm | % <0.20 µm | % <0.30 µm | % < 0.5 µm | % < 1.0 µm | % < 2.0 µm | | |
| A | IND | 1.20 | 12 | | LAC | 8.80 | 88 | | | | 30 | 0.753 | 25 | 34 | 44 | 55 | 70 | | |
| B | IND | 1.20 | 12 | | LAC | 8.70 | 87 | SDS | 0.1 | 1 | 30 | 0.677 | 14 | 26 | 41 | 65 | 91 | | |
| C | IND | 1.20 | 12 | | LAC | 8.70 | 87 | B700 | 0.1 | 1 | 30 | 0.621 | 13 | 25 | 43 | 68 | 91 | | |
| D | MEL | 1.2 | 20.0 | | MAN | 4.62 | 77 | SDS | 0.18 | 3.0 | 10 | 0.277 | 37 | 53 | 66 | 74 | 86 | 83 | |
| E | MEL | 1.2 | 20.0 | | MAN | 4.8 | 80 | | | | 15 | 2.493 | 10 | 12 | 12 | 15 | 39 | 33 | |
| F | DIC | 3 | 30 | 30 | MAN | 6.7 | 67 | SDS | 0.3 | 3 | 90 | 0.157 | 63 | 79 | 86 | 88 | 93 | | |

**Figure 2A**

| Sample No. | Active material | | | Primary Matrix | | | 2nd Matrix | | | Time (mins.) | Particle Size | | | | | | Yield (%) | Variations |
|---|---|---|---|---|---|---|---|---|---|---|---|---|---|---|---|---|---|---|
| | Name | Mass (g) | % w/w | Name | Mass (g) | % w/w | Name | Mass (g) | % w/w | | D(0.5) µm | % <0.20 µm | % <0.30 µm | % < 0.5 µm | % < 1.0 µm | % < 2.0 µm | | |
| A | NAA | 1.2 | 30.0 | LAC | 2 | 50.0 | TCD | 0.8 | 20.0 | 20 | 0.188 | 48 | 64 | 75 | 81 | 92 | | |
| B | NAA | 1.2 | 30.0 | LAC | 2 | 50.0 | CAC | 0.8 | 20.0 | 20 | 0.213 | 47 | 63 | 76 | 84 | 91 | | |
| C | NAA | 1 | 25.0 | LAA | 2.2 | 55.0 | XYL | 0.8 | 20.0 | 20 | 0.2 | 50 | 65 | 75 | 79 | 89 | | |
| D | NAA | 1 | 25.0 | LAA | 2.2 | 55.0 | MAA | 0.8 | 20.0 | 20 | 0.223 | 46 | 60 | 70 | 76 | 87 | | |
| E | NAA | 1 | 25.0 | LAA | 2.2 | 55.0 | TCD | 0.8 | 20.0 | 20 | 0.215 | 47 | 62 | 70 | 73 | 83 | | |

**Figure 3A**

**Figure 4A**

| Sample No. | Active material Name | Active material Mass (g) | Active material %w/w | Active material %v/v | Primary Matrix Name | Primary Matrix Mass (g) | Primary Matrix %w/w | Surfactant #1 Name | Surfactant #1 Mass (g) | Surfactant #1 %w/w | 2nd Matrix Name | 2nd Matrix Mass (g) | 2nd Matrix %w/w | Time (mins.) | D(0.5) μm | %<0.20 μm | %<0.30 μm | %<0.5 μm | %<1.0 μm | %<2.0 μm | Yield (%) | Variations |
|---|---|---|---|---|---|---|---|---|---|---|---|---|---|---|---|---|---|---|---|---|---|---|
| A | MEL | 20 | 20.0 | | LAC | 77 | 77.0 | SDS | 3 | 3.0 | | | | 15 | 0.24 | 39 | 64 | 87 | 97 | 100 | 90 | |
| B | MEL | 20 | 20.0 | | LAC | 80 | 80.0 | | | | | | | 15 | 0.166 | 59 | 74 | 82 | 87 | 90 | 0.7 | |
| C | IND | 13 | 13.0 | | LAC | 87 | 87.0 | | | | | | | 30 | 3.255 | 0 | 0 | 0 | 4 | 27 | 1 | |
| D | IND | 13 | 13.0 | | LAC | 65.5 | 65.5 | SDS | 1 | 1.0 | TA | 22 | 21.5 | 30 | 0.272 | 34 | 55 | 76 | 87 | 93 | 0 | |
| E | IND | 13 | 13.0 | | LAC | 86 | 86.0 | SDS | 1 | 1.0 | | | | 30 | 0.836 | 22 | 31 | 39 | 56 | 83 | 76 | |
| F | IND | 13 | 13.0 | | LAC | 64.5 | 64.5 | SDS | 1 | 1.0 | TA | 22 | 21.5 | 30 | 0.629 | 15 | 28 | 43 | 67 | 91 | 85 | |
| G | MEL | 25 | 25 | 25 | LAC | 72 | 72 | SDS | 3 | 3 | | | | 15 | 0.283 | 33 | 53 | 73 | 84 | 92 | | |

**Figure 5A**

| Sample No. | Active material Name | Active material Mass (g) | Active material %w/w | Primary Matrix Name | Primary Matrix Mass (g) | Primary Matrix %w/w | Surfactant #1 Name | Surfactant #1 Mass (g) | Surfactant #1 %w/w | Surfactant #2 Name | Surfactant #2 Mass (g) | Surfactant #2 %w/w | Time (mins.) | D(0.5) μm | %<0.20 μm | %<0.30 μm | %<0.5 μm | %<1.0 μm | %<2.0 μm | Yield (%) | Variations |
|---|---|---|---|---|---|---|---|---|---|---|---|---|---|---|---|---|---|---|---|---|---|
| A | NAA | 17.5 | 35.0 | MAN | 32 | 64.0 | SDS | 0.5 | 1.0 | | | | 80 | 0.249 | 42 | 56 | 64 | 67 | 74 | | |
| B | NAA | 17.5 | 35.0 | MAN | 31.5 | 63.0 | SDS | 0.5 | 1.0 | P40S | 0.5 | 1 | 80 | 0.261 | 39 | 55 | 67 | 77 | 88 | | |
| C | NAA | 17.5 | 35.0 | MAN | 31.5 | 63.0 | SDS | 0.5 | 1.0 | P3000 | 0.5 | 1 | 80 | 0.188 | 53 | 70 | 81 | 88 | 95 | | |
| D | IND | 6 | 12.0 | LAC | 43.5 | 87.0 | SDS | 0.5 | 1.0 | | | | 40 | 0.231 | 43 | 61 | 78 | 91 | 97 | | |
| E | IND | 6 | 12.0 | LAC | 43 | 86.0 | SDS | 0.5 | 1.0 | P407 | 0.5 | 1 | 40 | 0.152 | 66 | 85 | 95 | 97 | 98 | | |
| F | IND | 6 | 12.0 | LAC | 43 | 86.0 | SDS | 0.5 | 1.0 | P40S | 0.5 | 1 | 40 | 0.155 | 65 | 85 | 96 | 98 | 98 | | |

| Sample No. | Active material | | | | Primary Matrix | | | Surfactant #1 | | | Surfactant #2 | | | 2nd Matrix | | | Time (mins.) | Particle Size | | | | | | | Yield (%) | Variations |
|---|---|---|---|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | Name | Mass (g) | % w/w | | D(0.5) μm | % <0.20 μm | % <0.30 μm | % < 0.5 μm | % < 1.0 μm | % < 2.0 μm | No. Ave. nm | | |
| A | NAA | 70 | 35 | | LAC | 128 | 64 | SDS | 2 | 1 | | | | | | | 60 | 0.345 | 35 | 47 | 56 | 61 | 73 | | 98 | O |
| B | NAA | 70 | 35 | | MAN | 128 | 64 | SDS | 2 | 1 | | | | | | | 50 | 0.73 | 31 | 41 | 48 | 51 | 58 | | | C |
| C | NAA | 60 | 30.0 | | MAN | 138 | 69.0 | SDS | 2 | 1.0 | | | | | | | 50 | 0.181 | 55 | 73 | 86 | 92 | 96 | | 92 | C |
| D | NAA | 60 | 30.3 | | MAN | 138 | 69.7 | | | | | | | | | | 50 | 0.319 | 35 | 48 | 59 | 64 | 75 | | 23 | C |
| E | DIC | 52.5 | 15.0 | | LAC | 294 | 84.0 | SDS | 3.5 | 1.0 | | | | | | | 40 | 0.16 | 64 | 84 | 97 | 99 | 99 | | 64 | E |
| F | DIC | 52.5 | 13.0 | | LAC | 224 | 66.0 | SDS | 3.5 | 1.0 | | | | TA | 70 | 20 | 40 | 0.16 | 63 | 83 | 95 | 98 | 99 | | 87 | E |
| G | NAA | 60 | 30 | 35 | LAC | 138 | 69 | SDS | 2 | 1 | | | | | | | 40 | 0.232 | 44 | 59 | 70 | 78 | 90 | | 79 | E |
| H | 2,4D | 40 | 20 | | LAC | 160 | 80 | | | | | | | | | | 30 | 0.212 | 47 | 69 | 90 | 100 | 100 | | 95 | |
| I | 2,4D | 40 | 20 | | LAC | 158 | 79 | SDA | 2 | 1 | | | | | | | 30 | 0.189 | 53 | 72 | 87 | 95 | 97 | | 97 | |
| J | 2,4D | 40 | 20 | | LAC | 158 | 79 | K1251 | 2 | 1 | | | | | | | 30 | 0.2 | 50 | 71 | 89 | 97 | 97 | | 97 | |
| K | 2,4D | 40 | 20 | | LAC | 158 | 79 | D920 | 2 | 1 | | | | | | | 30 | 0.204 | 49 | 69 | 86 | 94 | 97 | | 94 | |
| L | 2,4D | 60 | 20 | | LAC | 234 | 78 | SDA | 3 | 1 | PVP | 3 | 1 | | | | 30 | 0.281 | 30 | 54 | 82 | 96 | 96 | | 93 | |
| M | 2,4D | 60 | 20 | | LAC | 234 | 78 | D920 | 4 | 1 | PVP | 3 | 1 | | | | 40 | 0.183 | 55 | 75 | 91 | 98 | 98 | | 90 | |
| N | 2,4D | 60 | 20 | | LAC | 234 | 78 | K1251 | 3 | 1 | PVP | 3 | 1 | | | | 40 | 0.208 | 48 | 68 | 88 | 99 | 100 | | 92 | |
| O | GLY | 40 | 20 | | LAC | 158 | 79 | B700 | 2 | 1 | | | | | | | 90 | 0.297 | 38 | 50 | 61 | 74 | 87 | | 18 | |
| P | GLY | 40 | 20 | | LAC | 156 | 78 | B700 | 2 | 1 | T2700 | 2 | 1 | | | | 45 | 0.188 | 53 | 71 | 85 | 93 | 96 | | 79 | D |
| Q | GLY | 60 | 20 | | LAC | 234 | 78 | B700 | 3 | 1 | T2700 | 3 | 1 | | | | 30 | 4.798 | 0 | 0 | 0 | 0.2 | 9.9 | | | D |
| R | GLY | 60 | 20 | | LAC | 234 | 78 | B700 | 3 | 1 | T2700 | 3 | 1 | | | | 50 | 0.204 | 49 | 66 | 79 | 89 | 94 | | | D |

Figure 6A

## Figure 6B

| Sample No. | Active material Name | Active material Mass (g) | Active material %w/w | Active material %v/v | Primary Matrix Name | Primary Matrix Mass (g) | Primary Matrix %w/w | Surfactant #1 Name | Surfactant #1 Mass (g) | Surfactant #1 %w/w | Surfactant #2 Name | Surfactant #2 Mass (g) | Surfactant #2 %w/w | 2nd Name | 2nd Mass (g) | 2nd %w/w | Time (mins.) | D(0.5) μm | %<0.20 μm | %<0.30 μm | %>0.5 μm | %>1.0 μm | %>2.0 μm | No. Ave. nm | Yield (%) | Variations |
|---|---|---|---|---|---|---|---|---|---|---|---|---|---|---|---|---|---|---|---|---|---|---|---|---|---|---|
| S | GLY | 60 | 20 | | LAC | 234 | 78 | B700 | 3 | 1 | T2700 | 3 | 1 | | | | 70 | 0.17 | 58 | 75 | 88 | 95 | 97 | | 94.7 | D |
| T | MEL | 35 | 10 | | LAC | 311.5 | 89 | LEC | 3.5 | 1 | | | | | | | 40 | 0.127 | 80 | 94 | 98 | 98 | 99 | | 81 | |
| U | MEL | 35 | 10 | | MAN | 311.5 | 89 | LEC | 3.5 | 1 | | | | | | | 20 | 0.199 | 50 | 67 | 76 | 82 | 91 | | 59 | 1 |
| V | MEL | 35 | 10 | | LAC | 309.8 | 89 | P188 | 3.5 | 1 | DS | 1.77 | 0.5 | | | | 20 | 0.13 | 77 | 94 | 100 | 100 | 100 | | 90 | 1 |
| W | MEL | 17.5 | 5 | | MAN | 323.8 | 93 | P188 | 7 | 2 | | | | | | | 25 | 0.124 | 80 | 96 | 100 | 100 | 100 | | 67 | 1 |
| X | MEL | 17.5 | 5 | | LAC | 320.3 | 92 | P188 | 10.5 | 3 | LEC | 1.75 | 0.5 | | | | 40 | 0.129 | 78 | 94 | 99 | 100 | 99 | | 94 | 1 |
| Y | MEL | 17.5 | 5 | | MAN | 320.3 | 92 | P188 | 10.5 | 3 | LEC | 1.75 | 0.5 | | | | 25 | 0.14 | 72 | 88 | 93 | 94 | 98 | | 97 | 1 |
| Z | MEL | 35 | 10 | | LAC | 302.8 | 87 | P188 | 10.5 | 3 | LEC | 1.75 | 0.5 | | | | 30 | 0.168 | 59 | 75 | 83 | 87 | 94 | | 52 | 1 |
| AA | MEL | 35 | 10 | | LAC | 311.5 | 89 | P188 | 3.5 | 1 | LEC | 1.75 | 0.5 | | | | 40 | 0.118 | 87 | 99 | 100 | 100 | 100 | | 87 | 1 |
| AB | MEL | 35 | 10 | | MAN | 311.5 | 89 | P188 | 3.5 | 1 | | | | | | | 30 | 0.164 | 60 | 77 | 87 | 93 | 97 | | 32 | 1 |
| AC | MEL | 35 | 10 | | LAC | 315.0 | 90 | | | | | | | | | | 20 | 0.143 | 71 | 89 | 95 | 96 | 98 | | 79 | 1 |
| AD | MEL | 35 | 10 | | MAN | 315.0 | 90 | | | | | | | | | | 25 | 0.26 | 39 | 55 | 66 | 73 | 85 | | 56 | 1 |
| AE | CRM | 60 | 20 | | LAC | 138 | 69 | LEC | 1 | 2 | | | | | | | 60 | 0.152 | 64 | 78 | 84 | 87 | 89 | | 79 | 7 |
| AF | CIL | 30 | 10 | | LAC | 267 | 89 | SDS | 3 | 1 | | | | | | | 20 | 0.162 | 64 | 86 | 99 | 100 | 100 | | 84 | 5,D |
| AG | PRO | 30 | 10 | | LAC | 267 | 89 | SDS | 3 | 1 | | | | | | | 30 | 0.62 | 12 | 24 | 42 | 67 | 89 | | 74 | 5,D |
| AH | PRO | 30 | 10 | | LAC | 270 | 90 | | | | | | | | | | 30 | 0.91 | 9 | 18 | 33 | 52 | 61 | | 66 | 5,D |
| AI | CIP | 30.0 | 15 | | LAA | 168.0 | 84 | T80 | 2.00 | 1 | | | | | | | 20 | 0.139 | 76 | 91 | 94 | 94 | 95 | 88 | 94 | E |
| AJ | CIP | 30.1 | 15 | | LAA | 170.0 | 85 | | | | | | | | | | 20 | 0.171 | 60 | 75 | 79 | 79 | 82 | | 36.7 | E |
| AK | CIP | 30.0 | 15 | | LAA | 168.0 | 84 | CEL | 2.00 | 1 | | | | | | | 20 | 0.277 | 41 | 51 | 54 | 54 | 56 | | 72 | E |

| Sample No. | Active material | | | | Primary Matrix | | | Surfactant #1 | | | Surfactant #2 | | | 2nd | | | Time (mins.) | Particle Size | | | | | | | Yield (%) | Variations |
|---|---|---|---|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | Name | Mass (g) | % w/w | | D(0.5) µm | % <0.20 µm | % <0.30 µm | % < 0.5 µm | % < 1.0 µm | % < 2.0 µm | No. Ave. nm | | |
| AL | GLY | 60.0 | 20 | | LAC | 240.0 | 80 | | | | | | | | | | 70 | 50.4 | 0 | 0 | 0 | 0.9 | 4.4 | 1282 | 26 | 3,P |
| AM | CEL | 20.0 | 10 | | LAC | 176.1 | 88 | SDS | 2.00 | 1 | P40S | 2 | 1 | | | | 10 | 0.205 | 49 | 66 | 79 | 86 | 92 | 81 | 86 | 1,D |
| AN | CEL | 20.0 | 10 | | LAC | 180.1 | 90 | | | | | | | | | | 10 | 4.775 | 0 | 0 | 0 | 0 | 6.4 | 2560 | 57 | 1,D |
| AO | CEL | 20.0 | 10 | | LAC | 176.0 | 88 | SDS | 2.00 | 1 | P8000 | 2 | 1 | | | | 10 | 0.353 | 34 | 46 | 56 | 64 | 77 | 80 | 86 | 1,D |
| AP | MAN | 150.1 | 50 | 45 | LAC | 147.1 | 49 | T3785 | 3.00 | 3 | | | | | | | 5 | 0.22 | 46 | 60 | 72 | 84 | 87 | 89 | 90 | 8,D |
| AQ | MAN | 150.1 | 50 | 45 | LAC | 150.0 | 50 | | | | | | | | | | 5 | 0.292 | 35 | 51 | 67 | 81 | 85 | 109 | 56 | 8,D |
| AR | MAN | 150.0 | 50 | 45 | LAC | 147.0 | 49 | DS | 3.02 | 3 | | | | | | | 5 | 0.274 | 38 | 53 | 67 | 80 | 84 | | 76 | 8,D |
| AS | NAA | 105.1 | 35 | 39 | MAN | 195.0 | 65 | | | | | | | | | | 80 | 0.189 | 53 | 70 | 82 | 87 | 91 | 80 | 81 | |
| AT | NAA | 105.0 | 35 | | MAN | 180.1 | 60 | MCC | 15.00 | 5 | | | | | | | 80 | 0.261 | 40 | 54 | 65 | 69 | 75 | 81 | 66 | D |
| AU | NAA | 105.0 | 35 | | MAN | 180.0 | 60 | PML | 15.10 | 5 | | | | | | | 80 | 0.243 | 42 | 58 | 69 | 76 | 85 | 83 | 51 | D |

**Figure 6C**

| Sample No. | Active material Name | Mass (g) | % w/w | % v/v | Primary Matrix Name | Mass (g) | % w/w | Surfactant #1 Name | Mass (g) | % w/w | Surfactant #2 Name | Mass (g) | % w/w | 2nd Matrix Name | Mass (g) | % w/w | Disintegrant Name | Mass (g) | % w/w | Time (mins.) | D(0.5) μm | % <0.20 μm | % <0.30 μm | % < 0.5 μm | % < 1.0 μm | % < 2.0 μm | Yield (%) | Variations |
|---|---|---|---|---|---|---|---|---|---|---|---|---|---|---|---|---|---|---|---|---|---|---|---|---|---|---|---|---|
| A | MTX | 1.5 | 30.0 | | LAC | 3.5 | 69.0 | P407 | 0.1 | 1.0 | | | | | | | | | | 60 | 0.16 | 63 | 77 | 84 | 89 | 93 | | 2 |
| B | MTX | 1.5 | 30.0 | | LAC | 3.5 | 70.0 | | | | | | | | | | | | | 60 | 0.28 | 40 | 52 | 59 | 59 | 71 | | 2 |
| C | MTX | 17.2 | 43.0 | | LAC | 22 | 56.0 | SDS | 0.4 | 1.0 | | | | | | | | | | 60 | 0.142 | 70 | 83 | 88 | 91 | 94 | | 2 |
| D | MTX | 20 | 50.0 | | LAC | 10.4 | 26.0 | SDS | 0.8 | 2.0 | P407 | 0.8 | 2 | SB | 8 | 20.0 | | | | 60 | 0.137 | 73 | 89 | 95 | 100 | 100 | | 9 |
| E | MTX | 2.5 | 50.0 | | LAC | 2.35 | 47.0 | SDS | 0.8 | 2.0 | P407 | 0.1 | 2 | | | | | | | 60 | 0.148 | 67 | 83 | 92 | 98 | 99 | | 2 |
| F | MTX | 17.2 | 43.0 | | MAN | 22.4 | 56.0 | SDS | 0.4 | 1.0 | | | | | | | | | | 60 | 0.254 | 42 | 55 | 64 | 67 | 72 | | 2 |
| G | MTX | 1 | 20 | | LAC | 4 | 80 | | | | | | | | | | | | | 60 | 13.45 | 0 | 0 | 0 | 0 | 0 | 92 | 2 |
| H | MTX | 1 | 20 | | LAC | 3.9 | 78 | SDS | 0.05 | 1 | P407 | 0.05 | 1 | | | | | | | 60 | 0.13 | 76 | 91 | 96 | 98 | 98 | 97 | 2 |
| I | MTX | 1.25 | 25 | | LAC | 2.85 | 68 | SDS | 0.05 | 1 | P407 | 0.05 | 1 | PVP | 0.05 | 1 | PRI | 0.25 | 5 | 50 | 0.201 | 50 | 67 | 80 | 84 | 84 | 85 | 2 |
| J | MTX | 60 | 30 | 33 | LAC | 137 | 67 | SDS | 3 | 1.5 | P407 | 3 | 1.5 | | | | | | | 5 | 3.943 | 20 | 27 | 30 | 31 | 38 | | 2 |
| K | MTX | 60 | 30 | 33 | LAC | 137 | 67 | SDS | 3 | 1.5 | P407 | 3 | 1.5 | | | | | | | 10 | 0.223 | 46 | 61 | 72 | 77 | 83 | | 2 |
| L | MTX | 60 | 30 | 33 | LAC | 137 | 67 | SDS | 3 | 1.5 | P407 | 3 | 1.5 | | | | | | | 16 | 0.153 | 64 | 79 | 86 | 93 | 96 | | 2 |
| M | MTX | 60 | 30 | 33 | LAC | 137 | 67 | SDS | 3 | 1.5 | P407 | 3 | 1.5 | | | | | | | 21 | 0.142 | 67 | 85 | 92 | 95 | 96 | | 2 |
| N | 7M | 151 | 94 | | | | | | | | PVP | 1.61 | 1 | | | | PRI | 8.04 | 5 | 2 | | | | | | | 97 | 2 |
| O | MTX | 60 | 30 | 33 | LAC | 137 | 67 | SDS | 3 | 1.5 | P407 | 3 | 1.5 | | | | | | | 20 | 0.8 | 32 | 42 | 48 | 51 | 63 | 70 | 2,E |

**Figure 7A**

| Sample No. | Active material | | | | Primary Matrix | | | Surfactant #1 | | | Surfactant #2 | | | 2nd Matrix | | | Time (mins.) | Particle Size | | | | | | Yield (%) | Variations |
|---|---|---|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | Name | Mass (g) | % w/w | | D(0.5) µm | % <0.20 µm | % <0.30 µm | % < 0.5 µm | % < 1.0 µm | % < 2.0 µm | | |
| A | MEL | 48 | 10 | | LAC | 417.6 | 87 | SDS | 14.4 | 3 | | | | | | | 3 | 0.15 | 66 | 83 | 90 | 91 | 94 | 97 | 1 |
| B | MEL | 24 | 5 | | LAC | 439.2 | 91.5 | P188 | 14.4 | 3 | LEC | 2.4 | 0.5 | | | | 8 | 0.159 | 63 | 81 | 91 | 94 | 98 | 97 | 1 |
| C | MEL | 24 | 5 | | MAN | 439.2 | 91.5 | P188 | 14.4 | 3 | LEC | 2.4 | 0.5 | | | | 8 | 0.144 | 70 | 88 | 94 | 95 | 98 | 92 | 1 |
| D | IND | 62.4 | 13 | | LAC | 312 | 65 | SDS | 4.8 | 1 | | | | TA | 100.8 | 21 | 4 | 0.197 | 51 | 68 | 81 | 88 | 94 | 91 | |
| E | IND | 62.4 | 13 | | LAC | 312 | 66 | | | | | | | TA | 100.8 | 21 | 4 | 0.19 | 53 | 71 | 85 | 92 | 97 | 74 | |
| F | IND | 62.4 | 13 | | LAC | 312 | 65 | SDS | 4.8 | 1 | | | | TA | 100.8 | 21 | 4 | 0.194 | 52 | 71 | 86 | 93 | 97 | 84 | |
| G | IND | 48 | 10 | | SUC | 427.2 | 89 | SDS | 4.8 | 1 | | | | | | | 5 | 0.213 | 47 | 64 | 76 | 84 | 92 | 93 | |
| H | IND | 48 | 10 | | SUC | 427.2 | 89 | SDS | 4.8 | 1 | | | | | | | 6 | 0.192 | 52 | 72 | 87 | 93 | 96 | 94 | |
| I | MTX | 144 | 30 | 33 | LAC | 321.6 | 67 | SDS | 7.2 | 1.5 | P407 | 7.2 | 1.5 | | | | 4 | 0.243 | 44 | 58 | 68 | 74 | 84 | 93 | 2 |
| J | ANT | 50 | 10 | | LAC | 445 | 89 | SDS | 5 | 1 | | | | | | | 4 | 0.288 | 32 | 51 | 73 | 86 | 91 | 90 | 5 |
| K | DIC | 72 | 15 | | LAC | 403.2 | 84 | SDS | 4.8 | 1 | | | | | | | 3 | 0.186 | 54 | 74 | 89 | 95 | 98 | 94 | |
| L | NAA | 168 | 35 | 39 | MAN | 302.4 | 63 | SDS | 4.8 | 1 | PVP | 4.8 | 1 | | | | 6 | 0.226 | 44 | 63 | 80 | 88 | 93 | 94 | |
| M | NAA | 168 | 35 | 39 | MAN | 297.6 | 62 | SDS | 4.8 | 1 | PVP | 4.8 | 1 | P3000 | 4.8 | 1 | 7 | 0.287 | 31 | 52 | 73 | 85 | 93 | 98 | |
| N | COP | 48 | 10 | | LFG | 427.2 | 89 | SDS | 4.8 | 1 | | | | | | | 7 | 4.319 | 0 | 0 | 0 | 3 | 16 | 93 | 10 |
| O | COP | 96 | 20 | | LFG | 374.4 | 78 | LEC | 9.6 | 2 | | | | | | | 18 | 2.375 | 0 | 0 | 0 | 9 | 39 | 80 | 10 |
| P | CON | 144 | 30 | | LFG | 326.4 | 68 | LEC | 9.6 | 2 | | | | | | | 1.5 | 4.027 | 0 | 0 | 0 | 7 | 23 | 83 | 10 |

**Figure 8A**

## Figure 9A

| Sample No. | Active material Name | Mass (g) | %w/w | Primary Matrix Name | Mass (g) | %w/w | Surfactant #1 Name | Mass (g) | %w/w | Surfactant #2 Name | Mass (g) | %w/w | 2nd Matrix Name | Mass (g) | %w/w | Time (mins.) | D(0.5) µm | %<0.20 µm | %<0.30 µm | %<0.5 µm | %<1.0 µm | %<2.0 µm | No.Ave.(nm) | Yield (%) | Variations |
|---|---|---|---|---|---|---|---|---|---|---|---|---|---|---|---|---|---|---|---|---|---|---|---|---|---|
| A | MEL | 40 | 5 | MAN | 732 | 91.5 | P188 | 24 | 3 | LEC | 4 | 0.5 | | | | 40 | 0.116 | 84 | 97 | 100 | 100 | 100 | | 96 | 1,I |
| B | MEL | 40 | 5 | MAN | 732 | 91.5 | P188 | 24 | 3 | LEC | 4 | 0.5 | | | | 45 | 0.122 | 82 | 97 | 100 | 100 | 100 | | 95 | 1,I |
| C | MEL | 40 | 5 | MAN | 732 | 91.5 | P188 | 24 | 3 | LEC | 4 | 0.5 | | | | 40 | 0.124 | 80 | 96 | 100 | 100 | 100 | | 97 | 1,I |
| D | MEL | 52.5 | 5 | LAC | 960.8 | 91.5 | P188 | 31.5 | 3 | LEC | 5.25 | 0.5 | | | | 50 | 0.156 | 64 | 81 | 89 | 90 | 93 | | 88 | 1,H |
| E | MEL | 40.0 | 5 | MAN | 732.0 | 91.5 | P188 | 24 | 3 | LEC | 4 | 0.5 | | | | 40 | 0.142 | 71 | 88 | 93 | 93 | 95 | | 96 | 1,I |
| F | SAL | 100.0 | 10 | LAC | 890.0 | 89 | LEC | 10.00 | | | | | | | | 15 | 0.137 | 72 | 85 | 89 | 90 | 92 | 75 | 92 | L |
| G | SAL | 100.0 | 10 | LAC | 900.0 | 90 | | | | | | | | | | 15 | 4.954 | 0 | 0 | 2 | 11 | 24 | | 95 | L |
| H | IND | 130.0 | 13 | LAC | 870.0 | 87 | | | | | | | | | | 36 | 0.18 | 56 | 74 | 89 | 96 | 98 | 80 | 65 | |
| I | IND | 130.1 | 13 | LAC | 860.1 | 86 | SDS | 10.00 | 1 | | | | | | | 36 | 0.192 | 52 | 73 | 90 | 95 | 97 | 83 | 85 | |
| J | IND | 130.1 | 13 | LAA | 870.0 | 87 | | | | | | | | | | 36 | 0.186 | 54 | 72 | 86 | 93 | 97 | 80 | 51 | |
| K | DIC | 150.1 | 15 | LAA | 850.3 | 85 | | | | | | | | | | 36 | 0.242 | 41 | 60 | 79 | 92 | 99 | 87 | 27 | |
| L | MEL | 105.0 | 10 | LAC | 913.5 | 87 | SDS | 31.50 | 3 | | | | | | | 20 | 0.137 | 74 | 90 | 95 | 95 | 96 | 79 | 94 | G |
| M | MEL | 105.1 | 10 | LAC | 945 | 90.0 | | | | | | | TA | 215 | 21.5 | 20 | | | | | | | | | 1,G |
| N | IND | 130.0 | 13 | LAA | 860 | 86 | SDS | 10 | 1 | | | | | | | 36 | 0.161 | 62 | 79 | 90 | 93 | 95 | | 80 | 11,N |
| O | IND | 130.0 | 13 | LAA | 645 | 64.5 | SDS | 10 | 1 | | | | | | | 36 | 0.160 | 62 | 79 | 90 | 94 | 96 | | 87 | 11,N |
| P | DIC | 150 | 15 | LAA | 840 | 84 | SDS | 10 | 1 | | | | | | | 36 | 0.152 | 66 | 84 | 95 | 98 | 99 | | 80 | 11,N |
| Q | MEL | 75 | 7.1 | LAC | 943.5 | 90.0 | SDS | 31.5 | 3 | | | | | | | 30 | 0.129 | 78 | 94 | | 100 | | | 89 | 1,M |
| R | MEL | 71.6 | 6.8 | LAC | 946.9 | 90.2 | SDS | 31.5 | 3 | | | | | | | 30 | 0.312 | 72 | 89 | 94 | 94 | 96 | | 92 | 1,F |
| S | IND | 120 | 12 | LAC | 435 | 43.5 | SDS | 10 | 1 | | | | TA | 435 | 43.5 | 44 | 0.168 | 60 | 79 | 92 | 98 | 100 | | 80 | 11,K |

## Figure 9B

| Sample No. | Active material Name | Active material Mass (g) | Active material %w/w | Primary Matrix Name | Primary Matrix Mass (g) | Primary Matrix %w/w | Surfactant #1 Name | Surfactant #1 Mass (g) | Surfactant #1 %w/w | Surfactant #2 Name | Surfactant #2 Mass (g) | Surfactant #2 %w/w | 2nd Matrix Name | 2nd Matrix Mass (g) | 2nd Matrix %w/w | Time (mins.) | D(0.5) µm | %<0.20 µm | %<0.30 µm | %<0.5 µm | %<1.0 µm | %<2.0 µm | No.Ave. (nm) | Yield (%) | Variations |
|---|---|---|---|---|---|---|---|---|---|---|---|---|---|---|---|---|---|---|---|---|---|---|---|---|---|
| T | IND | 130 | 13 | LAC | 645 | 64.5 | SDS | 10 | 1 | | | | TA | 215 | 21.5 | 36 | 0.160 | 63 | 79 | 93 | 97 | 99 | | | 11 |
| U | IND | 130 | 13 | LAC | 645 | 64.5 | SDS | 10 | 1 | | | | TA | 215 | 21.5 | 36 | 0.179 | 56 | 72 | 89 | 95 | 97 | | | 11 |
| V | IND | 130 | 13 | LAC | 645 | 64.5 | SDS | 10 | 1 | | | | TA | 215 | 21.5 | 40 | 0.182 | 55 | 70 | 83 | 87 | 92 | | | 11 |
| W | DIC | 150 | 15 | LAC | 840 | 84 | SDS | 10 | 1 | | | | | | | 36 | 0.183 | 55 | 72 | 91 | 96 | 97 | | | 11 |
| X | DIC | 150 | 15 | LAC | 840 | 84 | SDS | 10 | 1 | | | | | | | 36 | 0.186 | 54 | 74 | 94 | 98 | 99 | | | 11 |
| Y | DIC | 150 | 15 | LAC | 840 | 84 | SDS | 10 | 1 | | | | | | | 36 | 0.203 | 49 | 69 | 92 | 97 | 98 | | | 11 |
| Z | NAA | 334 | 35.1 | MAN | 599 | 62.9 | SDS | 9.55 | 1.0 | PVP | 9.55 | 1.0 | | | | 60 | 0.399 | 33 | 44 | 53 | 59 | 69 | | | |
| AA | NAA | 334 | 35.1 | MAN | 599 | 62.9 | SDS | 9.55 | 1.0 | PVP | 9.55 | 1.0 | | | | 60 | 0.337 | 34 | 47 | 58 | 65 | 71 | | | |
| AB | NAA | 334 | 35.1 | MAN | 599 | 62.9 | SDS | 9.55 | 1.0 | PVP | 9.55 | 1.0 | | | | 60 | 0.300 | 37 | 50 | 61 | 69 | 76 | | | |
| AC | NAA | 334 | 35.1 | MAN | 599 | 62.9 | SDS | 9.55 | 1.0 | PVP | 9.55 | 1.0 | | | | 60 | 0.360 | 34 | 46 | 56 | 61 | 69 | | | |
| AD | NAA | 334 | 35.1 | MAN | 599 | 62.9 | SDS | 9.55 | 1.0 | PVP | 9.55 | 1.0 | | | | 60 | 0.366 | 33 | 45 | 55 | 61 | 69 | | | |
| AE | NAA | 334 | 35.1 | MAN | 599 | 62.9 | SDS | 9.55 | 1.0 | PVP | 9.55 | 1.0 | | | | 60 | 0.301 | 36 | 50 | 62 | 69 | 75 | | | |
| AF | NAA | 334 | 35.1 | MAN | 599 | 62.9 | SDS | 9.55 | 1.0 | PVP | 9.55 | 1.0 | | | | 60 | 0.298 | 37 | 50 | 62 | 68 | 74 | | | |
| AG | NAA | 334 | 35.1 | MAN | 599 | 62.9 | SDS | 9.55 | 1.0 | PVP | 9.55 | 1.0 | | | | 60 | 0.195 | 51 | 65 | 74 | 78 | 83 | | | |
| AH | NAA | 334 | 35.1 | MAN | 599 | 62.9 | SDS | 9.55 | 1.0 | PVP | 9.55 | 1.0 | | | | 60 | 0.294 | 37 | 51 | 62 | 68 | 76 | | | |
| AI | MEL | 105 | 11 | LAC | 864 | 86.4 | SDS | 31.5 | 3 | | | | | | | 20 | 0.189 | 53 | 72 | 84 | 88 | 94 | | | F |
| AJ | MEL | 105 | 11 | LAC | 864 | 86.4 | SDS | 31.5 | 3 | | | | | | | 25 | 0.153 | 65 | 84 | 94 | 95 | 98 | | | F |
| AK | MEL | 105 | 11 | LAC | 864 | 86.4 | SDS | 31.5 | 3 | | | | | | | 30 | 0.138 | 74 | 91 | 96 | 96 | 97 | | | F |
| AL | MEL | 105 | 11 | LAC | 864 | 86.4 | SDS | 31.5 | 3 | | | | | | | 35 | 0.126 | 79 | 96 | 100 | 100 | 100 | | 90 | F |

| Sample No. | Active material | | | | Primary Matrix | | | Surfactant #1 | | | Time (mins.) | Particle Size | | | | | | Yield (%) | Variations |
|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | | D(0.5) μm | % <0.20 μm | % <0.30 μm | % < 0.5 μm | % < 1.0 μm | % > 2.0 μm | | |
| A | DIC | 2.50 | 10 | | MAN | 22.5 | 89 | SDS | 0.25 | 1 | 30 | 0.237 | 40 | 63 | 83 | 93 | 97 | | |
| B | NAA | 70 | 35 | | LAC | 128 | 64 | SDS | 2 | 1 | 60 | 0.224 | 72 | 81 | 92 | 81 | 92 | | |
| C | NAA | 70 | 35 | | MAN | 128 | 64 | SDS | 2 | 1 | 60 | 0.177 | 57 | 74 | 86 | 90 | 93 | | |
| D | NAA | 80 | 40 | 40 | LAC | 118 | 60 | | | | 45 | 2.039 | 19 | 26 | 31 | 36 | 49 | | |
| E | DIC | 1650 | 15 | | LAC | 9240 | 84 | SDS | 110 | 1 | 20 | 0.24 | 42 | 58 | 74 | 86 | 94 | 91 | |
| F | DIC | 3750 | 15 | | LAC | 21000 | 84 | SDS | 250 | 1 | 25 | 0.214 | 49 | 68 | 82 | 93 | 97 | 97 | |

**Figure 10A**

| Sample No. | Active material | | | | Primary Matrix | | | Surfactant #1 | | | Surfactant #2 | | | Surfactant #3 | | | Disintegrant | | | Time (mins.) | Particle Size | | | | | | Yield (%) | Variations |
|---|---|---|---|---|---|---|---|---|---|---|---|---|---|---|---|---|---|---|---|---|---|---|---|---|---|---|---|---|
| | Name | Mass (g) | % w/w | % v/v | Name | Mass (g) | % w/w | Name | Mass (g) | % w/w | Name | Mass (g) | % w/w | Name | Mass (g) | % w/w | Name | Mass (g) | % w/w | | D(0.5) μm | % <0.20 μm | % <0.30 μm | % < 0.5 μm | % < 1.0 μm | % < 2.0 μm | | |
| A | NAA | 105 | 35 | 39 | MAN | 189 | 63 | SDS | 3 | 1 | P3000 | 3 | 1 | | | | | | | 80 | 0.19 | 53 | 71 | 84 | 91 | 95 | 90 | |
| B | NAA | 105 | 35 | 39 | MAN | 189 | 63 | SDS | 3 | 1 | P407 | 3.1 | 1 | | | | | | | 40 | 0.89 | 26 | 36 | 45 | 51 | 57 | | |
| C | NAA | 105 | 35 | 39 | MAN | 189 | 63 | SDS | 3 | 1 | P407 | 3.1 | 1 | | | | | | | 60 | 0.31 | 36 | 49 | 61 | 69 | 76 | | |
| D | NAA | 105 | 35 | 39 | MAN | 189 | 63 | SDS | 3 | 1 | P407 | 3.1 | 1 | | | | | | | 80 | 0.19 | 52 | 70 | 84 | 90 | 93 | 82 | |
| E | NAA | 105.1 | 35 | 39 | MAN | 192 | 64 | SDS | 3 | 1 | | | | | | | | | | 80 | 0.24 | 43 | 59 | 72 | 78 | 81 | 84.6 | |
| F | NAA | 105 | 35 | 39 | MAN | 171 | 57 | SDS | 3 | 1 | P3000 | 3 | 1 | PVP | 3 | 1 | PML | 15 | 5 | 80 | 0.27 | 39 | 54 | 67 | 74 | 78 | 89.2 | 12 |
| G | NAA | 105 | 35 | 39 | MAN | 171 | 57 | SDS | 3 | 1 | P407 | 3 | 1 | PVP | 3.02 | 1 | PML | 15.1 | 5 | 80 | | | | | | | 88.2 | |
| H | NAA | 105.2 | 35 | 39 | MAN | 174 | 58 | SDS | 3 | 1 | PVP | 3 | 1 | | | | PML | 15.0 | 5 | 80 | | | | | | | 87.1 | |
| I | NAA | 105 | 35 | 39 | MAN | 189 | 63 | SDS | 3 | 1 | P3000 | 3 | 1 | | | | | | | 80 | 0.25 | 27 | 67 | 91 | 100 | 100 | 88 | |
| J | NAA | 105.7 | 35 | 39 | MAN | 186 | 64 | SDS | 3 | 1 | P3000 | 3 | 1 | PVP | 3.01 | 1 | | | | 80 | 0.24 | 29 | 68 | 90 | 99 | 100 | 89.7 | |
| K | NAA | 105.1 | 35.0 | 39 | MAN | 195 | 65.0 | | | | | | | | | | | | | 80 | 0.19 | 53 | 70 | 82 | 87 | 91 | 81 | |
| L | NAA | 105 | 35.0 | | MAN | 180 | 60.0 | | | | | | | | | | MCC | 15 | 5 | 80 | 0.26 | 40 | 54 | 65 | 69 | 75 | 66 | 12,D |
| M | NAA | 105 | 35.0 | | MAN | 180 | 60.0 | | | | | | | | | | PML | 15 | 5 | 80 | 0.24 | 42 | 58 | 69 | 76 | 85 | 51 | 12,D |

**Figure 11A**

EP 2 421 530 B1

| Sample No. | Active material | | | Primary Matrix | | | Surfactant #1 | | | Surfactant #2 | | | Time (mins.) | Particle Size | | | | | | Yield (%) | Variations |
|---|---|---|---|---|---|---|---|---|---|---|---|---|---|---|---|---|---|---|---|---|---|
| | Name | Mass (g) | % w/w | Name | Mass (g) | % w/w | Name | Mass (g) | % w/w | Name | Mass (g) | % w/w | | D(0.5) µm | % <0.20 µm | % <0.30 µm | % < 0.5 µm | % < 1.0 µm | % < 2.0 µm | | |
| A | NAA | 1.5 | 30 | LAC | 3.2 | 64 | SDS | 0.05 | 1 | MCC | 0.25 | 5 | 40 | 2.6 | 29 | 41 | 47 | 61 | 77 | 86 | |
| B | 14A | | | | | | | | | | | | | 0.2 | 68 | 79 | 84 | 94 | 99 | | 12 |
| C | NAA | 1.5 | 30 | LAC | 3.45 | 69 | SDS | 0.05 | 1 | | | | 40 | 0.2 | 79 | 95 | 98 | 100 | 100 | 95 | |
| D | 14C | | | | | | | | | | | | | 0.2 | 80 | 94 | 97 | 100 | 100 | | 12 |
| E | 14C | 2.5 | 95 | MCC | 0.13 | 5 | | | | | | | 1 | 1.3 | 34 | 49 | 52 | 56 | 60 | 88 | |
| F | 14C | 2.5 | 91 | MCC | 0.25 | 9 | | | | | | | 1 | 0.8 | 36 | 52 | 56 | 62 | 72 | 83 | |
| G | 14E | | | | | | | | | | | | | 0.2 | 79 | 92 | 96 | 99 | 100 | | 12 |
| H | 14F | | | | | | | | | | | | | 0.2 | 79 | 93 | 97 | 99 | 100 | | 12 |
| I | NAA | 1.5 | 30 | LAC | 2.95 | 59 | SDS | 0.05 | 1 | MCC | 0.5 | 10 | 40 | 6.4 | 12 | 19 | 25 | 43 | 64 | 96 | |
| J | NAA | 1.5 | 30 | LAC | 2.45 | 49 | SDS | 0.05 | 1 | MCC | 1 | 20 | 40 | 8.6 | 0 | 0 | 7 | 31 | 56 | 95 | |
| K | 14I | | | | | | | | | | | | | 1.7 | 32 | 44 | 53 | 77 | 94 | | 12 |
| L | 14J | | | | | | | | | | | | | 4.1 | 0 | 0 | 12 | 61 | 92 | | 12 |

**Figure 12A**

**EP 2 421 530 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6634576 B
- AU 2005001977 W
- AU 2007000910 W
- WO 2005016310 A1
- US 5478705 A
- US 5500331 A
- US 7101576 B
- US 20050063913 A
- AU 2009901740
- AU 2009901742